(19) Europäisches Patentamt
European Patent Office
Office européen des brevets

(11) EP 3 436 077 B1

(12) EUROPEAN PATENT SPECIFICATION

(45) Date of publication and mention of the grant of the patent:
**07.05.2025 Bulletin 2025/19**

(21) Application number: **17719387.7**

(22) Date of filing: **30.03.2017**

(51) International Patent Classification (IPC):
***A61K 48/00*** (2006.01) ***C12N 15/11*** (2006.01)
***C12N 9/22*** (2006.01) ***C12N 15/88*** (2006.01)
***A61P 1/16*** (2006.01) ***A61P 43/00*** (2006.01)

(52) Cooperative Patent Classification (CPC):
**C12N 15/111; A61P 1/16; A61P 43/00; C12N 9/22; C12N 15/88;** C12N 2310/20; C12N 2320/32

(86) International application number:
**PCT/US2017/024973**

(87) International publication number:
**WO 2017/173054 (05.10.2017 Gazette 2017/40)**

(54) **LIPID NANOPARTICLE FORMULATIONS FOR CRISPR/CAS COMPONENTS**

LIPIDNANOPARTIKELFORMULIERUNGEN FÜR CRISPR/CAS-KOMPONENTEN

FORMULATIONS DE NANOPARTICULES LIPIDIQUES POUR DES COMPOSÉS CRISPR/CAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2016 US 201662315602 P**
**16.08.2016 US 201662375776 P**
**12.12.2016 US 201662433228 P**
**07.03.2017 US 201762468300 P**

(43) Date of publication of application:
**06.02.2019 Bulletin 2019/06**

(60) Divisional application:
**25164405.0**

(73) Proprietor: **Intellia Therapeutics, Inc.**
**Cambridge, MA 02139 (US)**

(72) Inventors:
- **MORRISSEY, David V.**
  **Winchester, MA 01890 (US)**
- **PATEL, Mihir, Chandrakant**
  **Perkasie, PA 18944 (US)**
- **FINN, Jonathan D.**
  **Melrose, MA 02176 (US)**
- **SMITH, Amy Madison Rhoden**
  **Watertown, MA 02472 (US)**
- **SHAW, Lucinda J.**
  **Lexington, MA 02421 (US)**
- **DOMBROWSKI, Christian**
  **Boston, MA 02130 (US)**
- **SHAH, Ruchi Rudraprasad**
  **Brookline, MA 02445 (US)**

(74) Representative: **Schlich**
**9 St Catherine's Road**
**Littlehampton**
**West Sussex BN17 5HS (GB)**

(56) References cited:
**WO-A1-2010/129687** **WO-A1-2014/204726**
**WO-A1-2016/037053** **WO-A2-2010/088537**
**US-A1- 2012 295 832**

- **XIAO WANG ET AL: "CRISPR-Cas9 Targeting of PCSK9 in Human Hepatocytes In Vivo", ARTERIOSCLEROSIS, THROMBOSIS, AND VASCULAR BIOLOGY., vol. 36, no. 5, 3 March 2016 (2016-03-03), US, pages 783 - 786, XP055385148, ISSN: 1079-5642, DOI: 10.1161/ ATVBAHA.116.307227**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 436 077 B1

- KATHRYN A. WHITEHEAD ET AL: "Degradable lipid nanoparticles with predictable in vivo siRNA delivery activity", NATURE COMMUNICATIONS, vol. 5, 27 June 2014 (2014-06-27), XP055385085, DOI: 10.1038/ncomms5277
- CHENG XINWEI ET AL: "The role of helper lipids in lipid nanoparticles (LNPs) designed for oligonucleotide delivery", ADVANCED DRUG DELIVERY REVIEWS, vol. 99, 18 February 2016 (2016-02-18), pages 129 - 137, XP029445785, ISSN: 0169-409X, DOI: 10.1016/J.ADDR.2016.01.022
- HIRSCH-LERNER D ET AL: "Effect of ”helper lipid” on lipoplex electrostatics", BIOCHIMICA ET BIOPHYSICA ACTA (BBA) - BIOMEMBRANES, ELSEVIER, AMSTERDAM, NL, vol. 1714, no. 2, 15 August 2005 (2005-08-15), pages 71 - 84, XP027733952, ISSN: 0005-2736, [retrieved on 20050815]
- LAINÉ A-L ET AL: "Conventional versus stealth lipid nanoparticles: Formulation andin vivofate prediction through FRET monitoring", JOURNAL OF CONTROLLED RELEASE, ELSEVIER, AMSTERDAM, NL, vol. 188, 28 May 2014 (2014-05-28), pages 1 - 8, XP029011028, ISSN: 0168-3659, DOI: 10.1016/J.JCONREL.2014.05.042

## Description

The present application claims the benefit of priority to U.S. Provisional Patent Application No. 62/315,602 filed March 30, 2016, U.S. Provisional Patent Application No. 62/375,776 filed August 16, 2016, U.S. Provisional Patent Application No. 62/433,228 filed December 12, 2016, and U.S. Provisional Patent Application No. 62/468,300 filed March 7, 2017.

The delivery of biologically active agents (including therapeutically relevant compounds) to subjects is often hindered by difficulties in the agents reaching the target cell or tissue. In particular, the trafficking of many biologically active agents into living cells can be restricted by the membrane systems of the cells.

One class of biologically active agents that is particularly difficult to deliver to cells are biologics including proteins, nucleic acid-based drugs, and derivatives thereof. Certain nucleic acids and proteins are stable for only a limited duration in cells or plasma, and sometimes are highly charged, which can complicate delivery across cell membranes. Compositions that can stabilize and deliver such agents into cells are therefore of particular interest. Lipid carriers, biodegradable polymers and various conjugate systems can be used to improve delivery of these biologically active agents to cells.

A number of components and compositions for editing genes in cells *in vivo* now exist, providing tremendous potential for treating genetic, viral, bacterial, autoimmune, cancer, aging-related, and inflammatory diseases. Several of these editing technologies take advantage of cellular mechanisms for repairing double-stranded breaks ("DSB") created by enzymes such as meganucleases, clustered regularly interspaced short palindromic repeats (CRISPR) associated ("Cas") nucleases, zinc finger nucleases ("ZFN"), and transcription activator-like effector nucleases ("TALEN"). When DSBs are made in a cell, the cell may repair the break by one of several processes. One such process involves non-homologous end joining ("NHEJ") of the cleaved ends of DNA. During NHEJ, nucleotides may be added or removed by the cell, resulting in a sequence altered from the cleaved sequence. In other circumstances, cells repair DSBs by homology-directed repair ("HDR") or homologous recombination ("HR") mechanisms, where an endogenous or exogenous template with homology to each end of a DSB, for example, is used to direct repair of the break. Several of these editing technologies take advantage of cellular mechanisms for repairing single-stranded breaks or double-stranded breaks ("DSB").

CRISPR/Cas gene editing systems are active as ribonucleoprotein complexes in a cell. Compositions for delivery of the protein and nucleic acid components of CRISPR/Cas to a cell, such as a cell in a patient, are needed.

WO 2014/204726 relates to delivery, engineering and optimization of systems, methods, and compositions for manipulation of sequences and/or activities of target sequences, including methods of dircting CRISPR complex formation in eukaryotic cells to edit or modify a target site in a genomic locus of interest to alter the status of a disease or a condition.

WO 2010/129687 relates to methods of delivery of nucleic acid-based agents to immune cells.

Wang et al., Arterioscler Thromb Vasc Biol. 2016 May; 36(5): 783-786, relates to CRISPR-Cas9 targeting of *PCSK9* in human hepatocytes *in vivo* using humanized mice as a platform for the preclinical assessment of applications of somatic in vivo genome editing.

Whitehead et al., (2014) Nat Commun. 5: 4277, describes degradable lipid nanoparticles and their *in vivo* siRNA delivery activity in hepatocytes.

We herein provide lipid nanoparticle-based compositions useful for delivery of CRISPR/Cas gene editing components. The invention itself is defined by the appended claims.

Described herein is a method of producing a genetically engineered liver cell, comprising contacting a cell with lipid nanoparticles (LNPs) comprising: a Class 2 Cas nuclease mRNA; a guide RNA nucleic acid; a CCD lipid; a helper lipid; a neutral lipid; and a stealth lipid. Lipid nanoparticles (LNPs) comprising a Class 2 Cas nuclease mRNA, a guide RNA nucleic acid, a CCD lipid, a helper lipid, a neutral lipid, and a stealth lipid are also provided.

Described herein is a method of gene editing, comprising delivering a Class 2 Cas nuclease mRNA and a guide RNA nucleic acid to a liver cell, wherein the Class 2 Cas mRNA and the guide RNA nucleic acid are formulated as at least one LNP composition comprising: a CCD lipid; a helper lipid; a neutral lipid; and a stealth lipid. Described herein is a method of administering a CRISPR-Cas complex to a liver cell, comprising contacting a cell with LNPs comprising: a Class 2 Cas nuclease mRNA; a guide RNA nucleic acid; a CCD lipid; a helper lipid; a neutral lipid; and a stealth lipid.

Described herein is a method of altering expression of a gene in a liver cell, comprising administering to the subject a therapeutically effective amount of a Class 2 Cas nuclease mRNA and a guide RNA nucleic acid as one or more LNP formulations, wherein at least one LNP formulation comprises: a guide RNA nucleic acid or a Class 2 Cas nuclease mRNA; a CCD lipid; a helper lipid; a neutral lipid; and a stealth lipid is provided.

The method of producing a genetically engineered liver cell may comprise contacting a cell with lipid nanoparticles (LNPs) comprising: a Class 2 Cas nuclease mRNA; a guide RNA nucleic acid that is or encodes a single-guide RNA (sgRNA); a CCD lipid; a helper lipid; a neutral lipid; and a stealth lipid.

The Class 2 Cas nuclease mRNA may be formulated in a first LNP composition and the guide RNA nucleic acid is formulated in a second LNP composition. In other aspects, the Class 2 Cas nuclease mRNA and the guide RNA nucleic acid are formulated together in an LNP composition.

## BRIEF DESCRIPTION OF DRAWINGS

Fig. 1 shows the expression of GFP after delivery of various LNP formulations to mouse hepatocyte cells (Hepa1.6) at amounts of 100 ng and 500 ng eGFP mRNA delivered per well.
Fig. 2 shows gLUC expression in mice after administration of various LNP formulations at varying doses, resulting in a dose-dependent response.
Fig. 3A shows the editing efficiency of targeting Factor VII in mice after administration of various LNP formulations.
Fig. 3B shows the editing efficiency of targeting TTR in mice after administration of various LNP formulations.
Fig. 4A shows the editing efficiency of targeting TTR in mice after delivery of various LNP formulations, according to various dosing regiments, where the gRNA and Cas9 mRNA are formulated separately.
Fig. 4B shows the editing efficiency of targeting TTR in mice after delivery of an LNP formulation where the gRNA and Cas9 mRNA are formulated separately.
Fig. 5 shows the editing efficiency of targeting Factor VII or TTR in cells after administration of various LNP formulations where the gRNA and Cas9 mRNA are formulated separately.
Fig. 6 shows the editing efficiency of targeting Factor VII or TTR in mice after administration of various LNP formulations where the gRNA and Cas9 mRNA are formulated separately.
Fig. 7 shows the editing efficiency in cells after administration of various LNP formulations where the gRNA and Cas9 mRNA are formulated together and delivered at various concentrations.
Fig. 8A shows the editing efficiency of targeting TTR in mice after administration of various LNP formulations.
Fig. 8B shows the editing efficiency of targeting Factor VII in mice after administration of various LNP formulations.
Fig. 9 shows PCR amplification of excision-site DNA collected from animals that were administered various LNP formulations.
Fig. 10 shows serum TTR levels of mice that were administered various LNP formulations where the gRNA and Cas9 mRNA are formulated together.
Fig. 11 shows relative Factor VII activity in mice after animals were administered various LNP formulations where the gRNA and Cas9 mRNA are formulated together.
Fig. 12A shows the editing efficiency of targeting TTR in mice after administering LNP-169 at various doses, resulting in a dose-dependent response.
Fig. 12B shows serum TTR levels in mice, on various days, after administering LNP-169 at various doses, resulting in a dose-dependent response.
Fig. 13A shows the editing efficiency of targeting TTR in mice after administration of various LNP formulations where the ratio of Cas9 mRNA to sgRNA was varied.
Fig. 13B shows the serum TTR levels in mice, on two separate days, after administration of various LNP formulations where the ratio of Cas9 mRNA to sgRNA was varied.
Fig. 14A shows the editing efficiency of targeting TTR in mice after administration of LNP-169 in one or two doses.
Fig. 14B shows the serum TTR levels in mice nine days after administration of LNP-169 in one or two doses.
Fig. 15 shows the editing efficiency in the spleen of targeting TTR in mice after administration of various LNP formulations.
Fig. 16 shows the editing efficiency of targeting TTR in mice after administration of various LNP formulations.
Fig. 17 shows the editing efficiency of targeting TTR in primary mouse hepatocytes after delivery of LNP-169 to cells, in various concentrations, in the presence of mouse serum.
Fig. 18 shows an increase in LNP-binding by ApoE as the amount of ApoE present increases.
Fig. 19 shows the editing efficiency of various LNP formulations wherein the guide RNA was delivered as a DNA expression cassette.
Fig. 20 shows that editing efficiency correlates between primary hepatocyte cultures and *in vivo* liver cells in mice.
Fig. 21 shows the distinctive repair spectrum of editing in the Neuro 2A *in vitro* cell line versus primary mouse hepatocytes.
Fig. 22 shows the similar repair spectrum of editing in primary mouse hepatocytes versus *in vivo* mouse liver cells.
Fig. 23 shows, as a function of time, the plasma concentration of Cas9 mRNA and guide RNA.
Fig. 24 shows, as a function of time, the concentration of Cas9 mRNA and guide RNA in liver tissue.
Fig. 25 shows, as a function of time, the concentration of Cas9 mRNA and guide RNA in spleen tissue.
Fig. 26A shows, as a function of time, the relative concentrations of Cas9 mRNA and guide RNA in plasma and in tissue.
Fig. 26B shows, as a function of time, the concentration of Lipid A in plasma and in tissue.
Fig. 27 shows, as a function of time after administration of an LNP, the change in plasma cytokine levels.
Fig. 28 shows mouse serum TTR levels over time after administration of an LNP.

Fig. 29A shows TTR editing over time in mice after administration of an LNP.
Fig. 29B shows TTR editing and serum TTR levels over time in mice after administration of an LNP.
Fig. 30 shows mouse serum cytokine levels after administration of LNPs containing different mRNA preparations.
Fig. 31 shows mouse serum TTR concentration levels after administration of LNPs containing different mRNA preparations.
Fig. 32 shows TTR editing levels over time in mice after administration of LNPs containing different mRNA preparations.
Fig. 33 shows mouse serum TTR concentration levels after administration of LNPs stored at -80° C or 4° C.
Fig. 34 shows mouse TTR editing levels after administration of LNPs stored at -80° C or 4° C.
Fig. 35 shows mouse serum concentration levels after administration of various formulations.
Fig. 36 shows mouse liver TTR editing levels after administration of various formulations.

## DETAILED DESCRIPTION

The present disclosure describes lipid nanoparticle (LNP) compositions of CRISPR/Cas components (the "cargo") for delivery to a cell and methods for their use. The LNP may contain (i) a CCD lipid, (ii) a neutral lipid, (iii) a helper lipid, and (iv) a stealth lipid. The cargo may include an mRNA encoding a Cas nuclease, such as Cas9, and a guide RNA or a nucleic acid encoding a guide RNA.

## CRISPR/Cas Cargo

The CRISPR/Cas cargo delivered via LNP formulation includes an mRNA molecule encoding a Cas nuclease, allowing for expression of the Cas nuclease in a cell. The cargo further contains one or more guide RNAs or nucleic acids encoding guide RNAs. The cargo may further include a template nucleic acid for repair or recombination.

### Cas Nuclease

One component of the disclosed formulations may be an mRNA encoding a Cas nuclease, also called a Cas nuclease mRNA. The mRNA may be modified for improved stability and/or immunogenicity properties. The modifications may be made to one or more nucleosides within the mRNA. Examples of chemical modifications to mRNA nucleobases include pseudouridine, 1-methyl-pseudouridine, and 5-methyl-cytidine. Additional known modifications to improve stability, expression, and immunogenicity are contemplated. The mRNA encoding a Cas nuclease may be codon optimized for expression in a particular cell type, such as a eukaryotic cell, a mammalian cell, or more specifically, a human cell. The mRNA may encode a human codon optimized Cas9 nuclease or human codon optimized Cpf nuclease as the Cas nuclease. The mRNA may be purified. The mRNA may be purified using a precipation method *(e.g.,* LiCl precipitation, alcohol precipitation, or an equivalent method, *e.g.,* as described herein). The mRNA may be purified using a chromatography-based method, such as an HPLC-based method or an equivalent method *(e.g.,* as described herein). The mRNA may be purified using both a precipitation method *(e.g.,* LiCl precipitation) and an HPLC-based method.

In addition to the coding sequence for a Cas nuclease, the mRNA may comprise a 3' or 5' untranslated region (UTR). The 3' or 5' UTR can be derived from a human gene sequence. Exemplary 3' and 5' UTRs include $\alpha$- and $\beta$-globin, albumin, HSD17B4, and eukaryotic elongation factor 1$\alpha$. In addition, viral-derived 5' and 3' UTRs can also be used and include orthopoxvirus and cytomegalovirus UTR sequences. The mRNA may include a 5' cap, such as m7G(5')ppp(5')N. In addition, this cap may be a cap-0 where nucleotide N does not contain 2'OMe, or cap-1 where nucleotide N contains 2'OMe, or cap-2 where nucleotides N and N+1 contain 2'OMe. This cap may also be of the structure $m_2^{7,3'-O}G(5')N$ as incorporated by the anti-reverse-cap analog (ARCA), and may also include similar cap-0, cap-1, and cap-2, etc., structures. The 5' cap may regulate nuclear export; prevent degradation by exonucleases; promote translation; and promote 5' proximal intron excision. Stabilizing elements for caps include phosphorothioate linkages, boranophosphate modifications, and methylene bridges. In addition, caps may also contain a non-nucleic acid entity that acts as the binding element for eukaryotic translation initiation factor 4E, eIF4E. The mRNA may include a poly(A) tail. This tail may be about 40 to about 300 nucleotides in length. The tail may be about 40 to about 100 nucleotides in length. The tail may be about 100 to about 300 nucleotides in length. The tail may be about 100 to about 300 nucleotides in length. The tail may be about 50 to about 200 nucleotides in length. The tail may be about 50 to about 250 nucleotides in length. The tail may be about 100, 150, or 200 nucleotides in length. The poly(A) tail may contain modifications to prevent exonuclease degradation including phosphorotioate linkages and modifications to the nucleobase. In addition, the poly(A) tail may contain a 3' "cap" which could include modified or non-natural nucleobases or other synthetic moieties.

The mRNAs described herein may comprise at least one element that is capable of modifying the intracellular half-life of the RNA. The half-life of the RNA may be increased. The half-life of the RNA may be decreased. The element may be capable of increasing the stability of the RNA. The element may be capable of decreasing the stability of the RNA.

The element may promote RNA decay. The element may activate translation. The element may be within the 3' UTR of the RNA. For example, the element may be an mRNA decay signal. The element may include a polyadenylation signal (PA). The PA may be in the 3' UTR of the RNA. The RNA may comprise no PA such that it is subject to quicker degradation in the cell after transcription. The element may include at least one AU-rich element (ARE). The element may not necessarily include an ARE. The AREs may be bound by ARE binding proteins (ARE-BPs) in a manner that is dependent upon tissue type, cell type, timing, cellular localization, and environment. The ARE may comprise 50 to 150 nucleotides in length. The ARE may comprise at least one copy of the sequence AUUUA. At least one ARE may be added to the 3' UTR of the RNA. The element may be a Woodchuck Hepatitis Virus (WHV) Posttranscriptional Regulatory Element (WPRE), which creates a tertiary structure to enhance expression from the transcript. The WPRE may be added to the 3' UTR of the RNA. The element may be selected from other RNA sequence motifs that are present in fast- or slow-decaying transcripts. In addition, each element can be used alone. An element can be used in combination with one or more elements.

The nuclease encoded by the delivered mRNA may include a Cas protein from a CRISPR/Cas system. The Cas protein may comprise at least one domain that interacts with a guide RNA ("gRNA"). Additionally, the Cas protein may be directed to a target sequence by a guide RNA. The guide RNA interacts with the Cas protein as well as the target sequence such that it directs binding to the target sequence. The guide RNA may provide the specificity for the targeted cleavage, and the Cas protein may be universal and paired with different guide RNAs to cleave different target sequences. The Cas protein may cleave single or double-stranded DNA. The Cas protein may cleave RNA.

The Cas protein may nick RNA. The Cas protein may comprise at least one DNA binding domain and at least one nuclease domain. The nuclease domain may be heterologous to the DNA binding domain. The CRISPR/Cas system may comprise Class 1 or Class 2 system components, including ribonucleic acid protein complexes. See, *e.g.,* Makarova et al., Nat Rev Microbiol, 13(11): 722-36 (2015); Shmakov et al., Molecular Cell, 60:385-397 (2015). Class 2 CRISPR/Cas systems have single protein effectors. Cas proteins of Types II, V, and VI may be single-protein, RNA-guided endonucleases, herein called "Class 2 Cas nucleases." Class 2 Cas nucleases include, for example, Cas9, Cpfl, C2c1, C2c2, and C2c3 proteins. Cpfl protein, Zetsche et al., Cell, 163: 1-13 (2015), is homologous to Cas9, and contains a RuvC-like nuclease domain. See, *e.g.,* Zetsche, Tables S1 and S3.

The Cas protein may be from a Type-II CRISPR/Cas system, *i.e.,* a Cas9 protein from a CRISPR/Cas9 system, or a Type-V CRISPR/Cas system, *e.g.*, a Cpf1 protein. The Cas protein may be from a Class 2 CRISPR/Cas system, *i.e.,* a single-protein Cas nuclease such as a Cas9 protein or a Cpfl protein. The Class 2 Cas nuclease families of proteins are enzymes with DNA endonuclease activity, and they can be directed to cleave a desired nucleic acid target by designing an appropriate guide RNA, as described further herein.

A Class 2 CRISPR/Cas system component may be from a Type-IIA, Type-IIB, Type-IIC, Type V, or Type VI system. Cas9 and its orthologs are encompassed. Non-limiting exemplary species that the Cas9 protein or other components may be from include *Streptococcus pyogenes, Streptococcus thermophilus, Streptococcus sp., Staphylococcus aureus, Listeria innocua, Lactobacillus gasseri, Francisella novicida, Wolinella succinogenes, Sutterella wadsworthensis, Gamma proteobacterium, Neisseria meningitidis, Campylobacter jejuni, Pasteurella multocida, Fibrobacter succinogene, Rhodospirillum rubrum, Nocardiopsis dassonvillei, Streptomyces pristinaespiralis, Streptomyces viridochromogenes, Streptomyces viridochromogenes, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Lactobacillus buchneri, Treponema denticola, Microscilla marina, Burkholderiales bacterium, Polaromonas naphthalenivorans, Polaromonas sp., Crocosphaera watsonii, Cyanothece sp., Microcystis aeruginosa, Synechococcus sp.,* Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionium, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, Streptococcus pasteurianus, Neisseria cinerea, Campylobacter lari, Parvibaculum lavamentivorans, Corynebacterium diphtheria, or Acaryochloris marina. The Cas9 protein may be from Streptococcus pyogenes. The Cas9 protein may be from Streptococcus thermophilus. The Cas9 protein may be from Staphylococcus aureus. A Cpfl protein may be from Francisella tularensis, Lachnospiraceae bacterium, Butyrivibrio proteoclasticus, Peregrinibacteria bacterium, Parcubacteria bacterium, Smithella, Acidaminococcus, Candidatus Methanoplasma termitum, Eubacterium eligens, Moraxella bovoculi, Leptospira inadai, Porphyromonas crevioricanis, Prevotella disiens, or Porphyromonas macacae. The Cpf1 protein may be from Acidaminococcus or Lachnospiraceae.

A Class 2 Cas nuclease may comprise at least one RuvC-like nuclease domain, such as a Cas9 or Cpfl protein. A Class 2 Cas nuclease may comprise more than one nuclease domain. For example, a Class 2 Cas nuclease may comprise at least one RuvC-like nuclease domain and at least one HNH-like nuclease domain. The Class 2 Cas nuclease may be capable of introducing a DSB in the target sequence. The Class 2 Cas nuclease may be modified to contain only one functional nuclease domain. For example, the Class 2 Cas nuclease may be modified such that one of the nuclease

domains is mutated or fully or partially deleted to reduce its nucleic acid cleavage activity. The Class 2 Cas nuclease may be modified to contain no functional RuvC-like nuclease domain. The Class 2 Cas nuclease, *e.g.* a Cas9 protein, may be modified to contain no functional HNH-like nuclease domain. For Class 2 Cas nucleases in which only one nuclease domain is functional, the Class 2 Cas nuclease may be a nickase that is capable of introducing a single-stranded break (a "nick") into the target sequence. A conserved amino acid within a nuclease domain of the Class 2 Cas nuclease may be substituted to reduce or alter a nuclease activity. The nuclease domain mutation may inactivate DNA cleavage activity. The nuclease domain mutation may inactivate one nuclease domain of the Class 2 Cas nuclease, resulting in a nickase. The nickase may comprise an amino acid substitution in the RuvC-like nuclease domain. Exemplary amino acid substitutions in the RuvC-like nuclease domain include D10A (based on the *S. pyogenes* Cas9 protein, see, *e.g.*, UniProtKB - Q99ZW2 (CAS9_STRP1)). Further exemplary amino acid substitutions include D917A, E1006A, and D1255A (based on the *Francisella novicida* U112 Cpfl (FnCpf1) sequence (UniProtKB - A0Q7Q2 (CPF1_FRATN)). The nickase may comprise an amino acid substitution in the HNH-like nuclease domain. Exemplary amino acid substitutions in the HNH-like nuclease domain include E762A, H840A, N863A, H983A, and D986A (based on the *S. pyogenes* Cas9 protein). Exemplary mutations alter conserved catalytic residues in the nuclease domain and alter nucleolytic activity of the domain. The nuclease system described herein may comprise a nickase and a pair of guide RNAs that are complementary to the sense and antisense strands of the target sequence, respectively. The guide RNAs may direct the nickase to target and introduce a DSB by generating a nick on opposite strands of the target sequence *(i.e.,* double nicking). A chimeric Class 2 Cas nuclease may also be used, where one domain or region of the protein may be replaced by a portion of a different protein. For example, a nuclease domain may be replaced with a domain from a different nuclease such as Fok1. The Class 2 Cas nuclease may be modified to reduce or eliminate nuclease activity. It may be used to bind to and modulate the expression or activity of a DNA sequence.

The Cas protein may be a component of the Cascade complex of a Type-I CRISPR/Cas system. For example, the Cas protein may be a Cas3 protein. The Cas protein may be from a Type-II CRISPR/Cas system. The Cas protein may be from a Type-III CRISPR/Cas system. The Cas protein may be from a Type-IV CRISPR/Cas system. The Cas protein may be from a Type-V CRISPR/Cas system. The Cas protein may be from a Type-VI CRISPR/Cas system. The Cas protein may have an RNA cleavage activity.

The nuclease may be fused with at least one heterologous protein domain. At least one protein domain may be located at the N-terminus, the C-terminus, or in an internal location of the nuclease. The protein domain may facilitate transport of the nuclease into the nucleus of a cell. For example, the protein domain may be a nuclear localization signal (NLS). The nuclease may be fused with 1-10 NLS(s). The nuclease may be fused with 1-5 NLS(s). The nuclease may be fused with one NLS. Where one NLS is used, the NLS may be on the N-terminus or the C-terminus of the nuclease. The nuclease may be fused with more than one NLS. The nuclease may be fused with 2, 3, 4, or 5 NLSs. The nuclease may be fused with two NLSs. In certain circumstances, the two NLSs may be the same (*e.g.*, two SV40 NLSs) or different. The nuclease may be fused to two SV40 NLS sequences at the carboxy terminus. The nuclease may be fused with two NLSs, one on the N-terminus and one on the C-terminus. The nuclease may be fused with 3 NLSs. The nuclease may be fused with no NLS. The NLS may be a monopartite sequence, such as, *e.g.,* the SV40 NLS, PKKKRKV or PKKKRRV. The NLS may be a bipartite sequence, such as the NLS of nucleoplasmin, KRPAATKKAGQAKKKK. A single PKKKRKV NLS may be at the C-terminus of the nuclease.

The protein domain may be capable of modifying the intracellular half-life of the nuclease. The half-life of the nuclease may be increased. The half-life of the nuclease may be reduced. The protein domain may be capable of increasing the stability of the nuclease. The protein domain may be capable of reducing the stability of the nuclease. The protein domain may act as a signal peptide for protein degradation. The protein degradation may be mediated by proteolytic enzymes, such as, for example, proteasomes, lysosomal proteases, or calpain proteases. The protein domain may comprise a PEST sequence. The nuclease may be modified by addition of ubiquitin or a polyubiquitin chain. The ubiquitin may be a ubiquitin-like protein (UBL). Non-limiting examples of ubiquitin-like proteins include small ubiquitin-like modifier (SUMO), ubiquitin cross-reactive protein (UCRP, also known as interferon-stimulated gene-15 (ISG15)), ubiquitin-related modifier-1 (URM1), neuronal-precursor-cell-expressed developmentally downregulated protein-8 (NEDD8, also called Rub1 in *S. cerevisiae),* human leukocyte antigen F-associated (FAT10), autophagy-8 (ATG8) and - 12 (ATG12), Fau ubiquitin-like protein (FUB1), membrane-anchored UBL (MUB), ubiquitin fold-modifier-1 (UFM1), and ubiquitin-like protein-5 (UBL5).

The protein domain may be a marker domain. Non-limiting examples of marker domains include fluorescent proteins, purification tags, epitope tags, and reporter gene sequences. The marker domain may be a fluorescent protein. Non-limiting examples of suitable fluorescent proteins include green fluorescent proteins (*e.g.*, GFP, GFP-2, tagGFP, turboGFP, sfGFP, EGFP, Emerald, Azami Green, Monomeric Azami Green, CopGFP, AceGFP, ZsGreen1 ), yellow fluorescent proteins (*e.g.*, YFP, EYFP, Citrine, Venus, YPet, PhiYFP, ZsYellow1), blue fluorescent proteins (*e.g.*, EBFP, EBFP2, Azurite, mKalamal, GFPuv, Sapphire, T-sapphire,), cyan fluorescent proteins (*e.g.*, ECFP, Cerulean, CyPet, AmCyan1, Midoriishi-Cyan), red fluorescent proteins (*e.g.*, mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry,

Jred), and orange fluorescent proteins (mOrange, mKO, Kusabira-Orange, Monomeric Kusabira-Orange, mTangerine, tdTomato) or any other suitable fluorescent protein. The marker domain may be a purification tag and/or an epitope tag. Non-limiting exemplary tags include glutathione-S-transferase (GST), chitin binding protein (CBP), maltose binding protein (MBP), thioredoxin (TRX), poly(NANP), tandem affinity purification (TAP) tag, myc, AcV5, AU1, AU5, E, ECS, E2, FLAG, HA, nus, Softag 1, Softag 3, Strep, SBP, Glu-Glu, HSV, KT3, S, S1, T7, V5, VSV-G, 6xHis, 8xHis, biotin carboxyl carrier protein (BCCP), poly-His, and calmodulin. Non-limiting exemplary reporter genes include glutathione-S-transferase (GST), horseradish peroxidase (HRP), chloramphenicol acetyltransferase (CAT), beta-galactosidase, beta-glucuronidase, luciferase, or fluorescent proteins.

The protein domain may target the nuclease to a specific organelle, cell type, tissue, or organ. The protein domain may target the nuclease to mitochondria.

The protein domain may be an effector domain. When the nuclease is directed to its target sequence, *e.g.*, when a Cas9 protein is directed to a target sequence by a guide RNA, the effector domain may modify or affect the target sequence. The effector domain may be chosen from a nucleic acid binding domain, a nuclease domain, an epigenetic modification domain, a transcriptional activation domain, a methylation domain, or a transcriptional repressor domain. The DNA modification domain may be a methylation domain, such as a demethylation or methyltransferase domain. The effector domain may be a DNA modification domain, such as a base-editing domain. The DNA modification domain may be a nucleic acid editing domain that introduces a specific modification into the DNA, such as a deaminase domain. *See* WO 2015/089406; US 2016/0304846. The nucleic acid editing domains, deaminase domains, and Cas9 variants are described in WO 2015/089406 and US 2016/0304846.

## Guide RNA

The cargo for the LNP formulation may include at least one guide RNA. The guide RNA may guide the Class 2 Cas nuclease to a target sequence on a target nucleic acid molecule, where the guide RNA hybridizes with and the Cas nuclease cleaves or modulates the target sequence. A guide RNA may bind with and provides specificity of cleavage by a Class 2 nuclease. The guide RNA and the Cas protein may form a ribonucleoprotein (RNP), *e.g.,* a CRISPR/Cas complex. The CRISPR complex may be a Type-II CRISPR/Cas9 complex. The CRISPR/Cas complex may be a Type-V CRISPR/-Cas complex, such as a Cpf1/guide RNA complex. The Cas nuclease may be a single-protein Cas nuclease, *e.g.* a Cas9 protein or a Cpf1 protein. The guide RNA may target cleavage by a Cas9 protein.

A guide RNA for a CRISPR/Cas9 nuclease system comprises a CRISPR RNA (crRNA) and a tracr RNA (tracr). The crRNA may comprise a targeting sequence that is complementary to and hybridizes with the target sequence on the target nucleic acid molecule. The crRNA may also comprise a flagpole that is complementary to and hybridizes with a portion of the tracrRNA. The crRNA may parallel the structure of a naturally occurring crRNA transcribed from a CRISPR locus of a bacteria, where the targeting sequence acts as the spacer of the CRISPR/Cas9 system, and the flagpole corresponds to a portion of a repeat sequence flanking the spacers on the CRISPR locus. The guide RNA may target any sequence of interest via the targeting sequence of the crRNA. The degree of complementarity between the targeting sequence of the guide RNA and the target sequence on the target nucleic acid molecule may be about 60%, 65%, 70%, 75%, 80%, 85%, 90%, 95%, 97%, 98%, 99%, or 100%. The targeting sequence of the guide RNA and the target sequence on the target nucleic acid molecule may be 100% complementary. The targeting sequence of the guide RNA and the target sequence on the target nucleic acid molecule may contain at least one mismatch. For example, the targeting sequence of the guide RNA and the target sequence on the target nucleic acid molecule may contain 1, 2, 3, 4, 5, 6, 7, 8, 9, or 10 mismatches. The targeting sequence of the guide RNA and the target sequence on the target nucleic acid molecule may contain 1-6 mismatches. The targeting sequence of the guide RNA and the target sequence on the target nucleic acid molecule may contain 5 or 6 mismatches. The length of the targeting sequence may depend on the CRISPR/Cas system and components used. For example, different Cas proteins from different bacterial species have varying optimal targeting sequence lengths. Accordingly, the targeting sequence may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length. The targeting sequence may comprise 18-24 nucleotides in length. The targeting sequence may comprise 19-21 nucleotides in length. The targeting sequence may comprise 20 nucleotides in length.

The flagpole may comprise any sequence with sufficient complementarity with a tracr RNA to promote the formation of a functional CRISPR/Cas complex. The flagpole may comprise all or a portion of the sequence (also called a "tag" or "handle") of a naturally-occurring crRNA that is complementary to the tracr RNA in the same CRISPR/Cas system. The flagpole may comprise all or a portion of a repeat sequence from a naturally-occurring CRISPR/Cas system. The flagpole may comprise a truncated or modified tag or handle sequence. The degree of complementarity between the tracr RNA and the portion of the flagpole that hybridizes with the tracr RNA along the length of the shorter of the two sequences may be about 40%, 50%, 60%, 70%, 80%, or higher, but lower than 100%. Without being bound by theory, it may be that, the tracr RNA and the portion of the flagpole that hybridizes with the tracr RNA are not 100% complementary along the length of the shorter of the two sequences because of the presence of one or more bulge structures on the tracr and/or

wobble base pairing between the tracr and the flagpole. The length of the flagpole may depend on the CRISPR/Cas system or the tracr RNA used. For example, the flagpole may comprise 10-50 nucleotides, or more than 50 nucleotides in length. The flagpole may comprise 15-40 nucleotides in length. The flagpole may comprise 20-30 nucleotides in length. The flagpole may comprise 22 nucleotides in length. When a dual guide RNA is used, for example, the length of the flagpole may have no upper limit.

The tracr RNA may comprise all or a portion of a wild-type tracr RNA sequence from a naturally-occurring CRISPR/Cas system. The tracr RNA may comprise a truncated or modified variant of the wild-type tracr RNA. The length of the tracr RNA may depend on the CRISPR/Cas system used. The tracr RNA may comprise 5, 6, 7, 8, 9, 10, 11, 12, 13, 14, 15, 16, 17, 18, 19, 20, 25, 30, 40, 50, 60, 70, 80, 90, 100, or more than 100 nucleotides in length. The tracr may be at least 26 nucleotides in length. The tracr may be at least 40 nucleotides in length. The tracr RNA may comprise certain secondary structures, such as, *e.g.,* one or more hairpins or stem-loop structures, or one or more bulge structures.

The guide RNA may comprise two RNA molecules and is referred to herein as a "dual guide RNA" or "dgRNA". The dgRNA may comprise a first RNA molecule comprising a crRNA, and a second RNA molecule comprising a tracr RNA. The first and second RNA molecules may form a RNA duplex via the base pairing between the flagpole on the crRNA and the tracr RNA.

The guide RNA may comprise a single RNA molecule and is referred to herein as a "single guide RNA" or "sgRNA". The sgRNA may comprise a crRNA covalently linked to a tracr RNA. The crRNA and the tracr RNA may be covalently linked via a linker. The single-molecule guide RNA may comprise a stem-loop structure via the base pairing between the flagpole on the crRNA and the tracr RNA. The sgRNA may be a "Cas9 sgRNA" capable of mediating RNA-guided DNA cleavage by a Cas9 protein. The sgRNA may be a "Cpfl sgRNA" capable of mediating RNA-guided DNA cleavage by a Cpf1 protein. The guide RNA may comprise a crRNA and tracr RNA sufficient for forming an active complex with a Cas9 protein and mediating RNA-guided DNA cleavage. The guide RNA may comprise a crRNA sufficient for forming an active complex with a Cpfl protein and mediating RNA-guided DNA cleavage. *See* Zetsche 2015.

Also provided herein are nucleic acids, *e.g.*, expression cassettes, encoding the guide RNA described herein. A "guide RNA nucleic acid" is used herein to refer to a guide RNA (*e.g.* an sgRNA or a dgRNA) and a guide RNA expression cassette, which is a nucleic acid that encodes one or more guide RNAs.

The nucleic acid may be a DNA molecule. The nucleic acid may comprise a nucleotide sequence encoding a crRNA. The nucleotide sequence encoding the crRNA may comprise a targeting sequence flanked by all or a portion of a repeat sequence from a naturally-occurring CRISPR/Cas system. The nucleic acid may comprise a nucleotide sequence encoding a tracr RNA. The crRNA and the tracr RNA may be encoded by two separate nucleic acids. The crRNA and the tracr RNA may be encoded by a single nucleic acid. The crRNA and the tracr RNA may be encoded by opposite strands of a single nucleic acid. The crRNA and the tracr RNA may be encoded by the same strand of a single nucleic acid. The expression cassette may encode an sgRNA. The expression cassette may encode a Cas9 nuclease sgRNA. The expression cassette may encode a Cpfl nuclease sgRNA.

The nucleotide sequence encoding the guide RNA may be operably linked to at least one transcriptional or regulatory control sequence, such as a promoter, a 3' UTR, or a 5' UTR. In one example, the promoter may be a tRNA promoter, *e.g.*, $tRNA^{Lys3}$, or a tRNA chimera. *See* Mefferd et al., RNA. 2015 21:1683-9; Scherer et al., Nucleic Acids Res. 2007 35: 2620-2628. The promoter may be recognized by RNA polymerase III (Pol III). Non-limiting examples of Pol III promoters also include U6 and H1 promoters. The nucleotide sequence encoding the guide RNA may be operably linked to a mouse or human U6 promoter. The expression cassette may be a modified nucleic acid. The expression cassette may include a modified nucleoside or nucleotide. The expression cassette may include a 5' end modification, for example a modified nucleoside or nucleotide to stabilize and prevent integration of the expression cassette. The expression cassette may comprise a double-stranded DNA having a 5' end modification on each strand. The expression cassette may include an inverted dideoxy-T or an inverted abasic nucleoside or nucleotide as the 5' end modification. The expression cassette may include a label such as biotin, desthiobioten-TEG, digoxigenin, and fluorescent markers, including, for example, FAM, ROX, TAMRA, and AlexaFluor.

More than one guide RNA can be used with a CRISPR/Cas nuclease system. Each guide RNA may contain a different targeting sequence, such that the CRISPR/Cas system cleaves more than one target sequence. One or more guide RNAs may have the same or differing properties such as activity or stability within a CRISPR/Cas complex. Where more than one guide RNA is used, each guide RNA can be encoded on the same or on different expression cassettes. The promoters used to drive expression of the more than one guide RNA may be the same or different.

Chemically Modified RNAs

Modified nucleosides or nucleotides can be present in a guide RNA or mRNA. A guide RNA or Cas nuclease encoding mRNA comprising one or more modified nucleosides or nucleotides is called a "modified" RNA to describe the presence of one or more non-naturally and/or naturally occurring components or configurations that are used instead of or in addition to the canonical A, G, C, and U residues. A modified RNA may be synthesized with a non-canonical nucleoside

or nucleotide, here called "modified." Modified nucleosides and nucleotides can include one or more of: (i) alteration, *e.g.*, replacement, of one or both of the non-linking phosphate oxygens and/or of one or more of the linking phosphate oxygens in the phosphodiester backbone linkage (an exemplary backbone modification); (ii) alteration, *e.g.*, replacement, of a constituent of the ribose sugar, *e.g.,* of the 2' hydroxyl on the ribose sugar (an exemplary sugar modification); (iii) wholesale replacement of the phosphate moiety with "dephospho" linkers (an exemplary backbone modification); (iv) modification or replacement of a naturally occurring nucleobase, including with a non-canonical nucleobase (an exemplary base modification); (v) replacement or modification of the ribose-phosphate backbone (an exemplary backbone modification); (vi) modification of the 3' end or 5' end of the oligonucleotide, *e.g.*, removal, modification or replacement of a terminal phosphate group or conjugation of a moiety, cap or linker (such 3' or 5' cap modifications may comprise a sugar and/or backbone modification); and (vii) modification or replacement of the sugar (an exemplary sugar modification).

The modifications listed above can be combined to provide modified RNAs comprising nucleosides and nucleotides (collectively "residues") that can have two, three, four, or more modifications. For example, a modified residue can have a modified sugar and a modified nucleobase. Every base of a gRNA may be modified, *e.g.,* all bases have a modified phosphate group, such as a phosphorothioate group. All, or substantially all, of the phosphate groups of an sgRNA molecule may be replaced with phosphorothioate groups. Modified RNAs may comprise at least one modified residue at or near the 5' end of the RNA. Modified RNAs may comprise at least one modified residue at or near the 3' end of the RNA.

Modified residues may be incorporated into a guide RNA. Modified residues may be incorporated into an mRNA. The guide RNA may comprise one, two, three or more modified residues. The guide RNA may comprise one, two, three or more modified residues at each of the 5' and the 3' ends of the guide RNA. The mRNA may comprise 5, 10, 15, 50, 100, 200, 300, 400, 500, 600, 700, 800, 900, 1000, or more modified residues. Of the positions in a modified guide RNA or mRNA, at least 5% *(e.g.,* at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) may be modified nucleosides or nucleotides. Unmodified nucleic acids can be prone to degradation by, *e.g.,* cellular nucleases. For example, nucleases can hydrolyze nucleic acid phosphodiester bonds. Accordingly, in one aspect the guide RNAs described herein can contain one or more modified nucleosides or nucleotides, *e.g.*, to introduce stability toward nucleases. The mRNAs described herein may contain one or more modified nucleosides or nucleotides, *e.g.*, to introduce stability toward nucleases. The modified RNA molecules described herein can exhibit a reduced innate immune response when introduced into a population of cells, both *in vivo* and *ex vivo.* The term "innate immune response" includes a cellular response to exogenous nucleic acids, including single stranded nucleic acids, which involves the induction of cytokine expression and release, particularly the interferons, and cell death.

The phosphate group of a modified residue can be modified by replacing one or more of the oxygens with a different substituent. Further, the modified residue, *e.g.*, modified residue present in a modified nucleic acid, can include the wholesale replacement of an unmodified phosphate moiety with a modified phosphate group as described herein. The backbone modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. The phosphorous atom in an unmodified phosphate group is achiral. However, replacement of one of the non-bridging oxygens with one of the above atoms or groups of atoms can render the phosphorous atom chiral. The stereogenic phosphorous atom can possess either the "R" configuration (herein Rp) or the "S" configuration (herein Sp). The backbone can also be modified by replacement of a bridging oxygen, (*i.e.*, the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

The phosphate group can be replaced by non-phosphorus containing connectors in certain backbone modifications. The charged phosphate group can be replaced by a neutral moiety. Examples of moieties which can replace the phosphate group can include, without limitation, *e.g.*, methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

Scaffolds that can mimic nucleic acids can also be constructed wherein the phosphate linker and ribose sugar are replaced by nuclease resistant nucleoside or nucleotide surrogates. Such modifications may comprise backbone and sugar modifications. The nucleobases can be tethered by a surrogate backbone. Examples can include, without limitation, the morpholino, cyclobutyl, pyrrolidine and peptide nucleic acid (PNA) nucleoside surrogates.

The modified nucleosides and modified nucleotides can include one or more modifications to the sugar group, *i.e.* at sugar modification. For example, the 2' hydroxyl group (OH) can be modified, *e.g.* replaced with a number of different "oxy" or "deoxy" substituents. Modifications to the 2' hydroxyl group can enhance the stability of the nucleic acid since the

hydroxyl can no longer be deprotonated to form a 2'-alkoxide ion.

Examples of 2' hydroxyl group modifications can include alkoxy or aryloxy (OR, wherein "R" can be, *e.g.,* alkyl, cycloalkyl, aryl, aralkyl, heteroaryl or a sugar); polyethyleneglycols (PEG), $O(CH_2CH_2O)_nCH_2CH_2OR$ wherein R can be, *e.g.,* H or optionally substituted alkyl, and n can be an integer from 0 to 20 *(e.g.,* from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). The 2' hydroxyl group modification can be 2'-O-Me. The 2' hydroxyl group modification can be a 2'-fluoro modification, which replaces the 2' hydroxyl group with a fluoride. The 2' hydroxyl group modification can include "locked" nucleic acids (LNA) in which the 2' hydroxyl can be connected, *e.g.*, by a $C_{1-6}$ alkylene or $C_{1-6}$ heteroalkylene bridge, to the 4' carbon of the same ribose sugar, where exemplary bridges can include methylene, propylene, ether, or amino bridges; O-amino (wherein amino can be, *e.g.,* $NH_2$; alkylamino, dialkylamino, heterocyclyl, arylamino, diarylamino, heteroarylamino, or diheteroarylamino, ethylenediamine, or polyamino) and aminoalkoxy, $O(CH_2)_n$-amino, (wherein amino can be, *e.g.,* $NH_2$; alkylamino, dialkylamino, heterocyclyl, arylamino, diarylamino, heteroarylamino, or diheteroarylamino, ethylenediamine, or polyamino). The 2' hydroxyl group modification can include "unlocked" nucleic acids (UNA) in which the ribose ring lacks the C2'-C3' bond. The 2' hydroxyl group modification can include the methoxyethyl group (MOE), ($OCH_2CH_2OCH_3$, *e.g.,* a PEG derivative).

"Deoxy" 2' modifications can include hydrogen (*i.e.* deoxyribose sugars, *e.g.,* at the overhang portions of partially dsRNA); halo (*e.g.*, bromo, chloro, fluoro, or iodo); amino (wherein amino can be, *e.g.,* $-NH_2$, alkylamino, dialkylamino, heterocyclyl, arylamino, diarylamino, heteroarylamino, diheteroarylamino, or amino acid); $NH(CH_2CH_2NH)_nCH2CH_2$-amino (wherein amino can be, *e.g.,* as described herein), - NHC(O)R (wherein R can be, *e.g.,* alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.,* an amino as described herein. The sugar modification can comprise a sugar group which may also contain one or more carbons that possess the opposite stereochemical configuration than that of the corresponding carbon in ribose. Thus, a modified nucleic acid can include nucleotides containing *e.g.*, arabinose, as the sugar. The modified nucleic acids can also include abasic sugars. These abasic sugars can also be further modified at one or more of the constituent sugar atoms. The modified nucleic acids can also include one or more sugars that are in the L form, *e.g.* L- nucleosides.

The modified nucleosides and modified nucleotides described herein, which can be incorporated into a modified nucleic acid, can include a modified base, also called a nucleobase. Examples of nucleobases include, but are not limited to, adenine (A), guanine (G), cytosine (C), and uracil (U). These nucleobases can be modified or wholly replaced to provide modified residues that can be incorporated into modified nucleic acids. The nucleobase of the nucleotide can be independently selected from a purine, a pyrimidine, a purine analog, or pyrimidine analog. The nucleobase can include, for example, naturally-occurring and synthetic derivatives of a base.

When employing a dual guide RNA, each of the crRNA and the tracr RNA may contain modifications. Such modifications may be at one or both ends of the crRNA and/or tracr RNA. When using an sgRNA, one or more residues at one or both ends of the sgRNA may be chemically modified, or the entire sgRNA may be chemically modified. Thus they may comprise a 5' end modification. Thus they may comprise a 3' end modification. One or more or all of the nucleotides in single stranded overhang of a guide RNA molecule may be deoxynucleotides. The modified mRNA may contain 5' end and/or 3' end modifications.

## Template Nucleic Acid

The formulations disclosed herein may include a template nucleic acid. The template may be used to alter or insert a nucleic acid sequence at or near a target site for a Cas nuclease.

The template may be used in homologous recombination. The homologous recombination may result in the integration of the template sequence or a portion of the template sequence into the target nucleic acid molecule. A single template may be provided. Alternatively, two or more templates may be provided such that homologous recombination may occur at two or more target sites. For example, different templates may be provided to repair a single gene in a cell, or two different genes in a cell. Multiple copies of at least one template may be provided to a cell. The different templates may be provided in independent copy numbers or independent amounts.

The template may be used in homology-directed repair, which involves DNA strand invasion at the site of the cleavage in the nucleic acid. The homology-directed repair may result in including the template sequence in the edited target nucleic acid molecule. A single template may be provided. Alternatively, two or more templates having different sequences may be used at two or more sites by homology-directed repair. For example, different templates may be provided to repair a single gene in a cell, or two different genes in a cell. Multiple copies of at least one template may be provided to a cell. The different templates may be provided in independent copy numbers or independent amounts.

The template may be used in gene editing mediated by non-homologous end joining. It may be that the template sequence has no similarity to the nucleic acid sequence near the cleavage site. The template or a portion of the template sequence may be incorporated. A single template may be provided. Two or more templates having different sequences

may be inserted at two or more sites by non-homologous end joining. For example, different templates may be provided to insert a single template in a cell, or two different templates in a cell. The different templates may be provided in independent copy numbers. The template may include flanking inverted terminal repeat (ITR) sequences.

The template sequence may correspond to an endogenous sequence of a target cell. As used herein, the term "endogenous sequence" refers to a sequence that is native to the cell. The term "exogenous sequence" refers to a sequence that is not native to a cell, or a sequence whose native location in the genome of the cell is in a different location. The endogenous sequence may be a genomic sequence of the cell. The endogenous sequence may be a chromosomal or extrachromosomal sequence. The endogenous sequence may be a plasmid sequence of the cell. The template sequence may be substantially identical to a portion of the endogenous sequence in a cell at or near the cleavage site, but comprise at least one nucleotide change. The repair of the cleaved target nucleic acid molecule with the template may result in a mutation comprising an insertion, deletion, or substitution of one or more nucleotides of the target nucleic acid molecule. The mutation may result in one or more amino acid changes in a protein expressed from a gene comprising the target sequence. The mutation may result in one or more nucleotide changes in an RNA expressed from the target gene. The mutation may alter the expression level of the target gene. The mutation may result in increased or decreased expression of the target gene. The mutation may result in gene knockdown. The mutation may result in gene knockout. The mutation may result in restored gene function. The repair of the cleaved target nucleic acid molecule with the template may result in a change in an exon sequence, an intron sequence, a regulatory sequence, a transcriptional control sequence, a translational control sequence, a splicing site, or a non-coding sequence of the target gene.

The template sequence may comprise an exogenous sequence. The exogenous sequence may comprise a protein or RNA coding sequence operably linked to an exogenous promoter sequence such that, upon integration of the exogenous sequence into the target nucleic acid molecule, the cell is capable of expressing the protein or RNA encoded by the integrated sequence. Upon integration of the exogenous sequence into the target nucleic acid molecule, the expression of the integrated sequence may be regulated by an endogenous promoter sequence. The exogenous sequence may be a chromosomal or extrachromosomal sequence. The exogenous sequence may provide a cDNA sequence encoding a protein or a portion of the protein. The exogenous sequence may comprise an exon sequence, an intron sequence, a regulatory sequence, a transcriptional control sequence, a translational control sequence, a splicing site, or a non-coding sequence. The integration of the exogenous sequence may result in restored gene function. The integration of the exogenous sequence may result in a gene knock-in. The integration of the exogenous sequence may result in a gene knock-out.

The template may be of any suitable length. The template may comprise 10, 15, 20, 25, 50, 75, 100, 150, 200, 500, 1000, 1500, 2000, 2500, 3000, 3500, 4000, 4500, 5000, 5500, 6000, or more nucleotides in length. The template may be a single-stranded nucleic acid. The template can be double-stranded or partially double-stranded nucleic acid. The single stranded template may be 20, 30, 40, 50, 75, 100, 125, 150, 175, or 200 nucleotides in length. The template may comprise a nucleotide sequence that is complementary to a portion of the target nucleic acid molecule comprising the target sequence (*i.e.,* a "homology arm"). The template may comprise a homology arm that is complementary to the sequence located upstream or downstream of the cleavage site on the target nucleic acid molecule. The template may comprise a first homology arm and a second homology arm (also called a first and second nucleotide sequence) that are complementary to sequences located upstream and downstream of the cleavage site, respectively. Where a template contains two homology arms, each arm can be the same length or different lengths, and the sequence between the homology arms can be substantially similar or identical to the target sequence between the homology arms, or it can be entirely unrelated. The degree of complementarity between the first nucleotide sequence on the template and the sequence upstream of the cleavage site, and between the second nucleotide sequence on the template and the sequence downstream of the cleavage site, may permit homologous recombination, such as, *e.g.*, high-fidelity homologous recombination, between the template and the target nucleic acid molecule. The degree of complementarity may be about 50%, 55%, 60%, 65%, 70%, 75%, 80%, 85%, 90%, 95%, 97%, 98%, 99%, or 100%. The degree of complementarity may be about 95%, 97%, 98%, 99%, or 100%. The degree of complementarity may be at least 98%, 99%, or 100%. The degree of complementarity may be 100%.

The template may contain ssDNA or dsDNA containing flanking invert-terminal repeat (ITR) sequences. The template may be supplied as a plasmid, minicircle, nanocircle, or PCR product.

Purification of Nucleic Acids

The nucleic acid may be purified. The nucleic acid may be purified using a precipation method *(e.g.,* LiCl precipitation, alcohol precipitation, or an equivalent method, *e.g.,* as described herein). The nucleic acid may be purified using a chromatography-based method, such as an HPLC-based method or an equivalent method *(e.g.,* as described herein). The nucleic may be purified using both a precipitation method *(e.g.,* LiCl precipitation) and an HPLC-based method.

Target Sequences

A CRISPR/Cas system of the present disclosure may be directed to and cleave a target sequence on a target nucleic acid molecule. For example, the target sequence may be recognized and cleaved by the Cas nuclease. A Class 2 Cas nuclease may be directed by a guide RNA to a target sequence of a target nucleic acid molecule, where the guide RNA hybridizes with and the Cas protein cleaves the target sequence. The guide RNA may hybridize with and a Cas protein cleave the target sequence comprising its cognate PAM. The target sequence may be complementary to the targeting sequence of the guide RNA. The degree of complementarity between a targeting sequence of a guide RNA and the portion of the corresponding target sequence that hybridizes to the guide RNA may be about 50%, 55%, 60%, 65%, 70%, 75%, 80%, 85%, 90%, 95%, 97%, 98%, 99%, or 100%. The homology region of the target may be adjacent to a cognate PAM sequence. The target sequence may comprise a sequence 100% complementary with the targeting sequence of the guide RNA. The target sequence may comprise at least one mismatch, deletion, or insertion, as compared to the targeting sequence of the guide RNA. For example, the target sequence and the targeting sequence of the guide RNA may contain 1, 2, 3, 4, 5, 6, 7, 8, 9, or 10 mismatches, optionally in a portion of the target sequence adjacent to the PAM. The target sequence and the targeting sequence of the guide RNA may contain 1-9 mismatches. The target sequence and the targeting sequence of the guide RNA may contain 3-6 mismatches. The target sequence and the targeting sequence of the guide RNA may contain 5 or 6 mismatches.

The length of the target sequence may depend on the nuclease system used. For example, the targeting sequence of a guide RNA for a CRISPR/Cas system may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length and the target sequence is a corresponding length, optionally adjacent to a PAM sequence. The target sequence may comprise 15-24 nucleotides in length. The target sequence may comprise 17-21 nucleotides in length. The target sequence may comprise 20 nucleotides in length. When nickases are used, the target sequence may comprise a pair of target sequences recognized by a pair of nickases that cleave opposite strands of the DNA molecule. The target sequence may comprise a pair of target sequences recognized by a pair of nickases that cleave the same strands of the DNA molecule. The target sequence may comprise a part of target sequences recognized by one or more Cas nucleases.

The target nucleic acid molecule may be any DNA or RNA molecule that is endogenous or exogenous to a cell. The target nucleic acid molecule may be an episomal DNA, a plasmid, a genomic DNA, viral genome, mitochondrial DNA, or a chromosome from a cell or in the cell. The target sequence of the target nucleic acid molecule may be a genomic sequence from a cell or in a cell. The cell may be a mammalian cell. The cell may be a rodent cell. The cell may be a human cell. The cell may be a liver cell. The cell may be a human liver cell. The liver cell may be a hepatocyte. The hepatocyte may be a human hepatocyte. The liver cell may be a stem cell. The human liver cell may be a liver sinusoidal endothelial cell (LSEC). The human liver cell may be a Kupffer cell.

The human liver cell may be a hepatic stellate cell. The human liver cell may be a tumor cell. The cell may comprise ApoE-binding receptors. The human liver cell may be a liver stem cell. *See, e.g.,* Wang, et al. Nature, 2015; Font-Burgada, et al. Cell, 2015, 162:766-799.

The target sequence may be a viral sequence. The target sequence may be a pathogen sequence. The target sequence may be a synthesized sequence. The target sequence may be a chromosomal sequence. The target sequence may comprise a translocation junction, *e.g.,* a translocation associated with a cancer. The target sequence may be on a eukaryotic chromosome, such as a human chromosome. The target sequence is a liver-specific sequence, in that it is expressed in liver cells.

The target sequence may be located in a coding sequence of a gene, an intron sequence of a gene, a regulatory sequence, a transcriptional control sequence of a gene, a translational control sequence of a gene, a splicing site or a non-coding sequence between genes. The gene may be a protein coding gene. The gene may be a non-coding RNA gene. The target sequence may comprise all or a portion of a disease-associated gene. In certain cases, the gene may be expressed in liver.

The target sequence may be located in a non-genic functional site in the genome that controls aspects of chromatin organization, such as a scaffold site or locus control region.

With respect to a Cas nuclease, such as a Class 2 Cas nuclease, the target sequence may be adjacent to a protospacer adjacent motif ("PAM"). The PAM may be adjacent to or within 1, 2, 3, or 4, nucleotides of the 3' end of the target sequence. The length and the sequence of the PAM may depend on the Cas protein used. For example, the PAM may be selected from a consensus or a particular PAM sequence for a specific Cas9 protein or Cas9 ortholog, including those disclosed in Figure 1 of Ran et al., Nature, 520: 186-191 (2015), and Figure S5 of Zetsche 2015. The PAM may be 2, 3, 4, 5, 6, 7, 8, 9, or 10 nucleotides in length. Non-limiting exemplary PAM sequences include NGG, NGGNG, NG, NAAAAN, NNAAAAW, NNNNACA, GNNNCNNA, TTN, and NNNNGATT (wherein N is defined as any nucleotide, and W is defined as either A or T). The PAM sequence may be NGG. The PAM sequence may be NGGNG. The PAM sequence may be TTN. The PAM sequence may be NNAAAAW.

Lipid Formulation

Disclosed herein are various LNP formulations for CRISPR/Cas cargoes. Such LNP formulations may include a CCD lipid, along with a helper lipid, a neutral lipid, and a stealth lipid. By "lipid nanoparticle" is meant a particle that comprises a plurality of (*i.e.* more than one) lipid molecules physically associated with each other by intermolecular forces. The LNPs may be, *e.g.*, microspheres (including unilamellar and multilamellar vesicles, *e.g.*, "liposomes"-lamellar phase lipid bilayers that may be substantially spherical-and may comprise an aqueous core, *e.g.*, comprising a substantial portion of RNA molecules), a dispersed phase in an emulsion, micelles, or an internal phase in a suspension. Emulsions, micelles, and suspensions may be suitable compositions for local and/or topical delivery.

The LNP compositions provided herein are preferentially taken up by liver cells (*e.g.,* hepatocytes). Moreover, the LNP compositions are biodegradable, in that they do not accumulate to cytotoxic levels *in vivo* at a therapeutically effective dose. Thus some LNP compositions do not cause an innate immune response that leads to substantial adverse effects at a therapeutic dose level. Some LNP compositions provided herein do not cause toxicity at a therapeutic dose level. The LNP compositions specifically bind to apolipoproteins such as apolipoprotein E (ApoE) in the blood. Apolipoproteins are proteins circulating in plasma that are key in regulating lipid transport. ApoE represents one class of apolipoproteins which interacts with cell surface heparin sulfate proteoglycans in the liver during the uptake of lipoprotein. (See *e.g.,* Scherphof and Kamps, The role of hepatocytes in the clearance of liposomes from the blood circulation. Prog Lipid Res. 2001 May;40(3):149-66).

CCD Lipids

Lipid compositions for the delivery of biologically active agents can be adjusted to preferentially target a liver cell or organ. Certain lipid compositions preferentially target apolipoprotein E (ApoE)-binding cells, such as cells expressing an ApoE receptor. Lipid compositions for delivery of CRISPR/Cas mRNA and guide RNA components to a liver cell comprise a CCD Lipid.

The CCD lipid may be Lipid A, which is (9Z,12Z)-3-((4,4-bis(octyloxy)butanoyl)oxy)-2-((((3-(diethylamino)propoxy)carbonyl)oxy)methyl)propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate. Lipid A can be depicted as:

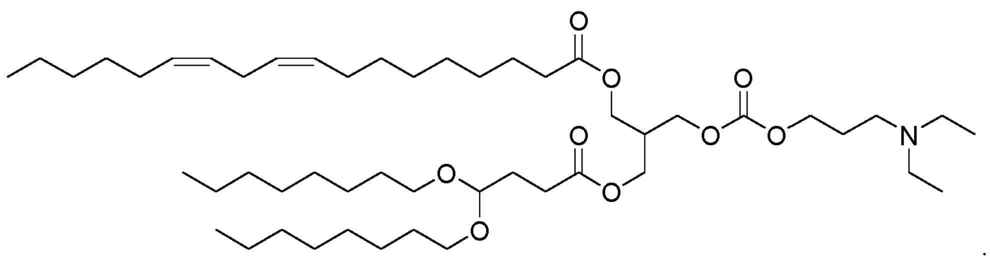

Lipid A may be synthesized according to WO2015/095340 *(e.g.,* pp. 84-86).

The CCD lipid may be Lipid B, which is ((5-((dimethylamino)methyl)-1,3-phenylene)bis(oxy))bis(octane-8,1-diyl) bis(decanoate), also called ((5-((dimethylamino)methyl)-1,3-phenylene)bis(oxy))bis(octane-8,1-diyl) bis(decanoate). Lipid B can be depicted as:

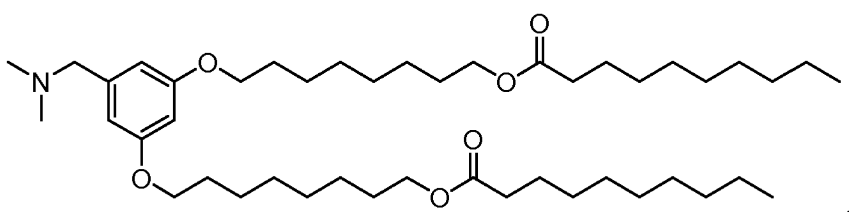

Lipid B may be synthesized according to WO2014/136086 (*e.g.*, pp. 107-09). The CCD lipid may be Lipid C, which is 2-((4-(((3-(dimethylamino)propoxy)carbonyl)oxy)hexadecanoyl)oxy)propane-1,3-diyl (9Z,9'Z,12Z,12'Z)-bis(octadeca-9,12-dienoate). Lipid C can be depicted as:

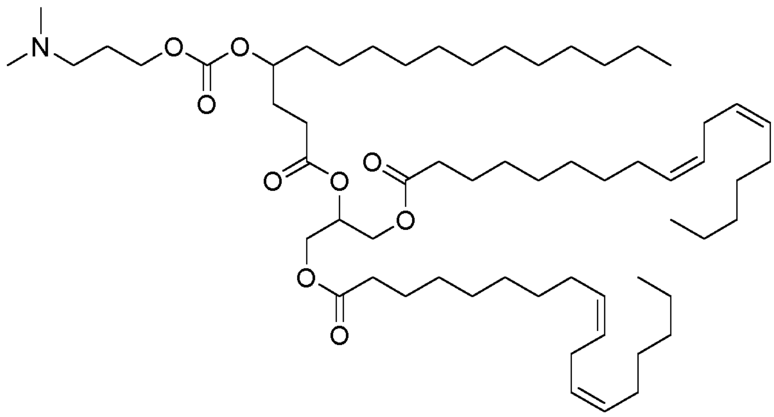

The CCD lipid may be Lipid D, which is 3-(((3-(dimethylamino)propoxy)carbonyl)oxy)-13-(octanoyloxy)tridecyl 3-octylundecanoate. Lipid D can be depicted as:

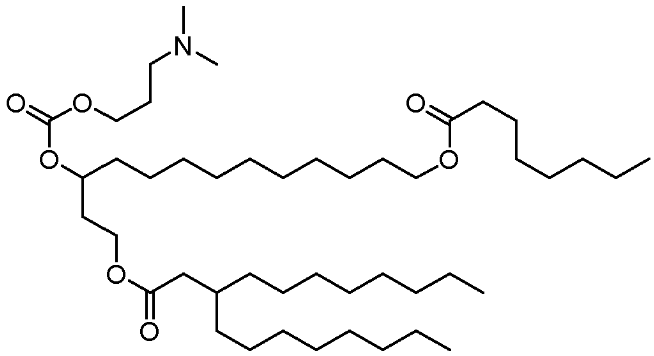

Lipid C and Lipid D may be synthesized according to WO2015/095340.

The CCD lipid can also be an equivalent to Lipid A, Lipid B, Lipid C, or Lipid D. The CCD lipid may be an equivalent to Lipid A or an equivalent to Lipid B.

CCD lipids suitable for use in the LNPs described herein are biodegradable *in vivo.* The CCD lipids have low toxicity *(e.g.,* are tolerated in animal models without adverse effect in amounts of greater than or equal to 10 mg/kg). LNPs comprising a CCD lipid may include those where at least 75% of the CCD lipid is cleared from the plasma within 8, 10, 12, 24, or 48 hours, or 3, 4, 5, 6, 7, or 10 days. LNPs comprising a CCD lipid may include those where at least 50% of the mRNA or guide RNA is cleared from the plasma within 8, 10, 12, 24, or 48 hours, or 3, 4, 5, 6, 7, or 10 days. LNPs comprising a CCD lipid may include those where at least 50% of the LNP is cleared from the plasma within 8, 10, 12, 24, or 48 hours, or 3, 4, 5, 6, 7, or 10 days, for example by measuring a lipid (e.g. CCD lipid), RNA (e.g. mRNA), or protein component. Lipid-encapsulated versus free lipid, RNA, or protein component of the LNP may be measured.

Lipid clearance may be measured as described in literature. *See* Maier, M.A., et al. Biodegradable Lipids Enabling Rapidly Eliminated Lipid Nanoparticles for Systemic Delivery of RNAi Therapeutics. Mol. Ther. 2013, 21(8), 1570-78 (*"Maier"*). For example, in *Maier,* LNP-siRNA systems containing luciferases-targeting siRNA were administered to six- to eight-week old male C57Bl/6 mice at 0.3 mg/kg by intravenous bolus injection *via* the lateral tail vein. Blood, liver, and spleen samples were collected at 0.083, 0.25, 0.5, 1, 2, 4, 8, 24, 48, 96, and 168 hours post-dose. Mice were perfused with saline before tissue collection and blood samples were processed to obtain plasma. All samples were processed and analyzed by LC-MS. Further, *Maier* describes a procedure for assessing toxicity after administration of LNP-siRNA formulations. For example, a luciferase-targeting siRNA was administered at 0, 1, 3, 5, and 10 mg/kg (5 animals/group) via single intravenous bolus injection at a dose volume of 5 mL/kg to male Sprague-Dawley rats. After 24 hours, about 1 mL of blood was obtained from the jugular vein of conscious animals and the serum was isolated. At 72 hours post-dose, all animals were euthanized for necropsy. Assessment of clinical signs, body weight, serum chemistry, organ weights and histopathology was performed. Although Maier describes methods for assessing siRNA-LNP formulations, these methods may be applied to assess clearance, pharmacokinetics, and toxicity of administration of formulations of the present disclosure.

The CCD lipids lead to an increased clearance rate. The clearance rate may be a lipid clearance rate, for example the rate at which a CCD lipid is cleared from the blood, serum, or plasma. The clearance rate may be an RNA clearance rate, for example the rate at which an mRNA or a guide RNA is cleared from the blood, serum, or plasma. The clearance

rate may be the rate at which LNP is cleared from the blood, serum, or plasma. The clearance rate may be the rate at which LNP is cleared from a tissue, such as liver tissue or spleen tissue. A high rate of clearance rate may yield a safety profile with no substantial adverse effects. The CCD lipids reduce LNP accumulation in circulation and in tissues. A reduction in LNP accumulation in circulation and in tissues may yield a safety profile with no substantial adverse effects.

The CCD lipids of the present disclosure may be ionizable depending upon the pH of the medium they are in. For example, in a slightly acidic medium, the CCD lipids may be protonated and thus bear a positive charge. Conversely, in a slightly basic medium, such as, for example, blood where pH is approximately 7.35, the CCD lipids may not be protonated and thus bear no charge. The CCD lipids of the present disclosure may be protonated at a pH of at least about 9. The CCD lipids of the present disclosure may be protonated at a pH of at least about 9. The CCD lipids of the present disclosure may be protonated at a pH of at least about 10.

The ability of a CCD lipid to bear a charge is related to its intrinsic pKa. For example, the CCD lipids of the present disclosure may each, independently, have a pKa in the range of from about 5.8 to about 6.2. This may be advantageous as it has been found that cationic lipids with a pKa ranging from about 5.1 to about 7.4 are effective for delivery of cargo to the liver. Further, it has been found that cationic lipids with a pKa ranging from about 5.3 to about 6.4 are effective for delivery to tumors. *See, e.g.,* WO 2014/136086.

Additional Lipids

"Neutral lipids" for use in a lipid composition of the disclosure are neutral, uncharged or zwitterionic lipids. Examples of neutral phospholipids suitable for use in the present disclosure include, but are not limited to, 5-heptadecylbenzene-1,3-diol (resorcinol), dipalmitoylphosphatidylcholine (DPPC), distearoylphosphatidylcholine (DSPC), pohsphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauryloylphosphatidylcholine (DLPC), dimyristoylphosphatidylcholine (DMPC), 1-myristoyl-2-palmitoyl phosphatidylcholine (MPPC), 1-palmitoyl-2-myristoyl phosphatidylcholine (PMPC), 1-palmitoyl-2-stearoyl phosphatidylcholine (PSPC), 1,2-diarachidoyl-sn-glycero-3-phosphocholine (DBPC), 1-stearoyl-2-palmitoyl phosphatidylcholine (SPPC), 1,2-dieicosenoyl-sn-glycero-3-phosphocholine (DEPC), palmitoyloleoyl phosphatidylcholine (POPC), lysophosphatidyl choline, dioleoyl phosphatidylethanolamine (DOPE), dilinoleoylphosphatidylcholine distearoylphosphatidylethanolamine (DSPE), dimyristoyl phosphatidylethanolamine (DMPE), dipalmitoyl phosphatidylethanolamine (DPPE), palmitoyloleoyl phosphatidylethanolamine (POPE), lysophosphatidylethanolamine and combinations thereof. The neutral phospholipid may be selected from the group consisting of distearoylphosphatidylcholine (DSPC) and dimyristoyl phosphatidyl ethanolamine (DMPE). The neutral phospholipid may be distearoylphosphatidylcholine (DSPC). Neutral lipids function to stabilize and improve processing of the LNPs.

"Helper lipids" are lipids that enhance transfection (e.g. transfection of the nanoparticle including the biologically active agent). The mechanism by which the helper lipid enhances transfection includes enhancing particle stability. The helper lipid may enhance membrane fusogenicity. The helper lipid is selected from a steroid, a sterol, and an alkyl resorcinol. Helper lipids suitable for use in the present disclosure include, but are not limited to, cholesterol, 5-heptadecylresorcinol, and cholesterol hemisuccinate. The helper lipid may be cholesterol. The helper lipid may be cholesterol hemisuccinate.

"Stealth lipids" are lipids that alter the length of time the nanoparticles can exist *in vivo (e.g.,* in the blood). Stealth lipids may assist in the formulation process by, for example, reducing particle aggregation and controlling particle size. Stealth lipids used herein may modulate pharmacokinetic properties of the LNP. Stealth lipids have a hydrophilic head group linked to a lipid moiety. Stealth lipids suitable for use in a lipid composition of the present disclosure and information about the biochemistry of such lipids can be found in Romberg et al., Pharmaceutical Research, Vol. 25, No. 1, 2008, pg. 55-71 and Hoekstra et al., Biochimica et Biophysica Acta 1660 (2004) 41-52. Additional suitable PEG lipids are disclosed, *e.g.,* in WO 2006/007712.

The hydrophilic head group of stealth lipid may comprise a polymer moiety selected from polymers based on PEG (sometimes referred to as poly(ethylene oxide)), poly(oxazoline), poly(vinyl alcohol), poly(glycerol), poly(N-vinylpyrrolidone), polyaminoacids and poly[N-(2-hydroxypropyl)methacrylamide].

Stealth lipids may comprise a lipid moiety. may havelipid moiety of the stealth lipid may be derived from diacylglycerol or diacylglycamide, including those comprising a dialkylglycerol or dialkylglycamide group having alkyl chain length independently comprising from about C4 to about C40 saturated or unsaturated carbon atoms, wherein the chain may comprise one or more functional groups such as, for example, an amide or ester. The dialkylglycerol or dialkylglycamide group can further comprise one or more substituted alkyl groups.

Unless otherwise indicated, the term "PEG" as used herein means any polyethylene glycol or other polyalkylene ether polymer. PEG may be an optionally substituted linear or branched polymer of ethylene glycol or ethylene oxide. PEG may be unsubstituted. The PEG may be substituted, *e.g.*, by one or more alkyl, alkoxy, acyl, hydroxy, or aryl groups. The term may include PEG copolymers such as PEG-polyurethane or PEG-polypropylene (see, *e.g.,* J. Milton Harris,

Poly(ethylene glycol) chemistry: biotechnical and, biomedical applications (1992)); Alternatively, the term does not include PEG copolymers. The PEG may have a molecular weight of from about 130 to about 50,000, about 150 to about 30,000, about 150 to about 20,000, about 150 to about 15,000, about 150 to about 10,000, about 150 to about 6,000, about 150 to about 5,000, about 150 to about 4,000, about 150 to about 3,000, about 300 to about 3,000, about 1,000 to about 3,000, or about 1,500 to about 2,500.

The PEG (*e.g.*, conjugated to a lipid, such as a stealth lipid), may be a "PEG-2K," also termed "PEG 2000," which has an average molecular weight of about 2,000 daltons. PEG-2K is represented herein by the following formula (I), wherein n is 45, meaning that the number averaged degree of polymerization comprises about 45 subunits

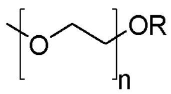 (I)

.

However, other PEG known in the art may be used, including, *e.g.,* those where the number-averaged degree of polymerization comprises about 23 subunits (n=23), and/or 68 subunits (n=68). n may range from about 30 to about 60. n may range from about 35 to about 55. n may range from about 40 to about 50. n may range from about 42 to about 48. n may be 45. R may be selected from H, substituted alkyl, and unsubstituted alkyl. R may be unsubstituted alkyl. R may be methyl.

The stealth lipid may be selected from PEG-dilauroylglycerol, PEG-dimyristoylglycerol (PEG-DMG) (catalog # GM-020 from NOF, Tokyo, Japan), PEG-dipalmitoylglycerol, PEG-distearoylglycerol (PEG-DSPE) (catalog # DSPE-020CN, NOF, Tokyo, Japan), PEG-dilaurylglycamide, PEG-dimyristylglycamide, PEG-dipalmitoylglycamide, and PEG-distearoylglycamide, PEG-cholesterol (1-[8'-(Cholest-5-en-3[beta]-oxy)carboxamido-3',6'-dioxaoctanyl]carbamoyl-[omega]-methyl-poly(ethylene glycol), PEG-DMB (3,4-ditetradecoxylbenzyl-[omega]-methyl-poly(ethylene glycol)ether), 1,2-dimyristoyl-sn-glycero-3-phosphoethanolamine-N-[methoxy(polyethylene glycol)-2000] (PEG2k-DMG) (cat. #880150P from Avanti Polar Lipids, Alabaster, Alabama, USA), 1,2-distearoyl-sn-glycero-3-phosphoethanolamine-N-[methoxy(polyethylene glycol)-2000] (PEG2k-DSPE) (cat. #880120C from Avanti Polar Lipids, Alabaster, Alabama, USA), 1,2-distearoyl-sn-glycerol, methoxypolyethylene glycol (PEG2k-DSG; GS-020, NOF Tokyo, Japan), poly(ethylene glycol)-2000-dimethacrylate (PEG2k-DMA), and 1,2-distearyloxypropyl-3-amine-N-[methoxy(polyethylene glycol)-2000] (PEG2k-DSA). The stealth lipid may be PEG2k-DMG. The stealth lipid may be PEG2k-DSPE. The stealth lipid may be PEG2k-DMA. The stealth lipid may be PEG2k-DSA. The stealth lipid may be PEG2k-C11. The stealth lipid may be PEG2k-C14. The stealth lipid may be PEG2k-C16. The stealth lipid may be PEG2k-C18.

LNP Formulations

The LNP may contain (i) a CCD lipid for encapsulation and for endosomal escape, (ii) a neutral lipid for stabilization, (iii) a helper lipid, also for stabilization, and (iv) a stealth lipid.

The cargo includes an mRNA encoding a Cas nuclease, such as Cas9, and a guide RNA or a nucleic acid encoding a guide RNA. An LNP composition comprises a CCD lipid, selected from Lipid A, Lipid B, Lipid C, or Lipid D. The CCD lipid may be Lipid A. The CCD lipid may be Lipid B. An LNP composition may comprise a CCD lipid, a neutral lipid, a helper lipid, and a stealth lipid. The helper lipid may be cholesterol. The neutral lipid may be DSPC. The stealth lipid may be PEG2k-DMG. An LNP composition may comprise a Lipid A, a helper lipid, a neutral lipid, and a stealth lipid. An LNP composition may comprise a CCD lipid, DSPC, cholesterol, and a stealth lipid. The LNP composition may comprise a stealth lipid comprising PEG. The CCD lipid is selected from Lipid A, Lipid B, Lipid C, or Lipid D. An LNP composition may comprise a CCD lipid selected from Lipid A or Lipid B, cholesterol, DSPC, and PEG2k-DMG.

An LNP composition may comprise a CCD lipid and an mRNA encoding a Cas nuclease. An LNP composition may comprise a CCD lipid, an mRNA encoding a Cas nuclease, and at least one other lipid component. In some compositions comprising an mRNA encoding a Cas nuclease, the LNP may include at least one other lipid component chosen from a helper lipid, a neutral lipid, or a stealth lipid. In certain compositions comprising an mRNA encoding a Cas nuclease, the helper lipid may be cholesterol. In other compositions comprising an mRNA encoding a Cas nuclease, the neutral lipid may be DSPC. In additional compositions comprising an mRNA encoding a Cas nuclease, the stealth lipid may be PEG2k-DMG. An LNP composition may comprise a CCD lipid, a helper lipid, a neutral lipid, a stealth lipid, and an mRNA encoding a Cas nuclease. In specific compositions comprising an mRNA encoding a Cas nuclease, the CCD lipid may be selected from Lipid A, Lipid B, Lipid C, or Lipid D. In additional compositions comprising an mRNA encoding a Cas nuclease, the CCD lipid may be selected from Lipid A, Lipid B, Lipid C, or Lipid D, the helper lipid may be cholesterol, the neutral lipid may be DSPC, and the stealth lipid may be PEG2k-DMG. The CCD lipid in compositions comprising an mRNA encoding a Cas nuclease may be Lipid A. The CCD lipid in compositions comprising an mRNA encoding a Cas nuclease may be Lipid B. The CCD lipid in compositions comprising an mRNA encoding a Cas nuclease may be Lipid C. The CCD lipid in compositions comprising an mRNA encoding a Cas nuclease may be Lipid D.

An LNP composition may comprise a CCD lipid and a Class 2 Cas nuclease mRNA. An LNP composition may comprise a CCD lipid, a Class 2 Cas nuclease mRNA, and at least one other lipid component. In some compositions comprising a Class 2 Cas nuclease mRNA, the LNP includes at least one other lipid component chosen from a helper lipid, a neutral lipid, or a stealth lipid. In certain compositions comprising a Class 2 Cas nuclease mRNA, the helper lipid is cholesterol. In other compositions comprising a Class 2 Cas nuclease mRNA, the neutral lipid is DSPC. In additional compositions comprising a Class 2 Cas nuclease mRNA, the stealth lipid may be PEG2k-DMG. An LNP composition may comprise a CCD lipid, a helper lipid, a neutral lipid, a stealth lipid, and a Class 2 Cas nuclease mRNA. In specific compositions comprising a Class 2 Cas nuclease mRNA, the CCD lipid is selected from Lipid A, Lipid B, Lipid C, or Lipid D. In additional compositions comprising a Class 2 Cas nuclease mRNA, the CCD lipid is selected from Lipid A, Lipid B, Lipid C, or Lipid D, the helper lipid is cholesterol, the neutral lipid is DSPC, and the stealth lipid is PEG2k-DMG. The CCD lipid in compositions comprising a Class 2 Cas nuclease mRNA may be Lipid A. The CCD lipid in compositions comprising a Class 2 Cas nuclease mRNA may be Lipid B. The CCD lipid in compositions comprising a Class 2 Cas nuclease mRNA may be Lipid C. The CCD lipid in compositions comprising a Class 2 Cas nuclease mRNA may be Lipid D.

An LNP composition may comprise a guide RNA. An LNP composition may comprise a CCD lipid, a guide RNA, a helper lipid, a neutral lipid, and a stealth lipid. In certain LNP compositions comprising a guide RNA, the helper lipid is cholesterol. In other compositions comprising a guide RNA, the neutral lipid is DSPC. In additional compositions comprising a guide RNA, the stealth lipid may be PEG2k-DMG or PEG2k-C11. The LNP composition comprises Lipid A, Lipid B, Lipid C, or Lipid D; a helper lipid; a neutral lipid; a stealth lipid; and a guide RNA. In certain compositions comprising a guide RNA, the CCD lipid Lipid A. In certain compositions comprising a guide RNA, the CCD lipid is Lipid B. In certain compositions comprising a guide RNA, the CCD lipid is Lipid C. In certain compositions comprising a guide RNA, the CCD lipid is Lipid D. In additional compositions comprising a guide RNA, the CCD lipid is Lipid A, Lipid B, Lipid C, or Lipid D; the helper lipid is cholesterol; the neutral lipid is DSPC; and the stealth lipid is PEG2k-DMG.

The LNP formulation may include a ratio of Class 2 Cas nuclease mRNA to gRNA nucleic acid ranging from about 25:1 to about 1:25. The LNP formulation may include a ratio of Class 2 Cas nuclease mRNA to gRNA nucleic acid ranging from about 10:1 to about 1:10. As measured herein, the ratios are by weight. The LNP formulation may include a ratio of Class 2 Cas nuclease mRNA to gRNA nucleic acid ranging from about 5:1 to about 1:5. The LNP formulation may include a ratio of Class 2 Cas nuclease mRNA to gRNA nucleic acid of about 1:1. The LNP formulation may include a ratio of Class 2 Cas nuclease mRNA to gRNA nucleic acid from about 1:1 to about 1:5. The LNP formulation may include a ratio of Class 2 Cas nuclease mRNA to gRNA nucleic acid of about 10:1. The LNP formulation may include a ratio of Class 2 Cas nuclease mRNA to gRNA nucleic acid of about 1:10. The ratio may be about 25:1, 10:1, 5:1, 3:1, 1:1, 1:3, 1:5, 1:10, or 1:25.

An LNP composition may comprise an sgRNA. An LNP composition may comprise a Cas9 sgRNA. An LNP composition may comprise a Cpfl sgRNA. In some compositions comprising an sgRNA, the LNP includes a CCD lipid, a helper lipid, a neutral lipid, and a stealth lipid. In certain compositions comprising an sgRNA, the helper lipid may be cholesterol. In other compositions comprising an sgRNA, the neutral lipid may be DSPC. In additional compositions comprising an sgRNA, the stealth lipid may be PEG2k-DMG or PEG2k-C11. An LNP composition may comprise a CCD lipid, a helper lipid, a neutral lipid, a stealth lipid, and an sgRNA. In specific compositions comprising an sgRNA, the CCD lipid is Lipid A, Lipid B, Lipid C, or Lipid D. In additional compositions comprising an sgRNA, the CCD lipid is Lipid A, Lipid B, Lipid C, or Lipid D; the helper lipid may be cholesterol; the neutral lipid may be DSPC; and the stealth lipid may be PEG2k-DMG.

An LNP composition comprises an mRNA encoding a Cas nuclease and a guide RNA, which may be an sgRNA. An LNP composition may comprise a CCD lipid, an mRNA encoding a Cas nuclease, a guide RNA, a helper lipid, a neutral lipid, and a stealth lipid. In certain compositions comprising an mRNA encoding a Cas nuclease and a guide RNA, the helper lipid may be cholesterol. In some compositions comprising an mRNA encoding a Cas nuclease and a guide RNA, the neutral lipid may be DSPC. In additional compositions comprising an mRNA encoding a Cas nuclease and a guide RNA, the stealth lipid may be PEG2k-DMG or PEG2k-C11. An LNP composition may comprise a CCD lipid, a helper lipid, a neutral lipid, a stealth lipid, an mRNA encoding a Cas nuclease, and a guide RNA. In specific compositions comprising an mRNA encoding a Cas nuclease and a guide RNA, the CCD lipid may be Lipid A, Lipid B, Lipid C, or Lipid D. In additional compositions comprising an mRNA encoding a Cas nuclease and a guide RNA, the CCD lipid is Lipid A, Lipid B, Lipid C, or Lipid D; the helper lipid may be cholesterol; the neutral lipid may be DSPC; and the stealth lipid may be PEG2k-DMG.

The LNP compositions disclosed herein may include a template nucleic acid. The template nucleic acid may be co-formulated with an mRNA encoding a Cas nuclease, such as a Class 2 Cas nuclease mRNA. The template nucleic acid may be co-formulated with a guide RNA. The template nucleic acid may be co-formulated with both an mRNA encoding a Cas nuclease and a guide RNA. The template nucleic acid may be formulated separately from an mRNA encoding a Cas nuclease or a guide RNA. In such formulations, the template nucleic acid may be single- or double-stranded, depending on the desired repair mechanism. The template may have regions of homology to the target DNA, or to sequences adjacent to the target DNA.

The present disclosure also provides lipid compositions described according to the respective molar ratios of the component lipids in the formulation. The mol-% of the CCD lipid is from about 30 mol-% to about 60 mol-%. The mol-% of

the CCD lipid may be from about 35 mol-% to about 55 mol-%. The mol-% of the CCD lipid may be from about 40 mol-% to about 50 mol-%. The mol-% of the CCD lipid may be from about 42 mol-% to about 47 mol-%. The mol-% of the CCD lipid may be about 45%. The CCD lipid mol-% of the LNP batch may be ±30%, ±25%, ±20%, ±15%, ±10%, ±5%, or ±2.5% of the target mol-%. LNP inter-lot variability may be less than 15%, less than 10% or less than 5%.

The mol-% of the helper lipid is from about 30 mol-% to about 60 mol-%. The mol-% of the helper lipid may be from about 35 mol-% to about 55 mol-%. The mol-% of the helper lipid may be from about 40 mol-% to about 50 mol-%. The mol-% of the helper lipid may be from about 41 mol-% to about 46 mol-%. The mol-% of the helper lipid may be about 44 mol-%. The helper mol-% of the LNP batch may be ±30%, ±25%, ±20%, ±15%, ±10%, ±5%, or ±2.5% of the target mol-%. The LNP inter-lot variability may be less than 15%, less than 10% or less than 5%.

The mol-% of the neutral lipid is from about 1 mol-% to about 20 mol-%. The mol-% of the neutral lipid may be from about 5 mol-% to about 15 mol-%. The mol-% of the neutral lipid may be from about 7 mol-% to about 12 mol-%. The mol-% of the neutral lipid may be about 9 mol-%. The neutral lipid mol-% of the LNP batch may be ±30%, ±25%, ±20%, ±15%, ±10%, ±5%, or ±2.5% of thae target molLNP inter-lot variability may be less than 15%, less than 10% or less than 5%.

The mol-% of the stealth lipid is from about 1 mol-% to about 10 mol-%. The mol-% of the stealth lipid may be from about 1 mol-% to about 5 mol-%. The mol-% of the stealth lipid may be from about 1 mol-% to about 3 mol-%. The mol-% of the stealth lipid may be about 2 mol-%. The mol-% of the stealth lipid may be about 1 mol-%. The stealth lipid mol-% of the LNP batch may be ±30%, ±25%, ±20%, ±15%, ±10%, ±5%, or ±2.5% of the target mol-%. LNP inter-lot variability may be less than 15%, less than 10% or less than 5%.

The present disclosure also provides lipid compositions described according to the ratio between the positively charged amine groups of the CCD lipid (N) and the negatively charged phosphate groups (P) of the nucleic acid to be encapsulated. This may be mathematically represented by the equation N/P. The N/P ratio may be from about 0.5 to about 100. The N/P ratio may be from about 1 to about 50. The N/P ratio may be from about 1 to about 25. The N/P ratio may be from about 1 to about 10. The N/P ratio may be from about 1 to about 7. The N/P ratio may be from about 3 to about 5. The N/P ratio may be from about 4 to about 5. The N/P ratio may be about 4. The N/P ratio may be about 4.5. The N/P ratio may be about 5.

LNPs may be formed by mixing an aqueous RNA solution with an organic solvent-based lipid solution, e.g., 100% ethanol. Suitable solutions or solvents include or may contain: water, PBS, Tris buffer, NaCl, citrate buffer, ethanol, chloroform, diethylether, cyclohexane, tetrahydrofuran, methanol, isopropanol. A pharmaceutically acceptable buffer, e.g., for *in vivo* administration of LNPs, may be used. A buffer may be used to maintain the pH of the composition comprising LNPs at or above pH 7.0. The composition may have a pH ranging from about 7.3 to about 7.7 or ranging from about 7.4 to about 7.6. The composition may have a pH of about 7.3, 7.4, 7.5, 7.6, or 7.7. The pH of a composition may be measured with a micro pH probe. A cryoprotectant may be included in the composition. Non-limiting examples of cryoprotectants include sucrose, trehalose, glycerol, DMSO, and ethylene glycol. Exemplary compositions may include up to 10% cryoprotectant, such as, for example, sucrose. The LNP composition may include about 1, 2, 3, 4, 5, 6, 7, 8, 9, or 10% cryoprotectant. The LNP composition may include about 1, 2, 3, 4, 5, 6, 7, 8, 9, or 10% sucrose. The LNP composition may include a buffer. The buffer may comprise a phosphate buffer (PBS), a Tris buffer, a citrate buffer, and mixtures thereof. The buffer comprises NaCl. Exemplary amounts of NaCl may range from about 40 mM to about 50 mM. The amount of NaCl may be about 45 mM. The buffer may be a Tris buffer. Exemplary amounts of Tris may range from about 40 mM to about 60 mM. The amount of Tris may be about 50 mM. The buffer comprises NaCl and Tris. The LNP compositions may contain 5% sucrose and 45 mM NaCl in Tris buffer. The compositions may contain sucrose in an amount of about 5% w/v, about 45 mM NaCl, and about 50 mM Tris. The salt, buffer, and cryoprotectant amounts may be varied such that the osmolality of the overall formulation is maintained. For example, the final osmolality may be maintained at less than 450 mOsm/L. The osmolality may be between 350 and 250 mOsm/L. The final osmolality may be 300 +/- 20 mOsm/L.

Microfluidic mixing, T-mixing, or cross-mixing may be used. Accordingly, flow rates, junction size, junction geometry, junction shape, tube diameter, solutions, and/or RNA and lipid concentrations may be varied. LNPs or LNP compositions may be concentrated or purified, *e.g.,* via dialysis or chromatography. The LNPs may be stored as a suspension, an emulsion, or a lyophilized powder, for example. The LNP compositions may be stored at 2-8° C, in certain aspects, the LNP compositions are stored at room temperature. The LNP composition may be stored frozen, for example at -20° C or -80° C. The LNP composition may be stored at a temperature ranging from about 0° C to about -80° C. Frozen LNP compositions may be thawed before use, for example on ice, at room temperature, or at 25° C

Dynamic Light Scattering ("DLS") can be used to characterize the polydispersity index ("pdi") and size of the LNPs of the present disclosure. DLS measures the scattering of light that results from subjecting a sample to a light source. PDI, as determined from DLS measurements, represents the distribution of particle size (around the mean particle size) in a population, with a perfectly uniform population having a PDI of zero. The pdi may range from about 0.005 to about 0.75. The pdi may range from about 0.01 to about 0.5. The pdi may range from about 0.02 to about 0.4. The pdi may range from about 0.03 to about 0.35. The pdi may range from about 0.1 to about 0.35.

The LNPs disclosed herein may have a size of about 1 to about 250 nm. The LNPs may have a size of about 10 to about 200 nm. The LNPs may have a size of about 20 to about 150 nm. The LNPs may have a size of about 50 to about 150

nm. The LNPs may have a size of about 50 to about 100 nm. The LNPs may have a size of about 50 to about 120 nm. The LNPs may have a size of about 75 to about 150 nm. The LNPs may have a size of about 30 to about 200 nm. Unless indicated otherwise, all sizes referred to herein are the average sizes (diameters) of the fully formed nanoparticles, as measured by dynamic light scattering on a Malvern Zetasizer. The nanoparticle sample may be diluted in phosphate buffered saline (PBS) so that the count rate is approximately 200-400 kcts. The data may be presented as a weighted-average of the intensity measure. The LNPs may be formed with an average encapsulation efficiency ranging from about 50% to about 100%. The LNPs may be formed with an average encapsulation efficiency ranging from about 50% to about 70%. The LNPs may be formed with an average encapsulation efficiency ranging from about 70% to about 90%. The LNPs may be formed with an average encapsulation efficiency ranging from about 90% to about 100%. The LNPs may be formed with an average encapsulation efficiency ranging from about 75% to about 95%.

Methods of Engineering Cells; Engineered Cells

The LNP compositions disclosed herein may be used in methods for engineering cells through gene editing, both *in vivo* and *in vitro.* The methods may involve contacting a cell with an LNP composition described herein. The cell may be a mammalian cell. The cell may be a rodent cell. The cell may be a human cell. The cell may be a liver cell. The cell may be a human liver cell. The liver cell may be a hepatocyte. The hepatocyte may be a human hepatocyte. The liver cell may be a stem cell. The human liver cell may be a liver sinusoidal endothelial cell (LSEC). The human liver cell may be a Kupffer cell. The human liver cell may be a hepatic stellate cell. The human liver cell may be a tumor cell. The human liver cell may be a liver stem cell. The cell may comprise ApoE-binding receptors.

Engineered cells are provided, for example an engineered cell derived from any one of the cell types in the preceding paragraph. Such engineered cells are produced according to the methods described herein. The engineered cell may reside within a tissue or organ, e.g., a liver within a subject.

In some of the methods and cells described herein, a cell comprises a modification, for example an insertion or deletion ("indel") or substitution of nucleotides in a target sequence. The modification may comprise an insertion of 1, 2, 3, 4 or 5 or more nucleotides in a target sequence. The modification may comprise an insertion of either 1 or 2 nucleotides in a target sequence. The modification may comprise a deletion of 1, 2, 3, 4, 5, 6, 7, 8, 9, 10, 15, 20 or 25 or more nucleotides in a target sequence. The modification may comprise a deletion of either 1 or 2 nucleotides in a target sequence. The modification may comprise an indel which results in a frameshift mutation in a target sequence. The modification may comprise a substitution of 1, 2, 3, 4, 5, 6, 7, 8, 9, 10, 15, 20 or 25 or more nucleotides in a target sequence. The modification may comprise a substitution of either 1 or 2 nucleotides in a target sequence. The modification may comprise one or more of an insertion, deletion, or substitution of nucleotides resulting from the incorporation of a template nucleic acid, for example any of the template nucleic acids described herein.

A population of cells comprising engineered cells may be provided, for example a population of cells comprising cells engineered according to the methods described herein. The population may comprise engineered cells cultured *in vitro.* The population may reside within a tissue or organ, e.g., a liver within a subject. At least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% or more of the cells within the population may be engineered. A method disclosed herein may result in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% editing efficiency (or "percent editing"), defined by detetion of indels. A method disclosed herein, may result in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% DNA modification efficiency, defined by detecting a change in sequence, whether by insertion, deletion, substitution or otherwise. A method disclosed herein may result in an editing efficiency level or a DNA modification efficiency level of between about 5% to about 100%, about 10% to about 50%, about 20 to about 100%, about 20 to about 80%, about 40 to about 100%, or about 40 to about 80%.

In some of the methods and cells described herein, cells within the population comprise a modification, e.g., an indel or substitution at a target sequence. The modification may comprise an insertion of 1, 2, 3, 4 or 5 or more nucleotides in a target sequence. The modification may comprise an insertion of either 1 or 2 nucleotides in a target sequence. The modification may comprise a deletion of 1, 2, 3, 4, 5, 6, 7, 8, 9, 10, 15, 20 or 25 or more nucleotides in a target sequence. The modification may comprise a deletion of either 1 or 2 nucleotides in a target sequence. The modification may comprise an indel which results in a frameshift mutation in a target sequence. At least 80%, at least 85%, at least 90%, at least 91%, at least 92%, at least 93%, at least 94%, at least 95%, at least 96%, at least 97%, at least 98%, or at least 99% or more of the engineered cells in the population may comprise a frameshift mutation. The modification may comprise a substitution of 1, 2, 3, 4, 5, 6, 7, 8, 9, 10, 15, 20 or 25 or more nucleotides in a target sequence. The modification may comprise a substitution of either 1 or 2 nucleotides in a target sequence. The modification may comprise one or more of an insertion, deletion, or

substitution of nucleotides resulting from the incorporation of a template nucleic acid, for example any of the template nucleic acids described herein.

**Methods of Treatment**

The references to methods of treatment in the subsequent paragraphs of this description are to be interpreted as references to the compounds, pharmaceutical compositions and medicaments of the present invention for use in a method for treatment of the human (or animal) body by therapy (or for in vivo diagnosis).

The LNP compositions disclosed herein may be used for gene editing *in vivo* and *in vitro.* Thus, one or more LNP compositions described herein may be administered to a subject in need thereof. A therapeutically effective amount of a composition described herein may contact a cell of a subject in need thereof. A genetically engineered cell may be produced by contacting a cell with an LNP composition described herein.

The methods may involve administering the tLNP composition to a cell associated with a liver disorder. The methods may involve treating a liver disorder. The methods may involve contacting a hepatic cell with the LNP composition. The methods may involve contacting a hepatocyte with the LNP composition. The methods may involve contacting an ApoE binding cell with the LNP composition.

An LNP composition comprising an mRNA encoding a Cas nuclease, a gRNA, and a template may be administered to a cell, such as an ApoE binding cell. In certain instances, an LNP composition comprising a Cas nuclease and an sgRNA may be administered to a cell, such as an ApoE binding cell. An LNP composition comprising an mRNA encoding a Cas nuclease, a gRNA, and a template may be administered to a liver cell. In certain instances, an LNP composition comprising a Cas nuclease and an sgRNA may be administered to a liver cell. In some cases, the liver cell may be in a subject. A subject may receive a single dose of an LNP composition. In other examples, a subject may receive multiple doses of an LNP composition. Where more than one dose is administered, the doses may be administered about 1, 2, 3, 4, 5, 6, 7, 14, 21, or 28 days apart; about 2, 3, 4, 5, or 6 months apart; or about 1, 2, 3, 4, or 5 years apart.

An LNP composition comprising an mRNA encoding a Cas nuclease may be administered to a liver cell (also called a hepatic cell), followed by the administration of a composition comprising a gRNA and optionally a template. An LNP composition comprising an mRNA encoding a Cas nuclease and a gRNA may be administered to a liver cell, followed by the administration of a composition comprising a template to the cell. An LNP composition comprising an mRNA encoding a Cas nuclease may be administered to a liver cell, followed by the sequential administration of an LNP composition comprising a gRNA and then an LNP composition comprising a template to the cell. Where an LNP composition comprising an mRNA encoding a Cas nuclease is administered before an LNP composition comprising a gRNA, the administrations may be separated by about 4, 6, 8, 12, or 24 hours; or 2, 3, 4, 5, 6, or 7 days.

The LNP compositions may be used to edit a gene resulting in a gene knockout. The LNP compositions may be used to edit a gene resulting in a gene correction. The LNP compositions may be used to edit a cell resulting in gene insertion.

Administration of the LNP compositions may result in gene editing which results in persistent response. For example, administration may result in a duration of response of a day, a month, a year, or longer. As used herein, "duration of response" means that, after cells have been edited using an LNP composition disclosed herein, the resulting modification is still present for a certain period of time after administration of the LNP composition. The modification may be detected by measuring target protein levels. The modification may be detected by detecting the target DNA. The duration of response may be at least 1 week. The duration of response may be at least 2 weeks. The duration of response may be at least 1 month. The duration of response may be at least 2 months. The duration of response may be at least 4 months. The duration of response may be at least 6 months. The duration of response may be about 26 weeks. The duration of response may be at least 1 year. The duration of response may be at least 5 years. The duration of response may be at least 10 years. A persistent response may be detectable after at least 6 months, either by measuring target protein levels or by detection of the target DNA.

The LNP compositions can be administered parenterally. The LNP compositions may be administered directly into the blood stream, into tissue, into muscle, or into an internal organ. Administration may be systemic, e.g., to injection or infusion. Administration may be local. Suitable means for administration include intravenous, intraarterial, intrathecal, intraventricular, intraurethral, intrasternal, intracranial, subretinal, intravitreal, intra-anterior chamber, intramuscular, intrasynovial and subcutaneous. Suitable devices for administration include needle (including microneedle) injectors, needle-free injectors and infusion techniques.

The LNP compositions will generally, but not necessarily, be administered as a formulation in association with one or more pharmaceutically acceptable excipients. The term "excipient" includes any ingredient other than the compound(s) of the disclosure, the other lipid component(s) and the biologically active agent. An excipient may impart either a functional (*e.g.* drug release rate controlling) and/or a non-functional (*e.g.* processing aid or diluent) characteristic to the formulations. The choice of excipient will to a large extent depend on factors such as the particular mode of administration, the effect of the excipient on solubility and stability, and the nature of the dosage form.

Parenteral formulations are typically aqueous or oily solutions or suspensions. Where the formulation is aqueous, excipients such as sugars (including but not restricted to glucose, mannitol, sorbitol, *etc.)* salts, carbohydrates and buffering agents (preferably to a pH of from 3 to 9), but, for some applications, they may be more suitably formulated with a sterile non-aqueous solution or as a dried form to be used in conjunction with a suitable vehicle such as sterile, pyrogen-free water (WFI).

The methods of gene editing may modify a Factor VII target gene. The LNP compositions may be administered to a liver cell to modify a Factor VII gene. The LNP compositions may be used for treating a liver disorder, such as Factor VII deficiency. The methods may modulate aberrant Factor VII activity. The LNP composition may be administered to treat or prevent hemophilia, or the inability to control blood clotting. *See, e.g.,* Lapecorella, M. and Mariani, G. Factor VII deficiency: defining the clinical picture and optimizing therapeutic options. Haemophilia (2008), 14, 1170-1175. The LNP compositions may be administered to treat or prevent thrombophilia, a condition where blood has an increased tendency to form clots.

When an injury to a tissue occurs, the formation of an equimolar complex between Factor VII zymogen and Tissue Factor, resulting in a cleavage at position 152 of the Factor VII sequence, leading to the formation of activated Factor VII, or Factor VIIa. The Factor VIIa/Tissue Factor complex leads to coagulation. The methods of treatment of a Factor VII-associated disorder include methods of increasing Factor VIIa coagulation, methods of improving blood clotting, or methods of improving a blood coagulation profile. The methods may be used to administer an LNP composition to a subject with a Factor VII deficiency. The methods may be used to administer an LNP composition to a subject previously treated for Factor VII deficiency, e.g. with recombinant Factor VIIa.

The methods of gene editing may modify a TTR target gene. The LNP compositions may be used for treating a disorder associated with TTR expression in the liver, such as amyloidosis. The LNP composition may be administered to treat or prevent amyloidosis, including transthyretin type amyloidosis. *See, e.g.,* Patel, K. and Hawkins, P. Cardiac amyloidosis: where are we today? J. Intern. Med. (2008), 278, 126-144. The TTR-associated disorder can lead to accumulation of amyloid deposits. Therefore, the methods to treat or prevent a TTR-associated disorder include methods of reducing TTR levels, methods of reducing TTR production, methods of reducing amyloid deposits, methods of treating inherited transthyretin type amyloidosis, methods of treating nonhereditary transthyretin type amyloidosis, or methods of affecting amyloid deposits in the heart, and autonomic and peripheral nerves. The methods of treating or preventing a TTR-associated disorder may comprise administering an LNP composition to a subject diagnosed amyloid deposits. The methods may be used to administer an LNP composition to a subject in need of reduced TTR production The methods of gene editing target a gene that may be selected from *SERPINA1, FVIII, FIX, SERPING1, KLKBl, KNGl, FXII, ASS1, ASL, BCKDHA, BCKDHB, G6PC, GO/HAO1, AGXT, PCCA, PCCB, OTC, LIPA, ABCB11*, *GALT, ATP7B, and PAH.* The methods of gene editing may be used to treat a subject afflicted with a disease selected from Alpha 1 Antitrypsin Deficiency, Hemophilia A, Hemophilia B, HAE, Type 1 Citrullinemia, Arginiosuccinic aciduria, Maple syrup urine disease, Glycogen storage disease, Primary hyperoxaluria type 1, Propionic academia, Ornithine transcarbamylase deficiency, Cholesteryl ester storage disease, Progressive familial intrahepatic cholestasis, Galactosemia, Wilson's disease, and Phenylketonuria.

The words "a", "an" or "the" when used in conjunction with the term "comprising" in the claims and/or the specification may mean "one," but each is also consistent with the meaning of "one or more," "at least one," and "one or more than one." The use of "or" means "and/or" unless stated otherwise. The use of the term "including" and "containing," as well as other forms, such as "includes," "included," "contains," and "contained" is not limiting. All ranges given in the application encompass the endpoints unless stated otherwise.

EXAMPLES

Example 1. Materials and Methods.

***Lipid Nanoparticle ("LNP") Formulation***

The LNPs were formulated with a CCD lipid amine to RNA phosphate (N:P) molar ratio of about 4.5. The lipid nanoparticle components were dissolved in 100% ethanol with the following molar ratios: 45 mol-% (12.7 mM) CCD lipid *(e.g.,* Lipid A or Lipid B); 44 mol-% (12.4 mM) helper lipid (*e.g.*, cholesterol); 9 mol-% (2.53 mM) neutral lipid *(e.g.,* DSPC); and 2 mol-% (.563 mM) PEG (*e.g.,* PEG2k-DMG or PEG2k-C11). The RNA cargo were dissolved in 50 mM acetate buffer, pH 4.5, resulting in a concentration of RNA cargo of approximately 0.45 mg/mL.

The LNPs were formed by microfluidic mixing of the lipid and RNA solutions using a Precision Nanosystems NanoAssemblr™ Benchtop Instrument, according to the manufacturer's protocol. A 2:1 ratio of aqueous to organic solvent was maintained during mixing using differential flow rates. After mixing, the LNPs were collected, diluted in phosphate buffered saline (PBS, approximately 1:1), and then remaining buffer was exchanged into PBS (100-fold excess of sample volume), overnight at 4°C under gentle stirring using a 10 kDa Slide-a-Lyzer™ G2 Dialysis Cassette (ThermoFisher

Scientific). The resulting mixture was then filtered using a 0.2 μm sterile filter. The resulting filtrate was stored at 2-8 °C. The isolated LNPs were characterized to determine the encapsulation efficiency, polydispersity index, and average particle size, as described below.

***In vitro transcription ("IVT") of nuclease mRNA and single guide RNA (sgRNA)***

Capped and polyadenylated Cas9 mRNA containing N1-methyl pseudo-U was generated by *in vitro* transcription using a linearized plasmid DNA template and T7 RNA polymerase. Plasmid DNA containing a T7 promoter and a 100 nt poly(A/T) region was linearized by incubating at 37 °C for 2 hrs with XbaI with the following conditions: 200 ng/μL plasmid, 2 U/μL XbaI (NEB), and 1x reaction buffer. The XbaI was inactivated by heating the reaction at 65 °C for 20 min. The linearized plasmid was purified from enzyme and buffer salts using a silica maxi spin column (Epoch Life Sciences) and analyzed by agarose gel to confirm linearization. The IVT reaction to generate Cas9 modified mRNA was incubated at 37 °C for 4 hours in the following conditions: 50 ng/μL linearized plasmid; 2 mM each of GTP, ATP, CTP, and N1-methyl pseudo-UTP (Trilink); 10 mM ARCA (Trilink); 5 U/μL T7 RNA polymerase (NEB); 1 U/μL Murine RNase inhibitor (NEB); 0.004 U/μL Inorganic *E. coli* pyrophosphatase (NEB); and 1x reaction buffer. After the 4 hr incubation, TURBO DNase (ThermoFisher) was added to a final concentration of 0.01 U/μL, and the reaction was incubated for an additional 30 minutes to remove the DNA template. The Cas9 mRNA was purified from enzyme and nucleotides using a MegaClear Transcription Clean-up kit according to the manufacturer's protocol (ThermoFisher). Alternatively, for example as shown in Example 15, the mRNA was purified through a precipitation protocol, which in some cases was followed by HPLC-based purification. Briefly, after the DNase digestion, the mRNA was precipitated by adding 0.21x vol of a 7.5 M LiCl solution and mixing, and the precipitated mRNA was pelleted by centrifugation. Once the supernatant was removed, the mRNA was reconstituted in water. The mRNA was precipitated again using ammonium acetate and ethanol. 5M Ammonium acetate was added to the mRNA solution for a final concentration of 2M along with 2x volume of 100% EtOH. The solution was mixed and incubated at -20 °C for 15 min. The precipitated mRNA was again pelleted by centrifugation, the supernatant was removed, and the mRNA was reconstituted in water. As a final step, the mRNA was precipitated using sodium acetate and ethanol. 1/10 volume of 3 M sodium acetate (pH 5.5) was added to the solution along with 2x volume of 100% EtOH. The solution was mixed and incubated at -20 °C for 15 min. The precipitated mRNA was again pelleted by centrifugation, the supernatant was removed, the pellet was washed with 70% cold ethanol and allowed to air dry. The mRNA was reconstituted in water. For HPLC purified mRNA, after the LiCl precipitation and reconstitution, the mRNA was purified by RP-IP HPLC (see, e.g., Kariko, et al. Nucleic Acids Research, 2011, Vol. 39, No. 21 e142). The fractions chosen for pooling were combined and deslated by sodium acetate/ethanol precipitation as described above. For all methods, the transcript concentration was determined by measuring the light absorbance at 260 nm (Nanodrop), and the transcript was analyzed by capillary electrophoresis by Bioanlayzer (Agilent).

IVT was also used to generate sgRNA in a similar process. DNA template for sgRNA was generated by annealing a top oligo composed of only the T7 RNA polymerase promoter sequence and a bottom strand containing the sgRNA template and the complementary sequence to the promoter site. The annealed template was used directly in an IVT reaction in the following conditions: 125 nM template; 7.5 mM each of GTP, ATP, CTP, and UTP; 5 U/μL T7 RNA polymerase (NEB); 1 U/μL Murine RNase inhibitor (NEB); 0.004 U/μL Inorganic *E. coli* pyrophosphatase (NEB); and 1x reaction buffer. The reaction was incubated at 37 °C for 8 hours, after which TURBO DNase (ThermoFisher) was added to a final concentration of 0.01 U/μL, and the reaction was incubated another 30 minutes to remove the DNA template. The sgRNA transcript was purified by a MegaClear Transcription Clean-up kit according to the manufacturer's protocol (ThermoFisher). The transcript concentration was determined by absorbance at 260 nm (Nanodrop), and the transcript was analyzed by PAGE.

***Formulation Analytics***

LNP formulations were analyzed for average particle size, polydispersity (pdi), total RNA content and encapsulation efficiency of RNA. Average particle size and polydispersity were measured by dynamic light scattering (DLS) using a Malvern Zetasizer DLS instrument. LNP samples were diluted 30X in PBS prior to being measured by DLS. Z-average diameter which is intensity based measurement of average particle size was reported along with pdi.

A fluorescence-based assay was used to determine total RNA concentration and encapsulation efficiency. LNPs were diluted 75X with 1x TE buffer to be within the linear range of the RiboGreen® dye (ThermoFisher Scientific, catalog number R11491). 50 μl of diluted LNP were further mixed with either 50μl 1x TE buffer or 1x TE buffer with 0.2% Triton X-100 in duplicate. Samples were incubated at 37 °C for 10 minutes to allow Triton to completely disrupt the LNPs and expose total RNA to interact with the RiboGreen® dye. Samples for standard curve were prepared by utilizing the starting RNA solution used to make the LNPs and following the same steps as above. Diluted RiboGreen® dye (100 μL, 100X in 1xTE buffer, according to the manufacturer's instructions) was then added to each of the samples and allowed to incubate for 10 minutes at room temperature, in the absence of light. A SpectraMax M5 Microplate Reader (Molecular Devices) was

used to read the samples with excitation, auto cutoff and emission wavelengths set to 488 nm, 515 nm, and 525 nm respectively. Encapsulation efficiency (%EE) was calculated using the following equation:

$$\%EE = \left(1 - \frac{Fluorescence\ @525nm - triton}{Fluorescence\ @525 + triton}\right) * 100$$

where Fluorescence @ 525 nm - triton is average fluorescence reading for sample without Triton, and Fluorescence @ 525 nm + triton is average fluorescence reading for sample with Triton. Total RNA concentration was determined using a liner standard curve and average fluorescence reading for sample with triton value.

The same procedure may be used for determining the encapsulation efficiency of a DNA-based cargo component. For single-strand DNA Oligreen Dye may be used, and for double-strand DNA, Picogreen Dye.

The values for average particle size, polydispersity, and %EE are reported in Table 1, below, for various LNP compositions.

**Table 1. Summary of LNP Formulation Data**

| LNP # | Target | RNA Cargo | CCD Lipid | Stealth Lipid | Avg. Particle Size (nm) | pdi | EE (%) |
|---|---|---|---|---|---|---|---|
| LNP002 | N/A | eGFP mRNA | Lipid A | PEG2k-DMG | 71.8 | 0.073 | 80% |
| LNP006 | N/A | eGFP mRNA | Lipid A | PEG2k-C11 | 83.2 | 0.130 | 92% |
| LNP007 | N/A | eGFP mRNA | Lipid A | PEG2k-C11 | 94.5 | 0.122 | 90% |
| LNP010 | N/A | eGFP mRNA | Lipid A | PEG2k-DMG | 71.0 | 0.135 | 96% |
| LNP011 | N/A | eGFP mRNA | Lipid A | PEG2k-C11 | 78.9 | 0.138 | 96% |
| LNP012 | N/A | eGFP mRNA | Lipid B | PEG2k-DMG | 88.8 | 0.029 | 94% |
| LNP013 | N/A | eGFP mRNA | Lipid B | PEG2k-C11 | 88.1 | 0.056 | 95% |
| LNP014 | N/A | gLUC mRNA | Lipid A | PEG2k-DMG | 66.6 | 0.129 | 92% |
| LNP015 | N/A | gLUC mRNA | Lipid B | PEG2k-DMG | 110.4 | 0.191 | 90% |
| LNP093 | FVII | cr004* + tr002* | Lipid A | PEG2k-DMG | 97.67 | 0.173 | 79% |
| LNP094 | FVII | cr004* + tr002* | Lipid A | PEG2k-DMG | 83.09 | 0.159 | 92% |
| LNP095 | TTR | cr005* + tr002* | Lipid A | PEG2k-DMG | 131 | 0.219 | 86% |
| LNP096 | TTR | cr005* + tr002* | Lipid A | PEG2k-DMG | 77.66 | 0.138 | 96% |
| LNP097 | N/A | Cas9 mRNA | Lipid A | PEG2k-DMG | 90.02 | 0.118 | 88% |
| LNP116 | TTR | cr005* + tr002* | Lipid A | PEG2k-DMG | 136.3 | 0.202 | 56% |
| LNP120 | N/A | Cas9 mRNA | Lipid A | PEG2k-DMG | 85.8 | 0.123 | 94% |
| LNP121 | TTR | cr005* + tr002* | Lipid A | PEG2k-DMG | 77.8 | 0.150 | 94% |

(continued)

| LNP # | Target | RNA Cargo | CCD Lipid | Stealth Lipid | Avg. Particle Size (nm) | pdi | EE (%) |
|---|---|---|---|---|---|---|---|
| LNP123 | TTR | sg003 | Lipid A | PEG2k-DMG | 93.4 | 0.215 | 86% |
| LNP136 | TTR | cr005* + tr002* | Lipid A | PEG2k-DMG | 72.2 | 0.043 | 96% |
| LNP137 | TTR | cr005* + tr002* | Lipid A | PEG2k-DMG | 76.1 | 0.090 | 96% |
| LNP 13 8 | FVII | sg008** | Lipid A | PEG2k-DMG | 86.9 | 0.305 | 96% |
| LNP139 | TTR | sg003 | Lipid A | PEG2k-DMG | 84.5 | 0.324 | 97% |
| LNP 140 | N/A | Cas9 mRNA | Lipid A | PEG2k-DMG | 71.95 | 0.183 | 95% |
| LNP152 | FVII | sg013** + Cas9 mRNA | Lipid A | PEG2k-DMG | 97.5 | 0.092 | 95% |
| LNP153 | FVII | sg014** + Cas9 mRNA | Lipid A | PEG2k-DMG | 96.5 | 0.057 | 97% |
| LNP154 | TTR | sg015** + Cas9 mRNA | Lipid A | PEG2k-DMG | 96.4 | 0.060 | 97% |
| LNP155 | TTR | sg016** + Cas9 mRNA | Lipid A | PEG2k-DMG | 92.9 | 0.060 | 97% |
| LNP169 | TTR | sg017** + Cas9 mRNA | Lipid A | PEG2k-DMG | 81.8 | 0.098 | 98% |
| LNP170 | TTR | sg017** + Cas9 mRNA | Lipid A | PEG2k-DMG | 75.3 | 0.088 | 99% |
| LNP171 | TTR | sg017** + Cas9 mRNA | Lipid A | PEG2k-DMG | 100.7 | 0.062 | 97% |
| LNP 172 | N/A | Cas9 mRNA | Lipid A | PEG2k-DMG | 111.4 | 0.028 | 98% |
| LNP 173 | TTR | cr005* + tr002* | Lipid A | PEG2k-DMG | 58.3 | 0.087 | 98% |
| LNP 174 | TTR | cr005* + tr002* + Cas9 mRNA | Lipid A | PEG2k-DMG | 85.5 | 0.079 | 98% |
| LNP175 | TTR | cr005* + tr002* | Lipid A | PEG2k-DMG | 82.6 | 0.065 | 98% |
| LNP 176 | TTR | sg004 (DNA) | Lipid A | PEG2k-DMG | 65.82 | 0.064 | 100% |
| LNP178 | N/A | Cas9 mRNA | Lipid A | PEG2k-DMG | 115.8 | 0.072 | 97% |
| LNP294 | TTR | sg009* + Cas9 mRNA | Lipid A | PEG2k-DMG | 83.6 | 0.115 | 92% |
| *** = phosphorothioate linkages between 3 terminal nucleotides at the 5' and 3' ends<br>** = 2'-O-methyl modifications and phosphorothioate linkages at and between the three terminal nucleotides at the 5' and 3' ends** | | | | | | | |

### *T7E1 Assay*

A T7E1 assay was used in some Examples to detect mutation events in genomic DNA such as insertions, deletions and substitutions created through non-homologous end joining (NHEJ) following DNA cleavage by Cas9 (See, *e.g.,* Cho et al., Targeted genome engineering in human cells with the Cas9 RNA-guided endonuclease. Nature Biotechnology. 2013; 31, 230-232).

The genomic DNA regions targeted by CRISPR/Cas9 were amplified by PCR, denatured at 95° C for 10 minutes, and then re-annealed by ramping down the temperature from 95° C to 25° C at a rate of 0.5° C/second. The combination of DNA to form heteroduplexes indicated the presence of mutations in the amplified region. The re-annealed heteroduplexes were then digested with bacteriophage resolvase T7E1 (New England Biolabs) at 37° C for 25 minutes or longer to generate double-stranded breaks where the T7E1 nuclease recognized mismatches. The resulting DNA fragments were analyzed using a Fragment Analyzer and quantified to determine an approximation of editing efficiency. For quantitative analysis of editing efficiency, Next-Generation Sequencing was used as described herein.

### *Next-Generation Sequencing ("NGS") and Analysis for On-Target Cleavage*

### *Efficiency*

To quantitatively determine the efficiency of editing at the target location in the genome, deep sequencing was utilized to identify the presence of insertions and deletions introduced by gene editing.

PCR primers were designed around the target site *(e.g.,* TTR, FVII), and the genomic area of interest was amplified. Primer sequences are provided below. Additional PCR was performed according to the manufacturer's protocols (Illumina) to add the necessary chemistry for sequencing. The amplicons were sequenced on an Illumina MiSeq instrument. The reads were aligned to the human reference genome (*e.g.*, hg38) after eliminating those having low quality scores. The resulting files containing the reads were mapped to the reference genome (BAM files), where reads that overlapped the target region of interest were selected and the number of wild type reads versus the number of reads which contain an insertion, substitution, or deletion was calculated. The editing percentage (*e.g.*, the "editing efficiency" or "percent editing") is defined as the total number of sequence reads with insertions or deletions over the total number of sequence reads, including wild type.

### *LNP Delivery in vitro*

Mouse cells lines (Neuro2A and Hepa1.6 ) were cultured in DMEM media supplemented with 10% fetal bovine serum and were plated at a density of 15,000 cells/well 24 hours prior to transfection with LNPs for 18-24 hours prior to lysis and analysis as described herein (*e.g.*, reporter expression, T7E1 assay, NGS). Mouse primary hepatocytes (Invitrogen) were cultured at 15,000 cells per well in hepatocyte plating media (Invitrogen) using collagen coated 96 well plates. After 5 hours, the plating media was removed and replaced with hepatocyte maintenance media containing LNPs and 3% mouse serum (pre-incubated for 5 min at 37° C). Cells were transfected for 42-48 hours prior to lysis and analysis as described herein *(e.g.,* T7E1 assay, NGS). For both cell lines and primary hepatocytes the LNPs were diluted and added to cells starting at 100 ng Cas9 mRNA and approximately 30 nM guide RNA per well, carrying out serial dilutions in a semi-log manner down to 0.1 ng Cas9 mRNA and 0.03 nM guide RNA per well.

### *LNP Delivery in vivo*

CD-1 female mice, ranging from 6-10 weeks of age were used in each study. Animals were weighed and grouped according to body weight for preparing dosing solutions based on group average weight. LNPs were dosed via the lateral tail vein in a volume of 0.2 mL per animal (approximately 10 mL per kilogram body weight). The animals were observed at approximately 6 hours post dose for adverse effects. Body weight was measured at twenty-four hours post-administration, and animals were euthanized at various time points by exsanguination via cardiac puncture under isoflourane anesthesia. Blood was collected into serum separator tubes or into tubes containing buffered sodium citrate for plasma as described herein. For studies involving *in vivo* editing, liver tissue was collected from the median lobe or from three independent lobes *(e.g.,* the right median, left median, and left lateral lobes) from each animal for DNA extraction and analysis. For some studies, spleen tissue was also collected.

### *Genomic DNA Isolation*

Genomic DNA was extracted from 10 mg of tissue using Invitrogen PureLink Genomic DNA Kit (Cat. K1820-02) according to manufacturer's protocol, which includes homogenizing the tissue in lysis buffer (approximately 200 μL/10 mg

tissue) and precipitating the DNA. All DNA samples were normalized to 100 ng/μL concentration for PCR and subsequent NGS analysis, as described herein.

***Transthyretin (TTR) ELISA analysis***

Blood was collected and the serum was isolated as indicated. The total TTR serum levels were determined using a Mouse Prealbumin (Transthyretin) ELISA Kit (Aviva Systems Biology, Cat. OKIA00111). Kit reagents and standards were prepared according to the manufacture's protocol. Mouse serum was diluted to a final dilution of 10,000-fold with 1x assay diluent. This was done by carrying out two sequential 50-fold dilutions resulting in a 2,500-fold dilution. A final 4-fold dilution step was carried out for a total sample dilution of 10,000-fold. Both standard curve dilutions (100 μL each) and diluted serum samples were added to each well of the ELISA plate pre-coated with capture antibody. The plate was incubated at room temperature for 30 minutes before washing. Enzyme-antibody conjugate (100 μL per well) was added for a 20 minute incubation. Unbound antibody conjugate was removed and the plate was washed again before the addition of the chromogenic substrate solution. The plate was incubated for 10 minutes before adding 100 μL of the stop solution, *e.g.*, sulfuric acid (approximately 0.3 M). The plate was read on a SpectraMax M5 plate reader at an absorbance of 450 nm. Serum TTR levels were calculated by SoftMax Pro software ver. 6.4.2 using a four parameter logistic curve fit off the standard curve. Final serum values were adjusted for the assay dilution.

***Factor-VII (FVII) Activity Assay***

Blood was collected for the plasma as indicated. Plasma Factor VII activity levels were measured using BIOPHEN FVII assay kit (Anaria Diagnostics, Cat. A221304). Kit reagents were prepared according to the manufacturer's protocol. Plasma was diluted 10,000-fold with the kit sample dilution buffer by carrying out two sequential 50-fold dilutions resulting in a 2,500-fold dilution. A final 4-fold dilution step was carried out for a total sample dilution of 10,000-fold. Diluted sample (30 μL) was added to kit reagent 1 (30 μL). Next, kit reagent 2 (60 μL) was added to the plate, which was subsequently incubated at 37° C for 7 minutes. Kit reagent 3 (60 μL) was then added to the plate and the plate was incubated for an additional 5 minutes at 37° C, before adding acetic acid (20% v/v in water, 60 μL) to stop the enzyme reaction. The plate was read on a SoftMax M5 plate reader at 405 nM. The relative values of FVII activity were calculated based upon a calibration curve prepared from plasma of control animals and reported as a percent of vehicle control.

Example 2. *In vitro* delivery of eGFP mRNA encapsulated LNPs.

LNPs comprising mRNA encoding eGFP (TriLink, Cat. L-6101) were prepared as described in Example 1. The components of each LNP preparation include a CCD lipid (45 mol-%), cholesterol (44 mol-%), DSPC (9 mol-%), and PEG2k-DMG or PEG2k-C11 (2 mol-%). LNP-002, -006, -007, -010, and -011 include Lipid A as the CCD lipid, whereas LNP-012 and -013 include Lipid B as the CCD lipid. LNP-002, -010, and -012 include PEG2k-DMG, and LNP-006, -007, -011, and -013 include PEG2k-C11. LNP details are provided in Table 1, including average particle size, polydispersity, and encapsulation efficiency. LNPs were delivered to a mouse hepatocyte cell line (Hepa1.6) as described in Example 1, with total amounts of eGFP mRNA delivered being either 100 ng or 500 ng per well, for each LNP. Cells were incubated with LNPs for approximately 18 hours, and eGFP expression was measured using a CytoFLEX Cell Analyzer (Beckman Coulter).

As shown in Fig. 1, eGFP expression was observed for each formulation. LNP formulations comprising Lipid A (LNP-002, -006, -007, -010, and -011) successfully delivered eGFP mRNA. LNP formulations comprising Lipid B (LNP-012 and -013) also delivered eGFP mRNA. LNPs that include PEG2k-C11 and PEG2k-DMG stealth lipids both deliver mRNA effectively in these experiments, demonstrating delivery of mRNA to a mouse hepatocyte cell line using LNPs *in vitro.*

Example 3. *In vivo* delivery of gLUC mRNA encapsulated LNPs.

LNPs comprising mRNA encoding *Gaussia* luciferase (gLUC) (TriLink, Cat. L-6123) were prepared as described in Example 1 and tested for mRNA delivery to animals *in vivo.* The components of each LNP preparation include a CCD lipid (45 mol-%), cholesterol (44 mol-%), DSPC (9 mol-%), and PEG2k-DMG (2 mol-%). LNP-014 included Lipid A, whereas LNP-015 included Lipid B. Details for these formulations are provided in Table 1, such as average particle size, polydispersity, and encapsulation efficiency. gLUC mRNA doses of 0.1 mg/kg and 0.3 mg/kg were delivered with each LNP formulation. The animals were euthanized 24 hours later with blood collection and serum isolation performed as described in Example 1. Serum luciferase expression was measured using a Pierce™ Gaussia Luciferase Flash Assay Kit (ThermoFisher Scientific, catalog number 16158) according to the manufacturer's protocol.

As shown in Fig. 2, dose dependent increases in gLUC expression were observed for each animal (n=5 for each

group) as compared to a PBS control. LNPs comprising either Lipid A or Lipid B showed effective *in vivo* delivery and expression of mRNA as measured by luciferase activity.

Example 4. *In vivo* delivery and editing using Cas9 mRNA encapsulated LNPs (mRNA-LNP) with dual guide RNA encapsulated LNPs (dgRNA-LNP).

LNPs for delivering CRISPR/Cas RNA components (e.g., gRNA and mRNA encoding Cas9) for *in vivo* editing in the liver were tested in CD-1 mice. In these experiments, dgRNA and mRNA were formulated separately.

LNPs were formulated with *in vitro* transcribed Cas9 mRNA and chemically modified dgRNA (targeting either TTR or FVII), separately, as described in Example 1. The dgRNAs used in this Example were chemically synthesized and sourced from commercial suppliers, with phosphorothioate linkages between the three terminal nucleotides at both the 5' and 3' ends of the crRNA and the trRNA making up the dual guide. The components of each LNP preparation (LNP-093, -094, -095, -096, and -097) include a CCD lipid (Lipid A) (45 mol-%), cholesterol (44 mol-%), DSPC (9 mol-%), and PEG2k-DMG (2 mol-%). Details for these formulations are provided in Table 1, including average particle size, polydispersity, and encapsulation efficiency. Two different dosing regimens were employed: (1) combining the mRNA-LNP formulation (LNP-097) and a dgRNA-LNP formulation (LNP-093, -094, -095 or -096) together in equal parts (by weight of RNA) and dosing the combined formulation on two consecutive days (each day dosed at 1 mg /kg of each RNA component formulation, for a total of 2 mg/kg); or (2) dosing the mRNA-LNP (LNP-097) four hours prior to dosing a dgRNA-LNP (LNP-093, -094, -095, and -096), on two consecutive days (each formulation dosed at 1 mg/kg). The animals were euthanized 5 days following the first dose in each group. In addition to control group comparisons (animals receiving PBS), each experimental group had an internal sequencing control, and PCR reactions for NGS analysis, as described in Example 1, were run for both targets in each animal (n=3 for each group). Genomic DNA from liver was isolated and analyzed by NGS, as described in Example 1.

As shown in Figs. 3A and 3B, *in vivo* editing (approximately 1.8% editing - 2.8% editing) was observed in the livers of animals that received LNPs targeting FVII using either a co-dosing (A1 (LNP-093/-097) or A2 (LNP-094/-097)) or pre-dosing (A3 (LNP-093/-097)) dosing regimen. Animals that received LNPs targeting TTR showed approximately 2% - 4.5% editing in the livers of animals receiving dgRNA co-dosed with Cas9 mRNA (B1 (LNP-095/LNP-097) or B2 (LNP-096/-097)) or when pre-dosed (B3 (LNP-095/-097) or B4 (LNP-096/-097)). Serum and plasma analyses were conducted for all of the animals, as described in Example 1, with none of the animals displaying statistically significant differences (as compared to animals administered PBS) in either total serum levels of TTR or plasma FVII activity (not shown).

Example 5. *In vitro* and *in vivo* delivery and editing using dgRNA-LNPs and IVT sgRNA-LNPs.

The efficacy of LNPs comprising chemically modified dgRNA and LNPs comprising *in vitro* transcribed (IVT) sgRNA were tested in the context of co-dosing with Cas9 mRNA-LNPs.

LNP-115, -116, -117, -120, -121, and -123 were formulated according to Example 1, and the details about the specific formulations are provided in Table 1. The formulations of this Example were tested for delivery to Neuro2A cells, using the procedure as described in Example 1.

LNP-121 (gRNA) and LNP-120 (Cas9 mRNA) were mixed together and administered at gRNA concentrations of 152 nM, 76 nM, and 38 nM, plus mRNA at 570 ng, 285 ng, and 142 ng per well, respectively; LNP-123 (gRNA) and LNP-120 were mixed together and administered at gRNA concentrations of 156 nM, 78 nM, and 39 nM, plus mRNA at 528 ng, 264 ng, and 132 ng per well, respectively; and LNP-116 (gRNA) was mixed with LNP-120 (Cas9 mRNA) and administered at gRNA concentrations of 124 nM, 62 nM, and 31 nM, plus mRNA at 460 ng, 229 ng, and 114 ng per well, respectively.

LNP-121 was administered at gRNA concentrations of 198 nM, 99 nM, and 49.5 nM; LNP-123 was administered at gRNA concentrations of 189 nM, 94.5 nM, and 47 nM; and LNP-116 was administered at gRNA concentrations of 124 nM, 62 nM, and 31 nM, and the Cas9 mRNA (100 ng per well) was added by LF2K to the experiments according to the manufacturer's instructions. Editing was observed in samples involving both co-dosing IVT sgRNA-LNP (LNP-123) with Cas9 mRNA using either an LNP (LNP-120) or LF2K, as well as with chemically modified dgRNA at the tested concentrations of gRNA (LNP-121).

The formulations in this Example were then tested *in vivo.* Animals were administered, as described in Example 1, a mixture of Cas9 mRNA-LNP and one of the gRNA-LNPs (animals were administered 1 mg/kg of each formulation, each day) on two consecutive days, with one formulation being dosed on one day only (n=5 for each group). The animals were euthanized 6 days following the first dose (or 7 days with the group receiving only a single dose), and liver tissues were collected and analyzed by NGS, as described in Example 1.

As shown in Fig. 4A, single and dual doses were effective for delivery. There is no statistical difference between the group that received one dose on a single day (LNP-121 and LNP-120; C in Fig. 4A) and the group that received two doses on consecutive days when co-dosing Cas9 mRNA-LNP with chemically modified dgRNA-LNPs (LNP-116 and

LNP-120, A in Fig. 4A; LNP-121 and LNP-120, B in Fig. 4B). Animals that received Cas9 mRNA-LNP co-dosed with unmodified IVT sgRNA-LNPs (LNP-123 and LNP-120, D in Fig. 4B) displayed relatively lower levels of editing as compared to the dgRNA-LNPs used in this Example (Fig. 4B). These experiments establish that LNPs comprising modified dgRNA or IVT sgRNA allow for *in vitro* and *in vivo* editing when co-dosed with Cas9 mRNA-LNPs. The levels of *in vivo* editing observed when using LNPs comprising IVT sgRNA in this experiment may be affected by impurities in the isolated IVT sgRNA.

Example 6. *In vitro* and *in vivo* delivery and editing using modified dgRNA-LNPs or modified sgRNA-LNPs.

LNPs comprising chemically modified dgRNA and LNPs comprising chemically modified sgRNA were also tested by co-dosing with Cas9 mRNA-LNPs.

LNPs were formulated with chemically modified dgRNA (targeting TTR or FVII), chemically modified sgRNA (targeting TTR or FVII), and IVT Cas9 mRNA, as described in Example 1. The dgRNA in this Example were chemically synthesized and sourced from commercial suppliers, with phosphorothioate linkages between the three terminal nucleotides at both the 5' and 3' ends of both the crRNA and the trRNA making up the dual guide. The sgRNA in this Example was also chemically synthesized and sourced from a commercial supplier with 2'-O-methyl modifications and phosphorothioate linkages at and between the three terminal nucleotides at both the 5' and 3' ends of the sgRNA. The components of each LNP preparation include a CCD lipid (Lipid A, 45 mol-%), cholesterol (44 mol-%), DSPC (9 mol-%), and PEG2k-DMG (2 mol-%). LNP-136, -137, -138, -139, and -140 were used in these experiments.

Details are provided in Table 1, including average particle size, polydispersity, and encapsulation efficiency.

The formulations of this Example were tested for delivery to Neuro2A cells, as described in Example 1. Cells were co-transfected with guide LNP and Cas9 mRNA LNP by adding each formulation directly to the cell culture media, resulting in the concentrations listed in Table 2, and percent editing was determined using the T7E1 assay, as described in Example 1. In Fig. 5, the labels represent the formulations, as described in Table 2.

**Table 2. Formulations Employed in Example 6**

| Figure Label | guide LNP | Guide Concentration (nM) | Cas9 mRNA (ng) LNP 140 |
|---|---|---|---|
| A1 | LNP-136 | 66 | 245 |
| A2 | | 33 | 122.5 |
| A3 | | 16.5 | 61 |
| B1 | LNP-139 | 54 | 175 |
| B2 | | 27 | 87.5 |
| B3 | | 13.5 | 43 |
| C1 | LNP-137 | 49 | 343 |
| C2 | | 24.5 | 171.5 |
| C3 | | 12 | 85 |
| D1 | LNP-138 | 74 | 239 |
| D2 | | 37 | 119.5 |
| D3 | | 18.5 | 60 |

Large increases in editing were measured for both targets when using chemically modified sgRNA-LNPs co-transfected with Cas9 mRNA-LNPs, when compared to the dgRNA-LNP formulations that were tested (Fig. 5). The chemically modified sgRNA-LNPs co-transfected with Cas9 mRNA-LNPs (LNP-138 and -139, Fig. 5), resulted in approximately 35-50% and 65-70% editing in cells when targeting FVII and TTR, respectively.

The formulations in this Example were then tested *in vivo.* Animals were administered, as described in Example 1, a mixture of Cas9 mRNA-LNP (LNP-140) and one of the gRNA-LNPs tested (LNP-136, -137, -138, and -139), where each component formulation was dosed at 1 mg/kg/day (for a total of 2 mg/kg/day), on two consecutive days (n=5 for each group). The animals were euthanized 6 days following the first dose, and liver tissues were collected and analyzed by NGS, as described in Example 1. In Fig. 6, A1 and A2 represent administration of the mixture of formulations LNP-136 and LNP-140; B1 and B2 represent administration of the mixture of formulations LNP-139 and LNP-140; C1 and C2 represent administration of the mixture of formulations LNP-137 and LNP-140; and D1 and D2 represent administration of the mixture of formulations LNP-138 and LNP-140. As shown in Fig. 6, increases in editing (approximately 10% editing-32%

editing) were measured for both targets when using chemically modified sgRNA-LNPs co-dosed with Cas9 mRNA-LNPs, as compared to the amount of editing the use of dgRNA-LNP formulations resulted in (approximately 2% editing-5% editing). Animals receiving the dgRNA-LNP formulations targeting TTR resulted in less than 5% editing across two liver biopsies, while sgRNA-LNP formulations resulted in average percent editing of over 20% (with a peak of over 30% in one animal). Similarly, animals receiving the dgRNA-LNP formulations targeting FVII displayed less than 3% editing across two liver biopsies, while sgRNA-LNP formulations resulted in average percent editing of approximately 10% (with a peak of over 12% in one animal).

These results established that LNPs separately formulated with Cas9 mRNA and gRNA (both dgRNA and sgRNA) achieve editing *in vivo* when co-dosed together, and the LNPs achieve editing *in vivo* when Cas9 mRNA-LNPs are dosed prior to gRNA-LNPs.

<u>Example 7. *In vitro* and *in vivo* delivery and editing using LNPs comprising sgRNA co-formulated with Cas9 mRNA.</u>

LNPs formulated for delivery of Cas9 mRNA and sgRNA encapsulated together in an LNP composition also effectively deliver the CRISPR/Cas components.

LNPs were formulated with IVT Cas9 mRNA together with chemically modified sgRNA (targeting TTR or FVII), as described in Example 1. The ratio of mRNA:sgRNA was approximately 1:1, by weight of the RNA component. The sgRNA in this Example was chemically synthesized and sourced from a commercial supplier, with 2'-O-methyl modifications and phosphorothioate linkages at and between the three terminal nucleotides at both the 5' and 3' ends of the sgRNA, respectively. The components of each LNP preparation include a CCD lipid (Lipid A, 45 mol-%), cholesterol (44 mol-%), DSPC (9 mol-%), and PEG2k-DMG (2 mol-%). LNP-152, - 153, -154, and -155 were used in these experiments, and details of these formulations are provided in Table 1, including average particle size, polydispersity, and encapsulation efficiency.

The formulations of this Example were tested for delivery to Neuro2A cells, as described in Example 1. Cells were transfected with the formulations and percent editing was determined using NGS, as described in Example 1.

In Fig. 7, A represents administration of LNP-152; B represents administration of LNP-153; C represents administration of LNP-154; D represents administration of LNP-155; and E represents administration of a combination of LNP-152 and LNP-153. Each formulation was administered at 300 ng Cas9 mRNA and 93 nM gRNA; 100 ng Cas9 mRNA and 31 nM gRNA; 30 ng Cas9 mRNA and 10 nM gRNA; and 10 ng Cas9 mRNA and 3 nM gRNA. As shown in Fig. 7, administration of each LNP formulation resulted in robust editing efficiency, with some formulations resulting in more than 80% of cells being edited (LNP-153 and -155). Cells were treated with a combination of two of the LNP formulations (LNP-152 and LNP-153) targeting FVII, which also resulted in efficient editing (approximately 70-90% editing), as well as excision of a portion of the FVII gene lying between the two sgRNAs delivered (Fig. 7, and data not shown).

The formulations in this Example were also tested *in vivo.* Animals were dosed as described in Example 1 (n=4 for each group). Treatment groups receiving LNPs targeting FVII received a single dose (at 2 mg/kg), with one of the treatment groups having received a single, combined dose (LNP-152 and LNP-153) of 2 mg/kg *(e.g.,* 1 mg/kg of each of LNP-152 and LNP-153). The treatment groups receiving LNPs targeting TTR received two doses (each at 2 mg/kg), wherein the second dose was delivered four days after the first dose *(i.e.,* dose 1 on day 1, dose 2 on day 5). Animals in all groups were euthanized 8 days following the first dose with blood and liver tissues collected and analyzed as described in Example 1. Each formulation was administered to four animals.

In Figs. 8A, 8B, and 9, A represents administration of LNP-152; B represents administration of LNP-153; and C represents administration of a combination of LNP-152 and LNP-153. Each formulation was tested in four animals. As shown in Fig. 8A and 8B, each LNP formulation that was tested resulted in robust *in vivo* editing efficiencies. For animals treated with LNP formulations targeting a TTR sequence, more than 50% of liver cells from each biopsy for some animals displayed indels at the target site, with overall averages (across all biopsies of all animals) for each treatment group of 45.2 $\pm$ 6.4% (LNP-154) and 51.1 $\pm$ 3.7% (LNP-155) (Fig. 8A).

Animals treated with LNPs targeting an FVII sequence displayed a range of percentage editing in liver biopsies, with a maximum observed editing of greater than 70% of liver cells being edited from biopsy samples (e.g., having either an indel or excision event at or between the target site(s)) for one animal receiving both LNP formulations targeting an FVII sequence. Overall averages (across all biopsies of all animals) for each treatment group (LNP-152, LNP-153, and LNP-152 and LNP-153) were 16.9 $\pm$ 6.5%, 38.6 $\pm$ 13.2%, and 50.7 $\pm$ 15.0%, respectively (Fig. 8B). For animals receiving both FVII-targeting LNPs, excision of the intervening genomic DNA between the target sites for each sgRNA was detected by PCR, as were indels at one or both of the target sites (Fig. 9).

In Figs. 10 and 11, A represents administration of LNP-152; B represents administration of LNP-153; C represents administration of LNP-154; D represents administration of LNP-155; and E represents administration of the combination of LNP-152 and LNP-153. The robust *in vivo* editing that was observed when the LNP formulations were administered in this Example also resulted in phenotypic changes. As shown in Fig. 10, large decreases (of up to approximately 75%) in serum TTR levels were observed in animals treated with LNPs targeting a TTR sequence (but not in controls or animals treated with LNPs targeting FVII). Similarly, reduced levels of plasma FVII activity were observed in

animals treated with LNPs targeting FVII (but not in controls or animals treated with LNPs targeting TTR) (Fig. 11).

Example 8. Variation of Formulation Parameters.

LNPs formulated for delivery of Cas9 mRNA and gRNA together in one formulation were tested (1) across a range of doses; (2) with altered ratios of mRNA:gRNA; (3) for efficacy with a single dose versus two doses; and (4) whether the LNPs are taken up by and result in editing in the spleen.

LNPs were formulated with IVT Cas9 mRNA together with chemically modified sgRNA (targeting TTR), as described in Example 1. The ratios tested (by weight of RNA component) of mRNA:sgRNA were approximately 1:1 (LNP-169), approximately 10:1 (LNP-170), or approximately 1:10 (LNP-171). The sgRNA used in this Example comprises 2'-O-methyl modifications and phosphorothioate linkages at and between the three terminal nucleotides at both the 5' and 3' ends of the sgRNA, respectively. The components of each LNP preparation included Lipid A (45 mol-%), cholesterol (44 mol-%), DSPC (9 mol-%), and PEG2k-DMG (2 mol-%). LNP-169, -170 and LNP-171 were used in these experiments. Details are provided in Table 1, including average particle size, polydispersity, and encapsulation efficiency.

### *Dose Response Study*

In this study, animals were dosed on day 1, as described in Example 1, with LNP-169 at doses of 0.1 mg/kg, 0.5 mg/kg, or 2 mg/kg (n=5 for each group). On days 5 and 9 blood was collected for TTR serum level analysis. Liver and spleen were collected at necropsy on day 9 for NGS analysis, as described in Example 1.

As shown in Fig. 12A, administration of all three doses resulted in significant editing efficiency in the liver, with a linear dose response observed having an $r^2$ value of 0.9441. In the highest dose group (2 mg/kg), nearly 60% of liver cells in one animal were edited at the TTR target site, with the group having an average of about 50% of liver cells edited. Each animal that was administered the highest dose also displayed statistically significant reductions in serum TTR levels when measured at days 5 and 9 post-administration, with an average reduction 75% of serum TTR levels (as compared to animals that were administered PBS; Fig. 12B).

### *Altering Ratios of mRNA:gRNA*

On day 1, animals were administered, as described in Example 1, LNP-169 at a mRNA:gRNA ratio of 1:1 *(i.e.,* 1 mg/kg mRNA, 1 mg/kg gRNA, for a total RNA dose of 2 mg/kg), LNP-170 at a ratio of 10:1 *(i.e.,* 1.8 mg/kg of mRNA, 0.18 mg/kg of gRNA, for a total RNA dose of 1.98 mg/kg) or LNP-171 at a ratio of 1:10 *(i.e., 0.18* mg/kg mRNA, 1.8 mg/kg gRNA, for a total RNA dose of 1.98 mg/kg) (n=5 for each group). (Note: The group and data receiving a dose with a 1:1 mRNA:gRNA ratio is the same group and data as described in the dose response study in this Example, *supra*.) Blood was collected on days 5 and 9, and the serum TTR levels were measured. Liver and spleen were collected at necropsy on day 9 for NGS analysis, as described in Example 1.

As shown in Fig. 13A, administration of LNP-169 (mRNA:gRNA ratio of 1:1) resulted in editing of nearly 60% in one animal at the TTR target site, with the group having an average of about 50% editing. Animals that received 1:10 and 10:1 LNP formulations also demonstrated editing, with the average percent editing for the group receiving LNP-171 showing approximately 32% editing and the group receiving LNP-170 showing approximately 17% editing in this experiment. Additionally, as shown in Fig. 13B, statistically significant reductions in serum TTR levels were detected for each treatment group at day 5 (as compared to PBS control). By day 9, the groups receiving 1:1 mRNA:sgRNA and 1:10 mRNA:sgRNA retained statistically significant reductions in serum TTR levels.

### *Single Dose versus Two Doses*

In this study, one group of animals received a single dose of LNP-169 (at 2 mg/kg) on day 1, while another group received two doses of LNP-169 (each at 2 mg/kg) with the first dose administered on day 1 and the second dose on day 5, administered as described in Example 1 (n=5 for both groups). (Note: The group and data receiving a single dose of LNP-169 is the same group and data as described in the dose response and mRNA:gRNA ratio studies in this Example, *supra*). Blood was collected for TTR serum levels from both groups at day 5 (prior to administration of the second dose for the group receiving the second dose), and again at necropsy on day 9, as described in Example 1.

As shown in Fig. 14A, in the group receiving a single dose of LNP-169, nearly 60% editing of the TTR target site was observed in one animal, with the group having an average of about 50% editing. Similar average numbers were achieved in animals receiving two doses of LNP-169, with lower standard deviation and with the group averaging approximately 55% editing of the TTR target site. As shown in Fig. 14B, both groups displayed significant reductions in serum TTR levels.

***Evaluating Uptake by and Editing in the Spleen***

The spleen from each animal in the above studies (within this Example) were collected at necropsy in order to determine whether the LNPs were directed to and taken up by the spleen, thereby resulting in gene editing. Genomic DNA was extracted from spleen tissues and subjected to NGS analysis as described in Example 1.

In Fig. 15, A represents LNP-169 administered at 2 mg/kg for 2 doses; B represents LNP-169 with a 1:1 ratio of mRNA:gRNA at 0.1 mg/kg as a single dose; C represents LNP-169 with a 1:1 ratio of mRNA:gRNA at 0.5 mg/kg as a single dose; D represents LNP-169 with a 1:1 ratio of mRNA:gRNA at 2 mg/kg as a single dose; E represents LNP-170 with a 10:1 ratio of mRNA:gRNA at 2 mg/kg as a single dose; and F represents LNP-171 with a 1:10 ratio of mRNA:gRNA at 2 mg/kg as a single dose. As shown in Fig. 15, significantly less editing (less than approximately 2% of cells) was observed in the spleens of these animals as compared to their livers. Editing of approximately 50% in the liver was observed (e.g., in those groups receiving LNP-169) in these studies. These results indicate that the LNPs provided herein are largely targeted to the liver, as opposed to the spleen.

Example 9. Comparative *in vivo* study between (1) modified dgRNA-LNPs co-dosed with Cas9 mRNA-LNPs and (2) LNPs comprising Cas9 mRNA and modified dgRNA together in one formulation.

LNPs formulated for delivery of Cas9 mRNA and modified dgRNA either as separate LNPs or together in one formulation effectively deliver the CRISPR/Cas components. LNPs were formulated with IVT Cas9 mRNA either together with (LNP-174, -175) or separately from (LNP-172, -173) chemically modified dgRNA (targeting TTR), as described in Example 1. Both the crRNA and the trRNA making up the dgRNA in this Example comprised phosphorothioate linkages between the three terminal nucleotides at both the 5' and 3' ends of each RNA. The components of each LNP preparation include a CCD lipid (Lipid A, 45 mol-%), cholesterol (44 mol-%), DSPC (9 mol-%), and PEG2k-DMG (2 mol-%). LNP-172, -173, -174, and -175 were used in these experiments. The compositions of LNP-174 and LNP-175 were identical, except that the crRNA and trRNA making up the dgRNA in LNP-175 were first pre-annealed to one another prior to being formulated with the LNP. This was accomplished by first incubating the crRNA and trRNA together at 95° C for 10 minutes before cooling to room temperature and proceeding to formulation, as previously described. Other details concerning the LNPs are provided in Table 1, including average particle size, polydispersity, and encapsulation efficiency.

Animals were dosed with each LNP at 2 mg/kg as described in Example 1 (n=5 for each group). Livers were collected at necropsy 8 days post-administration, and genomic DNA was isolated and subjected to NGS analysis, as described in Example 1.

In Fig. 16, A represents administration of the dgRNA split-formulation (LNP-172 and LNP-173; B represents administration of the dgRNA co-formulation (LNP-174); and C represents administration of the formulation wherein the dgRNA was pre-annealed (LNP-175). As shown in Fig. 16, editing was detected in livers from each group (with approximately 4-6% editing). Animals that received LNP that was co-formulated with Cas9 mRNA and dgRNA together and animals that received the mRNA and dgRNA from separately formulated LNPs showed editing. The editing efficiencies measured using LNPs formulated with dgRNA (either together with or separately from Cas9 mRNA) are substantially lower than those detected using LNPs formulated with sgRNA (*see, e.g.,* Examples 6-8).

Example 10. ApoE binding of LNPs and transfection of primary hepatocytes.

As demonstrated in Example 8, LNPs provided herein are effectively taken up by the liver, and only to a minor extent by the spleen. This Example provides data regarding ApoE-mediated uptake in primary hepatocytes and provides an assay for testing LNP-ApoE binding which demonstrated that the LNPs bind ApoE.

***LNP delivery to primary hepatocytes***

In addition to other proteins, serum provides a source of ApoE in culture media, and therefore whether the LNPs require serum (*e.g.*, as a source of ApoE) for uptake into primary hepatocytes was tested. This was accomplished by adding LNPs to primary hepatocytes *in vitro,* with and without the presence of serum.

LNPs were delivered to mouse primary hepatocytes as described in Example 1. In the absence of any serum, no editing was detected by T7E1 assay for any LNP tested (data not shown). However, when LNPs were incubated with 3% mouse serum prior to transfection, LNPs were taken up by the hepatocytes resulting in editing. A representative data set is shown in Fig. 17. In this experiment, LNP-169 (targeting TTR) was pre-incubated in 3% mouse serum, and then added to mouse primary hepatocytes at various concentrations. The labels in Fig. 17 are defined in Table 3 and describe the concentration of the LNP-169 that was administered. As shown in Fig. 17, the addition of serum resulted in a dose dependent increase in editing at the TTR target site as measured by NGS. These results suggest that ApoE present in the serum mediates LNP uptake in hepatocytes.

**Table 3. Concentration of LNP-169 Administered**

| Label | nM gRNA | ng Cas9 mRNA |
|---|---|---|
| **A** | 30.8 | 99.9 |
| **B** | 10.3 | 33.3 |
| **C** | 3.4 | 11.1 |
| **D** | 1.1 | 3.7 |
| **E** | 0.4 | 1.2 |
| **F** | 0.1 | 0.4 |
| **G** | 0.0 | 0.1 |

### *ApoE binding assay*

LNPs were incubated with recombinant ApoE3, the most common form of ApoE, and then separated with a heparin affinity column using a salt gradient on an HPLC. There were two peak groups in the HPLC run, corresponding to LNPs bound to ApoE3 and unbound LNPs. Un-bound is free LNP that did not bind with ApoE3 and flowed freely though the heparin column. Bound was a peak with a longer retention time representing the LNP/ApoE3 complex that was bound to the heparin column and was eluted in the salt gradient. To calculate the binding, the percentage of the bound peak area was calculated by dividing the peak area corresponding to the LNPs bound to ApoE3 and dividing that number by the sum of the area of both peaks.

LNPs were formulated with Cas9 mRNA and chemically modified sgRNA, as described in Example 1. The sgRNAs used in this Example were chemically synthesized and sourced from commercial suppliers, with 2'-O-methyl modifications and phosphorothioate linkages at and between the three terminal nucleotides at both the 5' and 3' ends of the sgRNA, respectively. The components of each LNP preparation (LNP-169 and LNP-171) include Lipid A (45 mol-%), cholesterol (44 mol-%), DSPC (9 mol-%), and PEG2k-DMG (2 mol-%). Details for these formulations are provided in Table 1, including average particle size, polydispersity, and encapsulation efficiency. Using a stock ApoE3 (Recombinant Human Apolipoprotein E3 , R&D Systems, cat #4144-AE-500) solution at 0.5 mg/mL, ApoE3 was added to LNP samples at 25 μg/mL, 50 μg/mL, 100 μg/mL, 200 μg/mL, and 300 μg/mL. The samples were incubated overnight at room temperature.

Two buffers were prepared (500 mL each); Buffer A is a 20 mM Tris buffer, adjusted to pH 8.0 and Buffer B is a 20 mM Tris buffer, with 1 M NaCl, adjusted to pH 8.0. The gradient and flow rate for the HPLC analysis is as described below.

| No | Time | Flow [ml/min] | %B | Curve |
|---|---|---|---|---|
| 1 | 0.000 | Run | | |
| 2 | 0.000 | 0.200 | 0.0 | 5 |
| 3 | 8.000 | 0.200 | 0.0 | 5 |
| 4 | 10.000 | 0.200 | 80.0 | 5 |
| 5 | 13.000 | 0.200 | 80.0 | 5 |
| 6 | 15.000 | 0.200 | 100.0 | 5 |
| 7 | 18.000 | 0.200 | 100.0 | 5 |
| 8 | 19.000 | 0.200 | 0.0 | 5 |
| 9 | 25.000 | 0.200 | 0.0 | 5 |
| 10 | *New Row* | | | |
| 11 | 25.000 | Stop Run | | |

After incubating the samples overnight, each sample was analyzed by HPLC and the percent area of the bound peak was calculated as previously described.

As shown in Fig. 18, with increasing amounts of ApoE3, more LNP (both LNP-169 (represented by the dashed line) and -171 (represented by the solid line)) was bound to the heparin column, *e.g.,* as a result of being bound to ApoE3. These results indicate that the LNPs bind ApoE3.

Example 11. *In vitro* and *in vivo* delivery and editing using LNPs with sgRNA expressed from DNA expression cassettes.

This example demonstrates gene editing using LNPs loaded with Cas9 mRNA and an expression cassette encoding an sgRNA.

***LNP delivery in vitro***

Amplicons encoding sgRNA were prepared by PCR amplification of a DNA sequence containing a U6 promoter linked to as sgRNA targeting mouse TTR. Each primer contained an inverted dideoxyT nucleotide at the 5' end to prevent integration of the DNA amplicon into genomic DNA. PCR product was purified by phenol/chloroform extraction followed by ethanol precipitation. The DNA pellet was dried and resuspended in TE buffer.

LNPs were formulated with IVT Cas9 mRNA ("mRNA-LNP" or LNP-178) or the sgRNA expression cassette ("DNA-LNP" or LNP-176) as described in Example 1. IVT Cas9 mRNA and the sgRNA expression cassette were also separately formulated with Lipofectamine 2000 (Thermo Fisher) according to manufacturer's instructions ("mRNA LF2K" or "DNA LF2K", respectively). Formulations were applied to mouse Neuro2A cells (100 ng Cas9 mRNA and 100 ng sgRNA expression cassette) by diluting directly into the cell culture media in each well according to the following regimens:

- Co-delivery of Cas9 mRNA and sgRNA expression cassette;
- sgRNA expression cassette administered 2 hours prior to Cas9 mRNA; and
- Cas9 mRNA administered 2 hours prior to sgRNA expression cassette.

Cells were incubated for 48 hours post transfection, and cell lysates were analyzed by T7E1 analysis as described in Example 1. As shown in Fig. 19, higher percentages of TTR editing were observed when both the mRNA and DNA components were formulated in LNPs, compared to when one component or the other was formulated with Lipofectamine 2000.

Example 12. Editing *in vitro* vs. *in vivo.*

Cas9 mRNA and chemically modified sgRNA targeting different mouse TTR sequences were formulated and dosed to mice (2 mg/kg) as described in Example 1. The same LNP preparations were used to transfect mouse primary hepatocytes *in vitro.* The sgRNA in this Example was chemically synthesized and sourced from a commercial supplier, with 2'-O-methyl modifications and phosphorothioate linkages at and between the three terminal nucleotides at both the 5' and 3' ends of the sgRNA, respectively.

Table 4. Formulations Employed in Example 12

| LNP # | Target | RNA Cargo | CCD Lipid | Stealth Lipid | Avg. Particle Size (nm) | pdi | EE (%) |
|---|---|---|---|---|---|---|---|
| LNP257 (TTR686) | TTR | sg009 + Cas9 mRNA | Lipid A | PEG2k-DMG | 77.86 | 0.015 | 99% |
| LNP258 (TTR705) | TTR | sg016 + Cas9 mRNA | Lipid A | PEG2k-DMG | 88.24 | 0.033 | 99% |
| LNP259 (TTR268) | TTR | cr013*** + Cas9 mRNA | Lipid A | PEG2k-DMG | 81.74 | 0.070 | 99% |
| LNP260 (TTR269) | TTR | cr018*** + Cas9 mRNA | Lipid A | PEG2k-DMG | 86.94 | 0.049 | 99% |
| LNP262 (TTR271) | TTR | cr021*** + Cas9 mRNA | Lipid A | PEG2k-DMG | 86.48 | 0.078 | 98% |
| LNP263 (TTR272) | TTR | cr009*** + Cas9 mRNA | Lipid A | PEG2k-DMG | 86.81 | 0.047 | 98% |
| LNP264 (TTR273) | TTR | cr010*** + Cas9 mRNA | Lipid A | PEG2k-DMG | 86.86 | 0.032 | 98% |

(continued)

| LNP # | Target | RNA Cargo | CCD Lipid | Stealth Lipid | Avg. Particle Size (nm) | pdi | EE (%) |
|---|---|---|---|---|---|---|---|
| LNP265 (TTR274) | TTR | cr007*** + Cas9 mRNA | Lipid A | PEG2k-DMG | 86.85 | 0.049 | 97% |
| LNP266 (TTR275) | TTR | cr019*** + Cas9 mRNA | Lipid A | PEG2k-DMG | 87.77 | 0.050 | 97% |
| LNP267 (TTR276) | TTR | cr008*** + Cas9 mRNA | Lipid A | PEG2k-DMG | 81.29 | 0.081 | 98% |
| LNP268 (TTR277) | TTR | cr011*** + Cas9 mRNA | Lipid A | PEG2k-DMG | 83.80 | 0.053 | 97% |
| *** = single guide format with 2'-O-methyl modifications and phosphorothioate linkages at and between the three terminal nucleotides at the 5' and 3' ends For the *in vitro* studies, a 7 point semi-log dose response was performed (starting at 100 ng/well). 48 hours post transfection, genomic DNA was harvested and editing percent was measured by NGS. Figure 20 shows the editing percentages for these *in vitro* and *in vivo* experiments, demonstrating that editing efficiency is correlated between primary hepatocytes in culture and *in vivo*. | | | | | | | |

Because NGS provides specific sequencing results in addition to overall editing efficiency, sequence-specific editing patterns were compared to Neuro 2A cells. Figure 21 shows representative data demonstrating that insertion and deletion patterns differ significantly between mouse Neuro2A cells (transfected with Cas9 mRNA and gRNA) and mouse primary hepatocytes (transfected with LNPs containing Cas9 mRNA and gRNA). Mouse primary hepatocytes yielded editing patterns very similar to those observed *in vivo* (transfected with LNPs containing Cas9 mRNA and gRNA) (Figure 22). As shown in Figure 22, 53.2% of the edits measured in mouse primary hepatocytes were deletions (primarily 1 bp deletions) and 16.8% were insertions (primarily 1 bp insertions), for a total of 70% editing. Out of the total of 70% editing, 64.5% of the edits resulted in a frameshift mutation, which represents ~92% of the total edits measured (not shown). Similarly, representative data is shown for the editing percentages and edit types as observed from LNP-based delivery of Cas9 mRNA and gRNA to mouse liver cells *in vivo:* 46.6% of the edits measured in mouse liver cells *in vivo* were deletions (again, primarily 1 bp deletions) and 12.9% were insertions (again, primarily 1 bp insertions), for a total of 59.5% editing. Out of the total of 59.5% editing, 57.4% of the edits resulted in a frameshift mutation, representing ~96% of the edits measured *in vivo* (not shown).

Example 13. Pharmacokinetics of CRISPR/Cas9 components delivered by LNP.

LNP-294, containing Cas9 mRNA and sgRNA targeting mouse TTR, was formulated as described in Example 1. The ratio of mRNA to guideRNA was confirmed by HPLC. Animals were dosed with each LNP at 2 mg/kg as described in Example 1 (n=3 for each group), and taken down at the following time points: 5 min, 15 min, 30 min, 60 min, 2 hr, 4 hr, 6 hr, 12 hr, 24 hr, 72 hr, and 7 days. At necropsy, plasma, liver, and spleen were collected for qPCR analysis of levels of Cas9 mRNA and guideRNA. Figure 23 shows plasma concentrations of these components, Figure 24 shows concentrations in liver, and Figure 25 shows concentrations in spleen. The following pharmacokinetic parameters were calculated for plasma concentrations:

Table 5. Pharmacokinetic parameters

| Parameter | sg009 (sgRNA) | Cas9 (mRNA) |
|---|---|---|
| Dose (mg/kg) | 1 (25mcg/ms) | 1 (25mcg/ms) |
| $C_{max}$ (mcg/mL) | 39.049 | 18.15 |
| $T_{max}$ (hr) | 0.083 | 0.5 |
| $T_{1/2}$ (hr) | 2.32 | 2.54 |
| Vd (mL/kg) | 195.6 | 208.4 |
| Cl (mL/hr*kg) | 58.4 | 56.7 |
| $AUC_{last}$ (mcg*hr/mL) | 21.99 | 18.39 |

Figure 26A shows the relative ratios of the sgRNA to Cas9 mRNA in plasma and tissue.

Cytokine induction in the treated mice was also measured. For this analysis, approximately 50-100 μL of blood was collected by tail vein nick for serum cytokine measurements. Blood was allowed to clot at room temperature for approximately 2 hours, and then centrifuged at 1000xg for 10 minutes before collecting the serum. A Luminex based magnetic bead multiplex assay (Affymetrix ProcartaPlus, catalog number Exp040-00000-801) measuring IL-6, TNF-alpha, IFN-alpha, and MCP-1 was used for cytokine analysis in collected in samples. Kit reagents and standards were prepared as directed in the manufacturer's protocol. Mouse serum was diluted 4-fold using the sample diluent provided and 50 μL was added to wells containing 50 μL of the diluted antibody coated magnetic beads. The plate was incubated for 2 hours at room temperature and then washed. Diluted biotin antibody (50 μL) was added to the beads and incubated for 1 hour at room temperature. The beads were washed again before adding 50 μL of diluted streptavidin-PE to each well, followed by incubation for 30 minutes. The beads were washed once again and then suspended in 100 μL of wash buffer and read on the Bio-Plex 200 instrument (Bio-Rad). The data was analyzed using Bioplex Manager ver. 6.1 analysis package with cytokine concentrations calculated off a standard curve using a five parameter logistic curve fit. Figure 27 shows plasma cytokine levels for the treated mice over time. As shown in Figure 27, each of the cytokines had a measureable increase between 2-4 hours post treatment, and each returned to baseline by 12-24 hours.

Three different guide sequences were separately formulated, according to Example 1, and injected into mice (n=3) to determine the pharmacokinetic profile of Lipid A. Levels of Lipid A in mouse liver and plasma were measured by LC/MS. Figure 26B shows the plasma and liver concentrations of Lipid A over time. $T_{max}$ in liver was achieved within 30 minutes of administration, whereas $T_{1/2}$ in plasma and liver were achieved within approximately 5-6 hours of LNP administration.

<u>Example 14. Duration of Response for *in vivo* editing</u>

Cas9 mRNA and chemically modified sgRNA targeting a mouse TTR sequence were formulated as described in Example 1:

Table 6. Formulation information for LNP 402.

| LNP # | Target | RNA Cargo | CCD Lipid | Stealth Lipid | Avg. Particle Size (nm) | pdi | EE (%) |
|---|---|---|---|---|---|---|---|
| LNP402 | TTR | sg282 + Cas9 mRNA | Lipid A | PEG2k-DMG | 82.3 | 0.171 | 97.43 |

The LNPs were dosed to mice (single dose at 3 mg/kg, 1 mg/kg, or 0.3 mg/kg) as described in Example 1. Cohorts of mice were measured for serum TTR levels at 1, 2, 4, 9, 13, and 16 weeks post-dosing, and liver TTR editing at 1, 2, 9, and 16 weeks post-dosing. To measure liver TTR editing, tissue sample from the liver was collected from the median lobe from each animal of the particular cohort for DNA extraction and analysis. The genomic DNA was extracted from 10 mg of tissue using a bead-based extraction kit, MagMAX-96 DNA Multi-Sample Kit (ThermoFisher, Catalog No. 4413020) according to the manufacturer's protocol, which includes homogenizing tissue in lysis buffer (approximately 400 μL/10 mg tissue) and precipitating the DNA. All DNA samples were normalized to 100 ng/μL concentration for PCR and subsequent NGS analysis.

The sgRNA in this example was chemically synthesized and sourced from a commercial supplier, with 2'-O-methyl modifications and phosphorothioate linkages as represented below (m = 2'-OMe; * = phosphorothioate):
sg282:

mU*mU*mA*CAGCCACGUCUACAGCAGUUUUAGAmGmCmUmAmGmAmAm

AmUmAmGmCAAGUUAAAAUAAGGCUAGUCCGUUAUCAmAmCmUmUmGm

AmAmAmAmAmGmUmGmGmCmAmCmCmGmAmGmUmCmGmGmUmGmCmU*

mU*mU*mU.

Figure 28 shows mouse serum TTR levels over time, and Figure 29A shows corresponding editing percentages as measured by NGS. Figure 29B shows both mouse serum TTR levels over time and the corresponding editing percentages as measured by NGS, through 16 weeks post-dosing.

Example 15. Formulations using mRNA preparations

Cas9 mRNA was prepared as described in Example 1 using both the precipitation-only and HPLC purification protocols LNP was formulated using the HPLC purified mRNA (LNP492), and compared to LNP formulated using the precipitation-only processed mRNA (LNP490, LNP494). The Cas9 mRNA cargo of LNP494 was prepared using a differnent synthesis lot of precipitation-only mRNA.

Table 7. Formulations employed in Example 15.

| LNP # | Target | RNA Cargo | CCD Lipid | Stealth Lipid | Avg. Particle Size (nm) | pdi | EE (%) |
|---|---|---|---|---|---|---|---|
| LNP490 | TTR | sg282 + Cas9 mRNA | Lipid A | PEG2k-DMG | 81.9 | 0.194 | 98.24 |
| LNP492 | TTR | sg282 + Cas9 mRNA | Lipid A | PEG2k-DMG | 85.9 | 0.207 | 96.33 |
| LNP494 | TTR | sg282 + Cas9 mRNA | Lipid A | PEG2k-DMG | 70.2 | 0.153 | 96.48 |

Mice were dosed with 0.5 or 1 mg/kg of each formulation as described in Example 1, LNP Delivery *in vivo.* The sgRNA used in this Example was sg282, as described in Example 14.

Figure 30 shows mouse serum cytokine activity at 4 hours post dosing. Figure 31 shows mouse serum TTR concentration levels, and Figure 32 shows mouse liver TTR editing levels.

Table 8. Figure Labels in Figures 30, 31, and 32.

| Figure Label | LNP | Dose (mg/kg) |
|---|---|---|
| Control | N/A (PBS) | N/A |
| A1 | LNP490 | 1 |
| A2 | | 0.5 |
| B1 | LNP492 | 1 |
| B2 | | 0.5 |
| C1 | LNP494 | 1 |
| C2 | | 0.5 |

Example 16. Frozen formulations

LNPs were formulated with a Lipid A to RNA phosphate (N:P) molar ratio of about 4.5. The lipid nanoparticle components were dissolved in 100% ethanol with the following molar ratios: 45 mol-% (12.7 mM) Lipid A; 44 mol-% (12.4 mM) cholesterol; 9 mol-% (2.53 mM) DSPC; and 2 mol-% (0.563 mM) PEG2k-DMG. The RNA cargo were dissolved in 50 mM acetate buffer, pH 4.5, resulting in a concentration of RNA cargo of approximately 0.45 mg/mL. For this study, sg282 described in Example 14 was used.

Table 9. LNP formulations employed in Example 16.

| LNP # | Target | RNA Cargo | CCD Lipid | Stealth Lipid | Avg. Particle Size (nm) | pdi | EE (%) |
|---|---|---|---|---|---|---|---|
| LNP493 | TTR | sg282 + Cas9 mRNA | Lipid A | PEG2k-DMG | 69.1 | 0.013 | 97.93 |
| LNP496 | PCSK9 | sg396 + Cas9 mRNA | Lipid A | PEG2k-DMG | 78.6 | 0.150 | 94.45 |

The LNPs (LNP493, LNP496) were formed by microfluidic mixing of the lipid and RNA solutions using a Precision Nanosystems NanoAssemblr™ Benchtop Instrument, according to the manufacturer's protocol. A 2:1 ratio of aqueous to

organic solvent was maintained during mixing using differential flow rates. After mixing, the LNPs were collected, diluted in 50 mM Tris buffer, pH 7.5 The formulated LNPs were filtered using a 0.2 μm sterile filter. The resulting filtrate was mixed 1:1 with 10% w/v sucrose 90 mM NaCl prepared in 50 mM Tris buffer at pH 7.5. The final LNP formulation at 5% w/v sucrose, 45 mM NaCl, 50 mM Tris buffer was stored at 4° C and -80° C for 1.5 days until the day of dosing.

The LNPs were administered to mice at 0.5 and 1 mg/kg (frozen formulation was thawed at 25° C one hour prior to administration). Figure 33 shows mouse serum TTR concentration levels, and Figure 34 shows mouse liver TTR editing levels after dosing.

Table 10. Figure Labels in Figures 33 and 34.

| Figure Label | LNP | Dose (mg/kg) |
|---|---|---|
| Control | N/A (PBS) | N/A |
| A1 | LNP493 (4° C storage) | 1 |
| A2 | | 0.5 |
| B1 | LNP493 (-80° C storage) | 1 |
| B2 | | 0.5 |
| C1 | LNP494 (4° C storage) | 1 |
| C2 | | 0.5 |
| D | LNP496 (non-TTR targeting control, targeting mouse PCSK9) | 2 |

The sgRNA in this example was chemically synthesized and sourced from a commercial supplier, with 2'-O-methyl modifications and phosphorothioate linkages as represented below (m = 2'-OMe; * = phosphorothioate): sg396:

mG*mC*mU*GCCAGGAACCUACAUUGGUUUUAGAmGmCmUmAmGmAmAm

AmUmAmGmCAAGUUAAAAUAAGGCUAGUCCGUUAUCAmAmCmUmUmGm

AmAmAmAmAmGmUmGmGmCmAmCmCmGmAmGmUmCmGmGmUmGmCmU*

mU*mU*mU.

Example 17: Alternative LNP formulation processes

LNPs were formulated with a Lipid A to RNA phosphate (N:P) molar ratio of about 4.5. The lipid nanoparticle components were dissolved in 100% ethanol with the following molar ratios: 45 mol-% (12.7 mM) Lipid A; 44 mol-% (12.4 mM) cholesterol; 9 mol-% (2.53 mM) DSPC; and 2 mol-% (0.563 mM) PEG2k-DMG. The RNA cargo were dissolved in either acetate buffer (in a final concentration of 25 mM sodium acetate, pH 4.5), or citrate buffer (in a final concentration of 25mM sodium citrate, 100 mM NaCl, pH 5) resulting in a concentration of RNA cargo of approximately 0.45 mg/mL. For this study, sg282 described in Example 14 was used.

The LNPs were formed either by by microfluidic mixing of the lipid and RNA solutions using a Precision Nanosystems NanoAssemblr™ Benchtop Instrument, per the manufacturer's protocol, or cross-flow mixing. LNP563 and LNP564 were prepared using the NanoAssemblr preparation, where a 2:1 ratio of aqueous to organic solvent was maintained during mixing using differential flow rates, 8 mL/min for aqueous and 4 mL/min for the organic phase. After mixing, the LNPs were collected and 1:1 diluted in 50 mM Tris buffer, pH 7.5. The LNPs were dialyzed in 50 mM Tris, pH 7.5 overnight and the next day filtered using a 0.2 μm sterile filter. The resulting filtrate was concentrated and mixed 1:1 with 10% w/v sucrose 90 mM NaCl prepared in 50 mM Tris buffer at pH 7.5. The final LNP formulation at 5% w/v sucrose, 45 mM NaCl, 50 mM Tris buffer was stored at 4° C and -80° C for 1.5 days until the day of dosing.

Table 11. Formulation information for LNPs used in Example 17.

| LNP # | Target | RNA Cargo | CCD Lipid | Stealth Lipid | Avg. Particle Size (nm) | pdi | EE (%) |
|---|---|---|---|---|---|---|---|
| LNP561 | TTR | sg282 + Cas9 mRNA* | Lipid A | PEG2k-DMG | 111.0 | 0.058 | 94.73 |

(continued)

| LNP # | Target | RNA Cargo | CCD Lipid | Stealth Lipid | Avg. Particle Size (nm) | pdi | EE (%) |
|---|---|---|---|---|---|---|---|
| LNP562 | TTR | sg282 + Cas9 mRNA* | Lipid A | PEG2k-DMG | 106.2 | 0.047 | 93.68 |
| LNP563 | TTR | sg282 + Cas9 mRNA* | Lipid A | PEG2k-DMG | 72.8 | 0.065 | 94.68 |
| LNP564 | TTR | sg282 + Cas9 mRNA* | Lipid A | PEG2k-DMG | 123.0 | 0.105 | 88.03 |
| *Cas9 1xNLS, no HA tag. | | | | | | | |

LNP561 and LNP562 were prepared using the cross-flow technique a syringe pump was used with two syringes of RNA at 0.45 mg/mL, one syringe of organice phase containing lipids and one syringe of water. These were mixed at 40 mL/min with variable tubing lengths, aqueous and organic phases were pushed through a 0.5 mm peek cross and this output was introduced into a 1 mm tee connected to the water tubing. LNPs were incubated at room temperature for one hour and then diluted 1: 1 with water. Briefly, LNPs and water were introduced at 25 mL/min in a 1 mm tee by a syringe pump.

For purification and concentration, tangential flow filtration was used. Generally for this procedure, Vivaflow 50 cartridges from Sartorius are primed with 500 mL water and then LNPs are introduced using Pall Minimate systems at feed rate of 60 mL/min. The permeate line is clamped to maintain a fixed flow rate of around 1.7 mL/min. Once the LNPs are concentrated a 15 times volume of either PBS or 5% sucrose, 45 mM NaCl, 50 mM Tris at pH 7.5 is introduced under vacuum at a feed rate of 80 mL/min. The permeate line is clamped to maintain a flow rate of 1.9 mL/min. Once the diafiltration is complete, LNPs are concentrated and collected in a sterile DNase RNase free collection tube and stored at 4° C for PBS formulations, or 4° C or -80° C for TSS (*i.e.,* Tris, sucrose, and salt) formulations until the day of dosing.

The LNPs were administered to mice at 1.0 and 2 mg/kg (frozen formulation was thawed at 25° C one hour prior to administration). Figure 35 shows mouse serum TTR concentration levels, while Figure 36 shows mouse liver TTR editing levels after dosing with the different formulations.

Table 12. Figure Labels in Figures 35 and 36.

| Figure Label | LNP | Dose (mg/kg) |
|---|---|---|
| Control | N/A (TSS buffer) | N/A |
| A1 | LNP561 | 2 |
| A2 | | 1 |
| B1 | LNP 562 (LNPs stored at 2-8° C) | 2 |
| B2 | | 1 |
| C1 | LNP562 (LNPs stored at -80° C) | 2 |
| C2 | | 1 |
| D1 | LNP563 | 2 |
| D2 | | 1 |
| E1 | LNP564 | 2 |
| E2 | | 1 |

<u>Example 18 Delivery of LNPs to higher species.</u>

Formulations were prepared similar to those described in Example 14. In certain experiments, the sgRNA was modified with the same chemical modifications as in sg282, but with targeting sequences specific to rat TTR sequences. Efficient editing in rat liver was observed. A 2 mg/kg (total cargo) dose and a 5 mg/kg (total cargo) dose were well tolerated in the experiment. Similar formulations containing mRNA encoding GFP were also well-tolerated by non-human primates at doses of 1 mg/kg and 3 mg/kg.

***Sequences***

Sequences described in the above examples are listed as follows (polynucleotide sequences from 5' to 3'):

Cas9 mRNA (Cas9 coding sequence in bold; HA tag in bold underlined; 2xNLS in underlined):

GGGUCCCGCAGUCGGCGUCCAGCGGCUCUGCUUGUUCGUGUGUGUGUCGU
UGCAGGCCUUAUUCGGAUCC**AUGGAUAAGAAGUACUCAAUCGGGCUGG**
**AUAUCGGAACUAAUUCCGUGGGUUGGGCAGUGAUCACGGAUGAAUACA**
**AAGUGCCGUCCAAGAAGUUCAAGGUCCUGGGGAACACCGAUAGACACA**
**GCAUCAAGAAAAAUCUCAUCGGAGCCCUGCUGUUUGACUCCGGCGAAA**
**CCGCAGAAGCGACCCGGCUCAAACGUACCGCGAGGCGACGCUACACCC**
**GGCGGAAGAAUCGCAUCUGCUAUCUGCAAGAGAUCUUUUCGAACGAAA**
**UGGCAAAGGUCGACGACAGCUUCUUCCACCGCCUGGAAGAAUCUUUCC**
**UGGUGGAGGAGGACAAGAAGCAUGAACGGCAUCCUAUCUUUGGAAAC**
**AUCGUCGACGAAGUGGCGUACCACGAAAAGUACCCGACCAUCUACCAU**
**CUGCGGAAGAAGUUGGUUGACUCAACUGACAAGGCCGACCUCAGAUUG**
**AUCUACUUGGCCCUCGCCCAUAUGAUCAAAUUCCGCGGACACUUCCUG**
**AUCGAAGGCGAUCUGAACCCUGAUAACUCCGACGUGGAUAAGCUUUUC**
**AUUCAACUGGUGCAGACCUACAACCAACUGUUCGAAGAAAACCCAAUC**
**AAUGCUAGCGGCGUCGAUGCCAAGGCCAUCCUGUCCGCCCGGCUGUC**
**GAAGUCGCGGCGCCUCGAAAACCUGAUCGCACAGCUGCCGGGAGAGA**
**AAAAGAACGGACUUUUCGGCAACUUGAUCGCUCUCUCACUGGGACUCA**
**CUCCCAAUUUCAAGUCCAAUUUUGACCUGGCCGAGGACGCGAAGCUGC**
**AACUCUCAAAGGACACCUACGACGACGACUUGGACAAUUUGCUGGCAC**

**AAAUUGGCGAUCAGUACGCGGAUCUGUUCCUUGCCGCUAAGAACCUUUCGGACGCAAUCUUGCUGUCCGAUAUCCUGCGCGUGAACACCGAAAUAACCAAAGCGCCGCUUAGCGCCUCGAUGAUUAAGCGGUACGACGAGCAUCACCAGGAUCUCACGCUGCUCAAAGCGCUCGUGAGACAGCAACUGCCUGAAAAGUACAAGGAGAUCUUCUUCGACCAGUCCAAGAAUGGGUACGCAGGGUACAUCGAUGGAGGCGCUAGCCAGGAAGAGUUCUAUAAGUUCAUCAAGCCAAUCCUGGAAAAGAUGGACGGAACCGAAGAACUGCUGGUCAAGCUGAACAGGGAGGAUCUGCUCCGGAAACAGAGAACCUUUGACAACGGAUCCAUUCCCCACCAGAUCCAUCUGGGUGAGCUGCACGCCAUCUUGCGGCGCCAGGAGGACUUUUACCCAUUCCUCAAGGACAACCGGGAAAAGAUCGAGAAAAUUCUGACGUUCCGCAUCCCGUAUUACGUGGGCCCACUGGCGCGCGGCAAUUCGCGCUUCGCGUGGAUGACUAGAAAAUCAGAGGAAACCAUCACUCCUUGGAAUUUCGAGGAAGUUGUGGAUAAGGGAGCUUCGGCACAAAGCUUCAUCGAACGAAUGACCAACUUCGACAAGAAUCUCCCAAACGAGAAGGUGCUUCCUAAGCACAGCCUCCUUUACGAAUACUUCACUGUCUACAACGAACUGACUAAAGUGAAAUACGUUACUGAAGGAAUGAGGAAGCCGGCCUUUCUGUCCGGAGAACAGAAGAAAGCAAUUGUCGAUCUGCUGUUCAAGACCAACCGCAAGGUGACCGUCAAGCAGCUUAAAGAGGACUACUUCAAGAAGAUCGAGUGUUUCGACUCAGUGGAAAUCAGCGGGGUGGAGGACAGAUUCAACGCUUCGCUGGGAACCUAUCAUGAUCUCCUGAAGAUCAUCAAGGACAAGGACUUCCUUGACAACGAGGAGAACGAGGACAUCCUGGAAGAUAUCGUCCUGACCUUGACCCUUUUCGAGGAUCGCGAGAUGAUCGAGGAGAGGCUUAAGACCUACGCUCAUCUCUUCGACGAUAAGGUCAUGAAACAACUCAAGCGCCGCCGGUACACUGGUUGGGGCCGCCUCUCCCGCAAGCUGAUCAACGGUAUUCGCGAUAAACAGAGCGGUAAAACUAUCCUGGAUUUCCUCAAAUCGGAUGGCUUCGCUAAUCGUAACUUCAUGCAAUUGAUCCACGACGACAGCCUGACCUUUAAGGAGGACAUCCAAAAAGCACAAGUGUCCGGACAGGGAGACUCACUCCAUGAACACAUCGCGAAUCUGGCCGGUUCGCCGGCGAUUAAGAAGGGAAUUCUGCAAACUGUGAAGGUGGUCGACGAGCUGGUGAAGGUCAUGGGACGGCACAAACCGGAGAAUAUCGUGAUUGAAAUGGCCCGAGAAAACCAGACUACCCAGAAGGGCCAGAAAAACUCCCGCGAAAGGAUGAAGCGGAUCGAAGAAGGAAUCAAGGAGCUGGGCAGCCAGAUCCUGAAAGAGCACCCGGUGGAAAACACGCAGCUGC**

**AGAACGAGAAGCUCUACCUGUACUAUUUGCAAAAUGGACGGGACAUGU
ACGUGGACCAAGAGCUGGACAUCAAUCGGUUGUCUGAUUACGACGUG
GACCACAUCGUUCCACAGUCCUUUCUGAAGGAUGACUCGAUCGAUAAC
AAGGUGUUGACUCGCAGCGACAAGAACAGAGGGAAGUCAGAUAAUGU
GCCAUCGGAGGAGGUCGUGAAGAAGAUGAAGAAUUACUGGCGGCAGC
UCCUGAAUGCGAAGCUGAUUACCCAGAGAAAGUUUGACAAUCUCACUA
AAGCCGAGCGCGGCGGACUCUCAGAGCUGGAUAAGGCUGGAUUCAUC
AAACGGCAGCUGGUCGAGACUCGGCAGAUUACCAAGCACGUGGCGCA
GAUCUUGGACUCCCGCAUGAACACUAAAUACGACGAGAACGAUAAGCU
CAUCCGGGAAGUGAAGGUGAUUACCCUGAAAAGCAAACUUGUGUCGGA
CUUUCGGAAGGACUUUCAGUUUUACAAAGUGAGAGAAAUCAACAACUA
CCAUCACGCGCAUGACGCAUACCUCAACGCUGUGGUCGGUACCGCCCU
GAUCAAAAAGUACCCUAAACUUGAAUCGGAGUUUGUGUACGGAGACUA
CAAGGUCUACGACGUGAGGAAGAUGAUAGCCAAGUCCGAACAGGAAAU
CGGGAAAGCAACUGCGAAAUACUUCUUUUACUCAAACAUCAUGAACUU
UUUCAAGACUGAAAUUACGCUGGCCAAUGGAGAAAUCAGGAAGAGGCC
ACUGAUCGAAACUAACGGAGAAACGGGCGAAAUCGUGUGGGACAAGG
GCAGGGACUUCGCAACUGUUCGCAAAGUGCUCUCUAUGCCGCAAGUCA
AUAUUGUGAAGAAAACCGAAGUGCAAACCGGCGGAUUUUCAAAGGAAU
CGAUCCUCCCAAAGAGAAAUAGCGACAAGCUCAUUGCACGCAAGAAAG
ACUGGGACCCGAAGAAGUACGGAGGAUUCGAUUCGCCGACUGUCGCA
UACUCCGUCCUCGUGGUGGCCAAGGUGGAGAAGGGAAAGAGCAAAAA
GCUCAAAUCCGUCAAAGAGCUGCUGGGGAUUACCAUCAUGGAACGAUC
CUCGUUCGAGAAGAACCCGAUUGAUUUCCUCGAGGCGAAGGGUUACAA
GGAGGUGAAGAAGGAUCUGAUCAUCAAACUCCCCAAGUACUCACUGUU
CGAACUGGAAAAUGGUCGGAAGCGCAUGCUGGCUUCGGCCGGAGAAC
UCCAAAAAGGAAAUGAGCUGGCCUUGCCUAGCAAGUACGUCAACUUCC
UCUAUCUUGCUUCGCACUACGAAAAACUCAAAGGGUCACCGGAAGAUA
ACGAACAGAAGCAGCUUUUCGUGGAGCAGCACAAGCAUUAUCUGGAUG
AAAUCAUCGAACAAAUCUCCGAGUUUUCAAAGCGCGUGAUCCUCGCCG
ACGCCAACCUCGACAAAGUCCUGUCGGCCUACAAUAAGCAUAGAGAUA
AGCCGAUCAGAGAACAGGCCGAGAACAUUAUCCACUUGUUCACCCUGA
CUAACCUGGGAGCCCCAGCCGCCUUCAAGUACUUCGAUACUACUAUCG**

**AUCGCAAAAGAUACACGUCCACCAAGGAAGUUCUGGACGCGACCCUGAUCCACCAAAGCAUCACUGGACUCUACGAAACUAGGAUCGAUCUGUCGCAGCUGGGUGGCGAUGGCUCGGCUUACCCAUACGACGUGCCUGACUACGCCUCGCUCGGAUCGGGCUCC**CCCAAAAAGAAACGGAAGGUG**GACGGAUCC**CCGAAAAAGAAGAGAAAGGUG**GACUCCGGAUGA**GAAUUAUGCAGUCUAGCCAUCACAUUUAAAAGCAUCUCAGCCUACCAUGAGAAUAAGAGAAAGAAAAUGAAGAUCAAUAGCUUAUUCAUCUCUUUUUCUUUUUCGUUGGUGUAAAGCCAACACCCUGUCUAAAAAACAUAAAUUUCUUUAAUCAUUUUGCCUCUUUUCUCUGUGCUUCAAUUAAUAAAAAAUGGAAAGAACCUCGAGAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAUCUAG

'Cas9 1xNLS, no HA tag' referenced in Table 11 and used in Example 17:

GGGUCCCGCAGUCGGCGUCCAGCGGCUCUGCUUGUUCGUGUGUGUCGUUGCAGGCCUUAUUCGGAUCCAUGGAUAAGAAGUACUCAAUCGGGCUGGAUAUCGGAACUAAUUCCGUGGGUUGGGCAGUGAUCACGGAUGAAUACAAAGUGCCGUCCAAGAAGUUCAAGGUCCUGGGGAACACCGAUAGACACAGCAUCAAGAAAAAUCUCAUCGGAGCCCUGCUGUUUGACUCCGGCGAAACCGCAGAAGCGACCCGGCUCAAACGUACCGCGAGGCGACGCUACACCCGGCGGAAGAAUCGCAUCUGCUAUCUGCAAGAGAUCUUUUCGAACGAAAUGGCAAAGGUCGACGACAGCUUCUUCCACCGCCUGGAAGAAUCUUUCCUGGUGGAGGAGGACAAGAAGCAUGAACGGCAUCCUAUCUUUGGAAACAUCGUCGACGAAGUGGCGUACCACGAAAAGUACCCGACCAUCUACCAUCUGCGGAAGAAGUUGGUUGACUCAACUGACAAGGCCGACCUCAGAUUGAUCUACUUGGCCCUCGCCCAUAUGAUCAAAUUCCGCGGACACUUCCUGAUCGAAGGCGAUCUGAACCCUGAUAACUCCGACGUGGAUAAGCUUUUCAUUCAACUGGUGCAGACCUACAACCAACUGUUCGAAGAAAACCCAAUCAAUGCUAGCGGCGUCGAUGCCAAGGCCAUCCUGUCCGCCCGGCUGUCGAAGUCGCGGCGCCUCGAAAACCUGAUCGCACAGCUGCCGGGAGAGAAAAAGAACGGACUUUUCGGCAACUUGAUCGCUCUCUCACUGGGACUCACUCCCAAUUUCAAGUCCAAUUUUGACCUGGCCGAGGACGCGAAGCUGCAACUCUCAAAGGACACCUACGACGACGACUUGGACAAUUUGCUGGCACAAAUUGGCGAUCAGUACGCGGAUCUGUUCCUUGCC

GCUAAGAACCUUUCGGACGCAAUCUUGCUGUCCGAUAUCCUGCGCGUGAA
CACCGAAAUAACCAAAGCGCCGCUUAGCGCCUCGAUGAUUAAGCGGUACG
ACGAGCAUCACCAGGAUCUCACGCUGCUCAAAGCGCUCGUGAGACAGCAA
CUGCCUGAAAAGUACAAGGAGAUCUUCUUCGACCAGUCCAAGAAUGGGU
ACGCAGGGUACAUCGAUGGAGGCGCUAGCCAGGAAGAGUUCUAUAAGUU
CAUCAAGCCAAUCCUGGAAAAGAUGGACGGAACCGAAGAACUGCUGGUCA
AGCUGAACAGGGAGGAUCUGCUCCGGAAACAGAGAACCUUUGACAACGG
AUCCAUUCCCCACCAGAUCCAUCUGGGUGAGCUGCACGCCAUCUUGCGGC
GCCAGGAGGACUUUUACCCAUUCCUCAAGGACAACCGGGAAAAGAUCGAG
AAAAUUCUGACGUUCCGCAUCCCGUAUUACGUGGGCCCACUGGCGCGCGG
CAAUUCGCGCUUCGCGUGGAUGACUAGAAAAUCAGAGGAAACCAUCACUC
CUUGGAAUUUCGAGGAAGUUGUGGAUAAGGGAGCUUCGGCACAAAGCUU
CAUCGAACGAAUGACCAACUUCGACAAGAAUCUCCCAAACGAGAAGGUGC
UUCCUAAGCACAGCCUCCUUUACGAAUACUUCACUGUCUACAACGAACUG
ACUAAAGUGAAAUACGUUACUGAAGGAAUGAGGAAGCCGGCCUUUCUGU
CCGGAGAACAGAAGAAAGCAAUUGUCGAUCUGCUGUUCAAGACCAACCGC
AAGGUGACCGUCAAGCAGCUUAAAGAGGACUACUUCAAGAAGAUCGAGU
GUUUCGACUCAGUGGAAAUCAGCGGGGUGGAGGACAGAUUCAACGCUUC
GCUGGGAACCUAUCAUGAUCUCCUGAAGAUCAUCAAGGACAAGGACUUCC
UUGACAACGAGGAGAACGAGGACAUCCUGGAAGAUAUCGUCCUGACCUU
GACCCUUUUCGAGGAUCGCGAGAUGAUCGAGGAGAGGCUUAAGACCUAC
GCUCAUCUCUUCGACGAUAAGGUCAUGAAACAACUCAAGCGCCGCCGGUA
CACUGGUUGGGGCCGCCUCUCCCGCAAGCUGAUCAACGGUAUUCGCGAUA
AACAGAGCGGUAAAACUAUCCUGGAUUUCCUCAAAUCGGAUGGCUUCGCU
AAUCGUAACUUCAUGCAAUUGAUCCACGACGACAGCCUGACCUUUAAGGA
GGACAUCCAAAAAGCACAAGUGUCCGGACAGGGAGACUCACUCCAUGAAC
ACAUCGCGAAUCUGGCCGGUUCGCCGGCGAUUAAGAAGGGAAUUCUGCAA
ACUGUGAAGGUGGUCGACGAGCUGGUGAAGGUCAUGGGACGGCACAAAC
CGGAGAAUAUCGUGAUUGAAAUGGCCCGAGAAAACCAGACUACCCAGAA
GGGCCAGAAAAACUCCCGCGAAAGGAUGAAGCGGAUCGAAGAAGGAAUC
AAGGAGCUGGGCAGCCAGAUCCUGAAAGAGCACCCGGUGGAAAACACGCA
GCUGCAGAACGAGAAGCUCUACCUGUACUAUUUGCAAAAUGGACGGGAC
AUGUACGUGGACCAAGAGCUGGACAUCAAUCGGUUGUCUGAUUACGACG

UGGACCACAUCGUUCCACAGUCCUUUCUGAAGGAUGACUCGAUCGAUAAC
AAGGUGUUGACUCGCAGCGACAAGAACAGAGGGAAGUCAGAUAAUGUGC
CAUCGGAGGAGGUCGUGAAGAAGAUGAAGAAUUACUGGCGGCAGCUCCU
GAAUGCGAAGCUGAUUACCCAGAGAAAGUUUGACAAUCUCACUAAAGCC
GAGCGCGGCGGACUCUCAGAGCUGGAUAAGGCUGGAUUCAUCAAACGGCA
GCUGGUCGAGACUCGGCAGAUUACCAAGCACGUGGCGCAGAUCUUGGACU
CCCGCAUGAACACUAAAUACGACGAGAACGAUAAGCUCAUCCGGGAAGUG
AAGGUGAUUACCCUGAAAAGCAAACUUGUGUCGGACUUUCGGAAGGACU
UUCAGUUUUACAAAGUGAGAGAAAUCAACAACUACCAUCACGCGCAUGAC
GCAUACCUCAACGCUGUGGUCGGUACCGCCCUGAUCAAAAAGUACCCUAA
ACUUGAAUCGGAGUUUGUGUACGGAGACUACAAGGUCUACGACGUGAGG
AAGAUGAUAGCCAAGUCCGAACAGGAAAUCGGGAAAGCAACUGCGAAAU
ACUUCUUUUACUCAAACAUCAUGAACUUUUUCAAGACUGAAAUUACGCU
GGCCAAUGGAGAAAUCAGGAAGAGGCCACUGAUCGAAACUAACGGAGAA
ACGGGCGAAAUCGUGUGGGACAAGGGCAGGGACUUCGCAACUGUUCGCA
AAGUGCUCUCUAUGCCGCAAGUCAAUAUUGUGAAGAAAACCGAAGUGCA
AACCGGCGGAUUUUCAAAGGAAUCGAUCCUCCCAAAGAGAAAUAGCGACA
AGCUCAUUGCACGCAAGAAAGACUGGGACCCGAAGAAGUACGGAGGAUU
CGAUUCGCCGACUGUCGCAUACUCCGUCCUCGUGGUGGCCAAGGUGGAGA
AGGGAAAGAGCAAAAAGCUCAAAUCCGUCAAAGAGCUGCUGGGGAUUAC
CAUCAUGGAACGAUCCUCGUUCGAGAAGAACCCGAUUGAUUUCCUCGAGG
CGAAGGGUUACAAGGAGGUGAAGAAGGAUCUGAUCAUCAAACUCCCCAA
GUACUCACUGUUCGAACUGGAAAAUGGUCGGAAGCGCAUGCUGGCUUCG
GCCGGAGAACUCCAAAAAGGAAAUGAGCUGGCCUUGCCUAGCAAGUACGU
CAACUUCCUCUAUCUUGCUUCGCACUACGAAAAACUCAAAGGGUCACCGG
AAGAUAACGAACAGAAGCAGCUUUUCGUGGAGCAGCACAAGCAUUAUCU
GGAUGAAAUCAUCGAACAAAUCUCCGAGUUUUCAAAGCGCGUGAUCCUCG
CCGACGCCAACCUCGACAAAGUCCUGUCGGCCUACAAUAAGCAUAGAGAU
AAGCCGAUCAGAGAACAGGCCGAGAACAUUAUCCACUUGUUCACCCUGAC
UAACCUGGGAGCCCCAGCCGCCUUCAAGUACUUCGAUACUACUAUCGAUC
GCAAAAGAUACACGUCCACCAAGGAAGUUCUGGACGCGACCCUGAUCCAC
CAAAGCAUCACUGGACUCUACGAAACUAGGAUCGAUCUGUCGCAGCUGGG
UGGCGAUGGCGGUGGAUCUCCGAAAAAGAAGAGAAAGGUGUAAUGAGCU

AGCCAUCACAUUUAAAAGCAUCUCAGCCUACCAUGAGAAUAAGAGAAAGAAAAUGAAGAUCAAUAGCUUAUUCAUCUCUUUUUCUUUUUCGUUGGUGUAAAGCCAACACCCUGUCUAAAAAACAUAAAUUUCUUUAAUCAUUUUGCCUCUUUUCUCUGUGCUUCAAUUAAUAAAAAAUGGAAAGAACCUCGAGAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAUCUAG

tr001 (trRNA):

AACAGCAUAGCAAGUUAAAAUAAGGCUAGUCCGUUAUCAACUUGAAAAAGUGGCACCGAGUCGGUGCUUUUUUU

cr002 (crRNA targeting FVII; targeting sequence underlined):
<u>AGGGCUCUUGAAGAUCUCCC</u>GUUUUAGAGCUAUGCUGUUUUG

sg001 (sgRNA targeting FVII; targeting sequence underlined):

<u>AGGGCUCUUGAAGAUCUCCC</u>GUUUUAGAGCUAGAAAUAGCAAGUUAAAAUAAGGCUAGUCCGUUAUCAACUUGAAAAAGUGGCACCGAGUCGGUGCUUUUUUU

cr003 (crRNA targeting TTR; targeting sequence underlined):
<u>CCAGUCCAGCGAGGCAAAGG</u>GUUUUAGAGCUAUGCUGUUUUG

sg006 (sgRNA targeting TTR made by IVT; targeting sequence underlined):

GG<u>CCAGUCCAGCGAGGCAAAGG</u>GUUUUAGAGCUAGAAAUAGCAAGUUAAAAUAAGGCUAGUCCGUUAUCAACUUGAAAAAGUGGCACCGAGUCGGUGCUUUUUUU

sg003 (sgRNA targeting TTR; targeting sequence underlined):

<u>CCAGUCCAGCGAGGCAAAGG</u>GUUUUAGAGCUAGAAAUAGCAAGUUAAAAUAAGGCUAGUCCGUUAUCAACUUGAAAAAGUGGCACCGAGUCGGUGCUUUU

sg007 (sgRNA targeting FVII; targeting sequence underlined):

<u>CUCAGUUUUCAUAACCCAGG</u>GUUUUAGAGCUAGAAAUAGCAAGUUAAAAUAAGGCUAGUCCGUUAUCAACUUGAAAAAGUGGCACCGAGUCGGUGCUUUU

sg002 (sgRNA targeting FVII; targeting sequence underlined):

CAGGGCUCUUGAAGAUCUCCGUUUUAGAGCUAGAAAUAGCAAGUUAAAAUAAGGCUAGUCCGUUAUCAACUUGAAAAAGUGGCACCGAGUCGGUGCUUUU

sg004 (sgRNA targeting TTR; targeting sequence underlined):

CUUUCUACAAGCUUACCCAGGUUUUAGAGCUAGAAAUAGCAAGUUAAAAUAAGGCUAGUCCGUUAUCAACUUGAAAAAGUGGCACCGAGUCGGUGCUUUU

sg005 (sgRNA targeting TTR; targeting sequence underlined):

UUACAGCCACGUCUACAGCAGUUUUAGAGCUAGAAAUAGCAAGUUAAAAUAAGGCUAGUCCGUUAUCAACUUGAAAAAGUGGCACCGAGUCGGUGCUUUU

* = Phosphorothioate linkage

m = 2'OMe

tr002 (trRNA):

A*A*C*AGCAUAGCAAGUUAAAAUAAGGCUAGUCCGUUAUCAACUUGAAAAAGUGGCACCGAGUCGGUGCUUUU*U*U*U

cr004 (crRNA targeting FVII; targeting sequence underlined):
A*G*G*GCUCUUGAAGAUCUCCCGUUUUAGAGCUAUGCUGUU*U*U*G

cr005 (crRNA targeting TTR; targeting sequence underlined):
C*C*A*GUCCAGCGAGGCAAAGGGUUUUAGAGCUAUGCUGUU*U*U*G

sg008 (sgRNA targeting FVII; targeting sequence underlined):

mA*mG*mG*GCUCUUGAAGAUCUCCCGUUUUAGAGCUAGAAAUAGCAAGUUAAAAUAAGGCUAGUCCGUUAUCAACUUGAAAAAGUGGCACCGAGUCGGUGCmU*mU*mU*U

sg009 (sgRNA targeting TTR; targeting sequence underlined):

mC*mC*mA*GUCCAGCGAGGCAAAGGGUUUUAGAGCUAGAAAUAGCAAGUUAAAAUAAGGCUAGUCCGUUAUCAACUUGAAAAAGUGGCACCGAGUCGGUGCmU*mU*mU*U

sg010 (sgRNA targeting FVII; targeting sequence underlined):

mC*mU*mC*AGUUUCAUAACCCAGGGUUUUAGAGCUAGAAAUAGCAAGUUAAAAUAAGGCUAGUCCGUUAUCAACUUGAAAAAGUGGCACCGAGUCGGUGCmU*mU*mU*U

sg002 (sgRNA targeting FVII; targeting sequence underlined):

mC*mA*mG*GGCUCUUGAAGAUCUCCGUUUUAGAGCUAGAAAUAGCAAGUUAAAAUAAGGCUAGUCCGUUAUCAACUUGAAAAAGUGGCACCGAGUCGGUGCmU*mU*mU*U

sg011 (sgRNA targeting TTR; targeting sequence underlined):

mC*mU*mU*UCUACAAGCUUACCCAGGUUUUAGAGCUAGAAAUAGCAAGUUAAAAUAAGGCUAGUCCGUUAUCAACUUGAAAAAGUGGCACCGAGUCGGUGCmU*mU*mU*U

sg012 (sgRNA targeting TTR; targeting sequence underlined):

mU*mU*mA*CAGCCACGUCUACAGCAGUUUUAGAGCUAGAAAUAGCAAGUUAAAAUAAGGCUAGUCCGUUAUCAACUUGAAAAAGUGGCACCGAGUCGGUGCmU*mU*mU*U

ec001 (expression cassette - amplicon for expressing sgRNA targeting TTR; U6 promoter in bold, targeting sequence underlined; construct contains inverted dideoxy T at each 5' end):

GCTGCAAGGCGATTAAGTTGGGTAACGCCAGGGTTTTCCCAGTCACGACGTTGTAAAACGACGGCCAGTGAATTC**GAGGGCCTATTTCCCATGATTCCTTCATATTTGCATATACGATACAAGGCTGTTAGAGAGATAATTGGAATTAATTTGACTGTAAACACAAAGATATTAGTACAAAATACGTGACGTAGAAAGTAATAATTTCTTGGGTAGTTTGCAGTTTTAAAATTATGTTTTAAAATGGACTATCATATGCTTACCGTAACTTGAAAGTATTTCGATTTCTTGGCTTTATATATCTTGTGGAAAGGACGAAACACC**GCTTTCTACAAGCTTACCCAGGTTTTAGAGCTAGAAATAGCAAGTTAAAATAAGGCTAGTCCGTTATCAACTTGAAAAAGTGGCACCGAGTCGGTGCTTTTTTTGGTACCCGGGGATCCTCTAGAGTCGACCTGCAGGCATGCAAGCTTGGCGTAATCATGGTCATAGCTGTTTCCTGTGTGAAATTGTTATCCGCTCACAATTCCACACAACATACGAGCCGGAAGCATAAAGTGTAAAGCCTGGGGTGCCTAATGAGTGAGCTA

Primer pairs for NGS analysis of FVII target site targeted by cr001:

Forward:

CACTCTTTCCCTACACGACGCTCTTCCGATCTGATCCAGTGTGGCTGTTTCCATTC

Reverse:

GGAGTTCAGACGTGTGCTCTTCCGATCTTTACACAAGAGCAGGCACGAGATG

Primer pairs for NGS analysis of FVII target site targeted by cr002 and sg001:

Forward:

CACTCTTTCCCTACACGACGCTCTTCCGATCTAGCACATGAGACCTTCTGTTTCTC

Reverse:
GGAGTTCAGACGTGTGCTCTTCCGATCTGACATAGGTGTGACCCTCACAATC

Primer pairs for NGS analysis of FVII target site targeted by sg002:

CACTCTTTCCCTACACGACGCTCTTCCGATCTAGCACATGAGACCTTCTGTTTCTC

Reverse:
GGAGTTCAGACGTGTGCTCTTCCGATCTGACATAGGTGTGACCCTCACAATC

Primer pairs for NGS analysis of TTR target site targeted by cr003 and sg003:

Forward:

CACTCTTTCCCTACACGACGCTCTTCCGATCTAGTCAATAATCAGAATCAGCAGGT

Reverse:

GGAGTTCAGACGTGTGCTCTTCCGATCTAGAAGGCACTTCTTCTTTATCTAAGGT

Primer pairs for NGS analysis of TTR target site targeted by sg004:

Forward:

CACTCTTTCCCTACACGACGCTCTTCCGATCTTGCTGGAGAATCCAAATGTCCTC

Reverse:

GGAGTTCAGACGTGTGCTCTTCCGATCTGCTAGGAATTAAACCTGTGTCTCTTAC

Primer pairs for NGS analysis of TTR target site targeted by sg005:

Forward:

CACTCTTTCCCTACACGACGCTCTTCCGATCTGTTTTGTTCCAGAGTCTATCACCG

Reverse:

GGAGTTCAGACGTGTGCTCTTCCGATCTACACGAATAAGAGCAAATGGGAAC

Primer pairs for PCR amplification of sg004 expression cassette:

/5InvddT/ = inverted dideoxyT
Forward:
/5InvddT/GCTGCAAGGCGATTAAGTTG
Reverse:
/5InvddT/TAGCTCACTCATTAGGCACC

**Table 13. Mouse TTR Guide Sequences.**

| Guide Name | Locations | Guide |
|---|---|---|
| cr006 | Chr18:20666429-20666451 | UCUUGUCUCCUCUGUGCCCA |
| cr007 | Chr18:20666435-20666457 | CUCCUCUGUGCCCAGGGUGC |
| cr008 | Chr18:20666458-20666480 | AGAAUCCAAAUGUCCUCUGA |
| cr009 | Chr18:20666533-20666555 | AGUGUUCAAAAAGACCUCUG |
| cr010 | Chr18:20666541-20666563 | AAAAGACCUCUGAGGGAUCC |
| cr011 | Chr18:20666558-20666580 | UCCUGGGAGCCCUUUGCCUC |
| cr012 | Chr18:20666500-20666522 | CGUCUACAGCAGGGCUGCCU |
| cr013 | Chr18:20666559-20666581 | CCCAGAGGCAAAGGGCUCCC |
| cr014 | Chr18:20670008-20670030 | UUCUACAAACUUCUCAUCUG |
| cr015 | Chr18:20670086-20670108 | AUCCGCGAAUUCAUGGAACG |
| cr016 | Chr18:20673606-20673628 | UGUCUCUCCUCUCUCCUAGG |
| cr017 | Chr18:20673628-20673650 | GUUUUCACAGCCAACGACUC |
| cr018 | Chr18:20673684-20673706 | CCCAUACUCCUACAGCACCA |
| cr019 | Chr18:20673657-20673679 | GCAGGGCUGCGAUGGUGUAG |
| cr020 | Chr18:20673675-20673697 | UGUAGGAGUAUGGGCUGAGC |
| cr021 | Chr18:20673685-20673707 | GCCGUGGUGCUGUAGGAGUA |
| cr022 | Chr18:20673723-20673745 | UGGGCUGAGUCUCUCAAUUC |
| cr023 | Chr18:20665448-20665470 | CUCUUCCUCCUUUGCCUCGC |
| cr024 | Chr18:20665472-20665494 | CUGGUAUUUGUGUCUGAAGC |
| cr025 | Chr18:20665453-20665475 | CCUCCUUUGCCUCGCUGGAC |
| cr026 | Chr18:20665496-20665518 | CUCACAGGAUCACUCACCGC |
| cr027 | Chr18:20665414-20665436 | UCCACAAGCUCCUGACAGGA |
| cr028 | Chr18:20665441-20665463 | GGCAAAGGAGGAAGAGUCGA |
| cr003 | Chr18:20665453-20665475 | CCAGUCCAGCGAGGCAAAGG |

(continued)

| Guide Name | Locations | Guide |
|---|---|---|
| cr029 | Chr18:20665456-20665478 | AUACCAGUCCAGCGAGGCAA |
| cr030 | Chr18:20665497-20665519 | GCUCACAGGAUCACUCACCG |
| cr031 | Chr18:20665462-20665484 | ACACAAAUACCAGUCCAGCG |
| cr032 | Chr18:20665495-20665517 | UCACAGGAUCACUCACCGCG |
| cr033 | Chr18:20665440-20665462 | GCAAAGGAGGAAGAGUCGAA |
| cr034 | Chr18:20666463-20666485 | UUUGACCAUCAGAGGACAUU |
| cr035 | Chr18:20666488-20666510 | GGCUGCCUCGGACAGCAUCC |
| cr036 | Chr18:20666470-20666492 | UCCUCUGAUGGUCAAAGUCC |
| cr037 | Chr18:20666542-20666564 | AAAGACCUCUGAGGGAUCCU |
| cr03 8 | Chr18:20666510-20666532 | UUUACAGCCACGUCUACAGC |
| cr039 | Chr18:20666534-20666556 | GUGUUCAAAAAGACCUCUGA |
| cr040 | Chr18:20666567-20666589 | CAAGCUUACCCAGAGGCAAA |
| cr041 | Chr18:20666503-20666525 | AGGCAGCCCUGCUGUAGACG |
| cr042 | Chr18:20666471-20666493 | UCCAGGACUUUGACCAUCAG |
| cr043 | Chr18:20666547-20666569 | GGGCUCCCAGGAUCCCUCAG |
| cr044 | Chr18:20666483-20666505 | AAAGUCCUGGAUGCUGUCCG |
| cr045 | Chr18:20666568-20666590 | ACAAGCUUACCCAGAGGCAA |
| cr046 | Chr18:20666574-20666596 | CUUUCUACAAGCUUACCCAG |
| cr047 | Chr18:20666509-20666531 | UUACAGCCACGUCUACAGCA |
| cr048 | Chr18:20669968-20669990 | UCCAGGAAGACCGCGGAGUC |
| cr049 | Chr18:20670093-20670115 | CACUUACAUCCGCGAAUUCA |
| cr050 | Chr18:20670056-20670078 | AAGUGUCUUCCAGUACGAUU |
| cr051 | Chr18:20670087-20670109 | CAUCCGCGAAUUCAUGGAAC |
| cr052 | Chr18:20670058-20670080 | AAAUCGUACUGGAAGACACU |
| cr053 | Chr18:20669981-20670003 | CGGAGUCUGGAGAGCUGCAC |
| cr054 | Chr18:20670030-20670052 | AGGAGUGUACAGAGUAGAAC |
| cr055 | Chr18:20670084-20670106 | UUCCCCGUUCCAUGAAUUCG |
| cr056 | Chr18:20670010-20670032 | ACAGAUGAGAAGUUUGUAGA |
| cr057 | Chr18:20670047-20670069 | AACUGGACACCAAAUCGUAC |
| cr058 | Chr18:20670088-20670110 | ACAUCCGCGAAUUCAUGGAA |
| cr059 | Chr18:20669980-20670002 | GCGGAGUCUGGAGAGCUGCA |
| cr060 | Chr18:20669978-20670000 | GUGCAGCUCUCCAGACUCCG |
| cr061 | Chr18:20669961-20669983 | UGUGCCCUCCAGGAAGACCG |
| cr062 | Chr18:20673723-20673745 | UGGGCUGAGUCUCUCAAUUC |
| cr063 | Chr18:20673675-20673697 | UGUAGGAGUAUGGGCUGAGC |
| cr064 | Chr18:20673665-20673687 | UGGGCUGAGCAGGGCUGCGA |
| cr065 | Chr18:20673638-20673660 | GUGGCGAUGGCCAGAGUCGU |
| cr066 | Chr18:20673651-20673673 | CUGCGAUGGUGUAGUGGCGA |
| cr067 | Chr18:20673685-20673707 | GCCGUGGUGCUGUAGGAGUA |

**Table 14. Human TTR Guide Sequences.**

| Guide Name | Guide | Locations | Exon | Strand |
|---|---|---|---|---|
| cr700 | CUGCUCCUCCUCUGCCUUGC | Chr18:31591918-31591940 | 1 | + |
| cr701 | CCUCCUCUGCCUUGCUGGAC | Chr18:31591923-31591945 | 1 | + |
| cr702 | CCAGUCCAGCAAGGCAGAGG | Chr18:31591923-31591945 | 1 | - |
| cr703 | AUACCAGUCCAGCAAGGCAG | Chr18:31591926-31591948 | 1 | - |
| cr704 | ACACAAAUACCAGUCCAGCA | Chr18:31591932-31591954 | 1 | - |
| cr705 | UGGACUGGUAUUUGUGUCUG | Chr18:31591938-31591960 | 1 | + |
| cr706 | CUGGUAUUUGUGUCUGAGGC | Chr18:31591942-31591964 | 1 | + |
| cr707 | CUUCUCUACACCCAGGGCAC | Chr18:31592881-31592903 | 2 | + |
| cr708 | CAGAGGACACUUGGAUUCAC | Chr18:31592900-31592922 | 2 | - |
| cr709 | UUUGACCAUCAGAGGACACU | Chr18:31592909-31592931 | 2 | - |
| cr710 | UCUAGAACUUUGACCAUCAG | Chr18:31592917-31592939 | 2 | - |
| cr711 | AAAGUUCUAGAUGCUGUCCG | Chr18:31592929-31592951 | 2 | + |
| cr712 | CAUUGAUGGCAGGACUGCCU | Chr18:31592946-31592968 | 2 | - |
| cr713 | AGGCAGUCCUGCCAUCAAUG | Chr18:31592949-31592971 | 2 | + |
| cr714 | UGCACGGCCACAUUGAUGGC | Chr18:31592956-31592978 | 2 | - |
| cr715 | CACAUGCACGGCCACAUUGA | Chr18:31592960-31592982 | 2 | - |
| cr716 | AGCCUUUCUGAACACAUGCA | Chr18:31592972-31592994 | 2 | - |
| cr717 | GAAAGGCUGCUGAUGACACC | Chr18:31592987-31593009 | 2 | + |
| cr718 | AAAGGCUGCUGAUGACACCU | Chr18:31592988-31593010 | 2 | + |
| cr719 | ACCUGGGAGCCAUUUGCCUC | Chr18:31593004-31593026 | 2 | + |
| cr720 | CCCAGAGGCAAAUGGCUCCC | Chr18:31593005-31593027 | 2 | - |
| cr721 | GCAACUUACCCAGAGGCAAA | Chr18:31593013-31593035 | 2 | - |
| cr722 | UUCUUUGGCAACUUACCCAG | Chr18:31593020-31593042 | 2 | - |
| cr723 | AUGCAGCUCUCCAGACUCAC | Chr18:31595125-31595147 | 3 | - |
| cr724 | AGUGAGUCUGGAGAGCUGCA | Chr18:31595127-31595149 | 3 | + |
| cr725 | GUGAGUCUGGAGAGCUGCAU | Chr18:31595128-31595150 | 3 | + |
| cr726 | GCUGCAUGGGCUCACAACUG | Chr18:31595141-31595163 | 3 | + |
| cr727 | GCAUGGGCUCACAACUGAGG | Chr18:31595144-31595166 | 3 | + |
| cr728 | ACUGAGGAGGAAUUUGUAGA | Chr18:31595157-31595179 | 3 | + |
| cr729 | CUGAGGAGGAAUUUGUAGAA | Chr18:31595158-31595180 | 3 | + |
| cr730 | UGUAGAAGGGAUAUACAAAG | Chr18:31595171-31595193 | 3 | + |
| cr731 | AAAUAGACACCAAAUCUUAC | Chr18:31595194-31595216 | 3 | + |
| cr732 | AGACACCAAAUCUUACUGGA | Chr18:31595198-31595220 | 3 | + |
| cr733 | AAGUGCCUUCCAGUAAGAUU | Chr18:31595203-31595225 | 3 | - |
| cr734 | CUCUGCAUGCUCAUGGAAUG | Chr18:31595233-31595255 | 3 | - |
| cr735 | CCUCUGCAUGCUCAUGGAAU | Chr18:31595234-31595256 | 3 | - |
| cr736 | ACCUCUGCAUGCUCAUGGAA | Chr18:31595235-31595257 | 3 | - |
| cr737 | UACUCACCUCUGCAUGCUCA | Chr18:31595240-31595262 | 3 | - |
| cr738 | GUAUUCACAGCCAACGACUC | Chr18:31598571-31598593 | 4 | + |

(continued)

| Guide Name | Guide | Locations | Exon | Strand |
|---|---|---|---|---|
| cr739 | GCGGCGGGGGCCGGAGUCGU | Chr18:31598581-31598603 | 4 | - |
| cr740 | AAUGGUGUAGCGGCGGGGGC | Chr18:31598590-31598612 | 4 | - |
| cr741 | CGGCAAUGGUGUAGCGGCGG | Chr18:31598594-31598616 | 4 | - |
| cr742 | GCGGCAAUGGUGUAGCGGCG | Chr18:31598595-31598617 | 4 | - |
| cr743 | GGCGGCAAUGGUGUAGCGGC | Chr18:31598596-31598618 | 4 | - |
| cr744 | GGGCGGCAAUGGUGUAGCGG | Chr18:31598597-31598619 | 4 | - |
| cr745 | GCAGGGCGGCAAUGGUGUAG | Chr18:31598600-31598622 | 4 | - |
| cr746 | GGGGCUCAGCAGGGCGGCAA | Chr18:31598608-31598630 | 4 | - |
| cr747 | GGAGUAGGGGCUCAGCAGGG | Chr18:31598614-31598636 | 4 | - |
| cr748 | AUAGGAGUAGGGGCUCAGCA | Chr18:31598617-31598639 | 4 | - |
| cr749 | AAUAGGAGUAGGGGCUCAGC | Chr18:31598618-31598640 | 4 | - |
| cr750 | CCCCUACUCCUAUUCCACCA | Chr18:31598627-31598649 | 4 | + |
| cr751 | CCGUGGUGGAAUAGGAGUAG | Chr18:31598627-31598649 | 4 | - |
| cr752 | GCCGUGGUGGAAUAGGAGUA | Chr18:31598628-31598650 | 4 | - |
| cr753 | GACGACAGCCGUGGUGGAAU | Chr18:31598635-31598657 | 4 | - |
| cr754 | AUUGGUGACGACAGCCGUGG | Chr18:31598641-31598663 | 4 | - |
| cr755 | GGGAUUGGUGACGACAGCCG | Chr18:31598644-31598666 | 4 | - |
| cr756 | GGCUGUCGUCACCAAUCCCA | Chr18:31598648-31598670 | 4 | + |
| cr757 | AGUCCCUCAUUCCUUGGGAU | Chr18:31598659-31598681 | 4 | - |

## Claims

**1.** A lipid nanoparticle (LNP) composition comprising:

an mRNA encoding a Cas nuclease;
a guide RNA nucleic acid; and
a plurality of component lipids comprising:

a helper lipid, wherein the helper lipid is selected from a steroid, a sterol, and an alkyl resorcinol;
a neutral lipid, wherein the neutral lipid is a neutral, uncharged, or zwitterionic lipid;
a stealth lipid, wherein the stealth lipid has a hydrophilic head group linked to a lipid moiety; and
an ionizable lipid selected from:

Lipid A: (9Z,12Z)-3-((4,4-bis(octyloxy)butanoyl)oxy)-2-((((3-(diethylamino)propoxy)carbonyl)oxy)methyl)propyl-octadeca-9, 12-dienoate
Lipid B: ((5-((dimethylamino)methyl)-1,3-phenylene)bis(oxy))bis(octane-8,1-diyl)bis(decanoate),
Lipid C: 2-((4-(((3-(dimethylamino)propoxy)carbonyl)oxy)hexadecanoyl)oxy)pro pane-1,3-diyl (9Z,9'Z,12Z,12'Z)-bis(octadeca-9,12-dienoate), and
Lipid D: 3-(((3-(dimethylamino)propoxy)carbonyl)oxy)-13-(octanoyloxy)tridecyl-3-octylundecanoate;

wherein the component lipids comprise from 30 mol-% to 60 mol-% of the ionizable lipid, from 30 mol-% to 60 mol-% of the helper lipid, from 1 mol-% to 20 mol-% of the neutral lipid, and from 1 mol-% to 10 mol-% of the stealth lipid.

**2.** An LNP composition according to claim 1, wherein the mRNA encoding a Cas nuclease is a Class 2 Cas nuclease mRNA; and the guide RNA nucleic acid is or encodes an sgRNA.

3. An LNP composition according to any one of the preceding claims, wherein the guide RNA nucleic acid is an sgRNA.

4. An LNP composition according to any one of the preceding claims, wherein the helper lipid is selected from cholesterol, 5-heptadecylresorcinol, and cholesterol hemisuccinate.

5. An LNP composition according to any one of the preceding claims, wherein the helper lipid is cholesterol.

6. An LNP composition according to any one of the preceding claims, wherein the neutral lipid is selected from DSPC and DMPE.

7. An LNP composition according to any one of the preceding claims, wherein the neutral lipid is DSPC.

8. An LNP composition according to any one of the preceding claims, wherein the stealth lipid is selected from PEG2k-DMG and PEG2k-C11.

9. An LNP composition according to any one of the preceding claims, wherein the stealth lipid is PEG2k-DMG.

10. An LNP composition according to any one of the preceding claims, wherein the ionizable lipid is lipid A.

11. An LNP composition according to any one of claims 1 to 3, wherein at least one LNP composition comprises Lipid B, cholesterol, DSPC, and PEG2k-DMG.

12. An LNP composition according to any one of claims 1 to 3, wherein at least one LNP composition comprises Lipid A, cholesterol, DSPC, and PEG2k-DMG.

13. An LNP composition according to any one of the preceding claims, wherein the lipid particles of the LNP composition have an average size of 75 nm to 150 nm.

14. An LNP composition according to any one of the preceding claims, wherein the mRNA and the guide RNA nucleic acid are co-encapsulated in the LNP composition.

15. An LNP composition according to any preceding claim for use in genetically editing a liver cell.

16. An LNP composition for use according to claim 15, wherein the liver cell is a hepatocyte.

17. An LNP composition for use according to claim 15, wherein the liver cell is a stem cell.

## Patentansprüche

1. Lipidnanopartikel(LNP-)Zusammensetzung, umfassend:

eine mRNA, die eine Cas-Nuklease kodiert;
eine Führungs-RNA-Nukleinsäure; und
eine Vielzahl von Komponentenlipiden, umfassend:

ein Helferlipid, wobei das Helferlipid ausgewählt ist aus einem Steroid, einem Sterol und einem Alkylresorcin;
ein neutrales Lipid, wobei das neutrale Lipid ein neutrales, ungeladenes oder zwitterionisches Lipid ist;
ein Stealth-Lipid, wobei das Stealth-Lipid eine hydrophile Kopfgruppe aufweist, die mit einer Lipideinheit verbunden ist; und
ein ionisierbares Lipid ausgewählt aus:

Lipid A: (9Z,12Z)-3-((4,4-bis(Octyloxy)butanoyl)oxy)-2-((((3-(diethylamino)propoxy)carbonyl)oxy)methyl)propyl-octadeca-9,12-dienoat,
Lipid B: ((5-((Dimethylamino)methyl)-1,3-phenylen)bis(oxy))bis(octan-8,1-diyl)bis(decanoat),
Lipid C: 2-((4-(((3-(Dimethylamino)propoxy)carbonyl)oxy)hexadecanoyl)oxy)propan-1,3-diyl(9Z,9'Z,12Z,12'Z)-bis(octadeca-9,12-dienoat), und
Lipid D: 3-(((3-(Dimethylamino)propoxy)carbonyl)oxy)-13-(octanoyloxy)tridecyl-3-octylundecanoat;

wobei die Komponentenlipide 30 Mol-% bis 60 Mol-% des ionisierbaren Lipids, 30 Mol-% bis 60 Mol-% des Helferlipids, 1 Mol-% bis 20 Mol-% des neutralen Lipids und 1 Mol-% bis 10 Mol-% des Stealth-Lipids umfassen.

**2.** LNP-Zusammensetzung nach Anspruch 1, wobei die mRNA, die eine Cas-Nuklease kodiert, eine Klasse-2-Cas-Nuklease-mRNA ist; und die Führungs-RNA-Nukleinsäure eine sgRNA ist oder kodiert.

**3.** LNP-Zusammensetzung nach einem der vorhergehenden Ansprüche, wobei die Führungs-RNA-Nukleinsäure eine sgRNA ist.

**4.** LNP-Zusammensetzung nach einem der vorhergehenden Ansprüche, wobei das Helferlipid ausgewählt ist aus Cholesterin, 5-Heptadecylresorcin und Cholesterinhemisuccinat.

**5.** LNP-Zusammensetzung nach einem der vorhergehenden Ansprüche, wobei das Helferlipid Cholesterin ist.

**6.** LNP-Zusammensetzung nach einem der vorhergehenden Ansprüche, wobei das neutrale Lipid ausgewählt ist aus DSPC und DMPE.

**7.** LNP-Zusammensetzung nach einem der vorhergehenden Ansprüche, wobei das neutrale Lipid DSPC ist.

**8.** LNP-Zusammensetzung nach einem der vorhergehenden Ansprüche, wobei das Stealth-Lipid ausgewählt ist aus PEG2k-DMG und PEG2k-C11.

**9.** LNP-Zusammensetzung nach einem der vorhergehenden Ansprüche, wobei das Stealth-Lipid PEG2k-DMG ist.

**10.** LNP-Zusammensetzung nach einem der vorhergehenden Ansprüche, wobei das ionisierbare Lipid Lipid A ist.

**11.** LNP-Zusammensetzung nach einem der Ansprüche 1 bis 3, wobei zumindest eine LNP-Zusammensetzung Lipid B, Cholesterin, DSPC und PEG2k-DMG umfasst.

**12.** LNP-Zusammensetzung nach einem der Ansprüche 1 bis 3, wobei zumindest eine LNP-Zusammensetzung Lipid A, Cholesterin, DSPC und PEG2k-DMG umfasst.

**13.** LNP-Zusammensetzung nach einem der vorhergehenden Ansprüche, wobei die Lipidpartikel der LNP-Zusammensetzung eine durchschnittliche Größe von 75 nm bis 150 nm aufweisen.

**14.** LNP-Zusammensetzung nach einem der vorhergehenden Ansprüche, wobei die mRNA und die Führungs-RNA-Nukleinsäure co-verkapselt in der LNP-Zusammensetzung sind.

**15.** LNP-Zusammensetzung nach einem vorhergehenden Anspruch zur Verwendung beim genetischen Editieren einer Leberzelle.

**16.** LNP-Zusammensetzung zur Verwendung nach Anspruch 15, wobei die Leberzelle ein Hepatozyt ist.

**17.** LNP-Zusammensetzung zur Verwendung nach Anspruch 15, wobei die Leberzelle eine Stammzelle ist.

## Revendications

**1.** Composition de nanoparticules lipidiques (LNP) comprenant :

un ARNm codant pour une nucléase Cas ;
un acide nucléique ARN guide ; et
une pluralité de lipides constitutifs comprenant :

un lipide auxiliaire, ledit lipide auxiliaire étant choisi parmi un stéroïde, un stérol et un alkylrésorcinol ;
un lipide neutre, ledit lipide neutre étant un lipide neutre, non chargé ou zwitterionique ;
un lipide indétectable, ledit lipide indétectable comportant un groupe de tête hydrophile lié à une fraction

lipidique ; et
un lipide ionisable choisi parmi :

un lipide A : (9Z,12Z)-3-((4,4-bis(octyloxy)butanoyl)oxy)-2-((((3-(diéthylamino)propoxy)carbonyl)oxy)méthyl)propyl-octadéca-9,12-diénoate
un lipide B : ((5-((diméthylamino)méthyl)-1,3-phénylène)bis(oxy))bis(octane-8,1-diyl)bis(décanoate),
un lipide C : (9Z,9'Z,12Z,12'Z)-bis(octadéca-9,12-diénoate) de 2-((4-(((3-(diméthylamino)propoxy)carbonyl)oxy)hexadécanoyl)oxy)propane-1,3-diyle, et
un lipide D : 3-(((3-(diméthylamino)propoxy)carbonyl)oxy)-13-(octanoyloxy)tridécyl-3-octylundécanoate ;
dans laquelle les lipides constitutifs comprennent de 30 % en moles à 60 % en moles du lipide ionisable, de 30 % en moles à 60 % en moles du lipide auxiliaire, de 1 % en moles à 20 % en moles du lipide neutre, et de 1 % en moles à 10 % en moles du lipide indétectable.

2. Composition de LNP selon la revendication 1, dans laquelle l'ARNm codant pour une nucléase Cas est un ARNm de nucléase Cas de classe 2 ; et l'acide nucléique ARN guide est ou code pour un ARNsg.

3. Composition de LNP selon l'une quelconque des revendications précédentes, dans laquelle l'acide nucléique ARN guide est un ARNsg.

4. Composition de LNP selon l'une quelconque des revendications précédentes, dans laquelle le lipide auxiliaire est choisi parmi le cholestérol, le 5-heptadécylrésorcinol et l'hémisuccinate de cholestérol.

5. Composition de LNP selon l'une quelconque des revendications précédentes, dans laquelle le lipide auxiliaire est le cholestérol.

6. Composition de LNP selon l'une quelconque des revendications précédentes, dans laquelle le lipide neutre est choisi parmi DSPC et DMPE.

7. Composition de LNP selon l'une quelconque des revendications précédentes, dans laquelle le lipide neutre est DSCP.

8. Composition de LNP selon l'une quelconque des revendications précédentes, dans laquelle le lipide indétectable est choisi parmi PEG2k-DMG et PEG2k-C11.

9. Composition de LNP selon l'une quelconque des revendications précédentes, dans laquelle le lipide indétectable est PEG2k-DMG.

10. Composition de LNP selon l'une quelconque des revendications précédentes, dans laquelle le lipide ionisable est le lipide A.

11. Composition de LNP selon l'une quelconque des revendications 1 à 3, dans laquelle au moins une composition de LNP comprend le lipide B, le cholestérol, DSPC et PEG2k-DMG.

12. Composition de LNP selon l'une quelconque des revendications 1 à 3, dans laquelle au moins une composition de LNP comprend le lipide A, le cholestérol, DSPC et PEG2k-DMG.

13. Composition de LNP selon l'une quelconque des revendications précédentes, dans laquelle les particules lipidiques de la composition de LNP comportent une taille moyenne de 75 nm à 150 nm.

14. Composition de LNP selon l'une quelconque des revendications précédentes, dans laquelle l'ARNm et l'acide nucléique ARN guide sont co-encapsulés dans la composition de LNP.

15. Composition de LNP selon l'une quelconque des revendications précédentes, destinée à être utilisée dans l'édition génétique d'une cellule hépatique.

16. Composition de LNP destinée à être utilisée selon la revendication 15, dans laquelle la cellule hépatique est un hépatocyte.

**17.** Composition de LNP destinée à être utilisée selon la revendication 15, dans laquelle la cellule hépatique est une cellule souche.

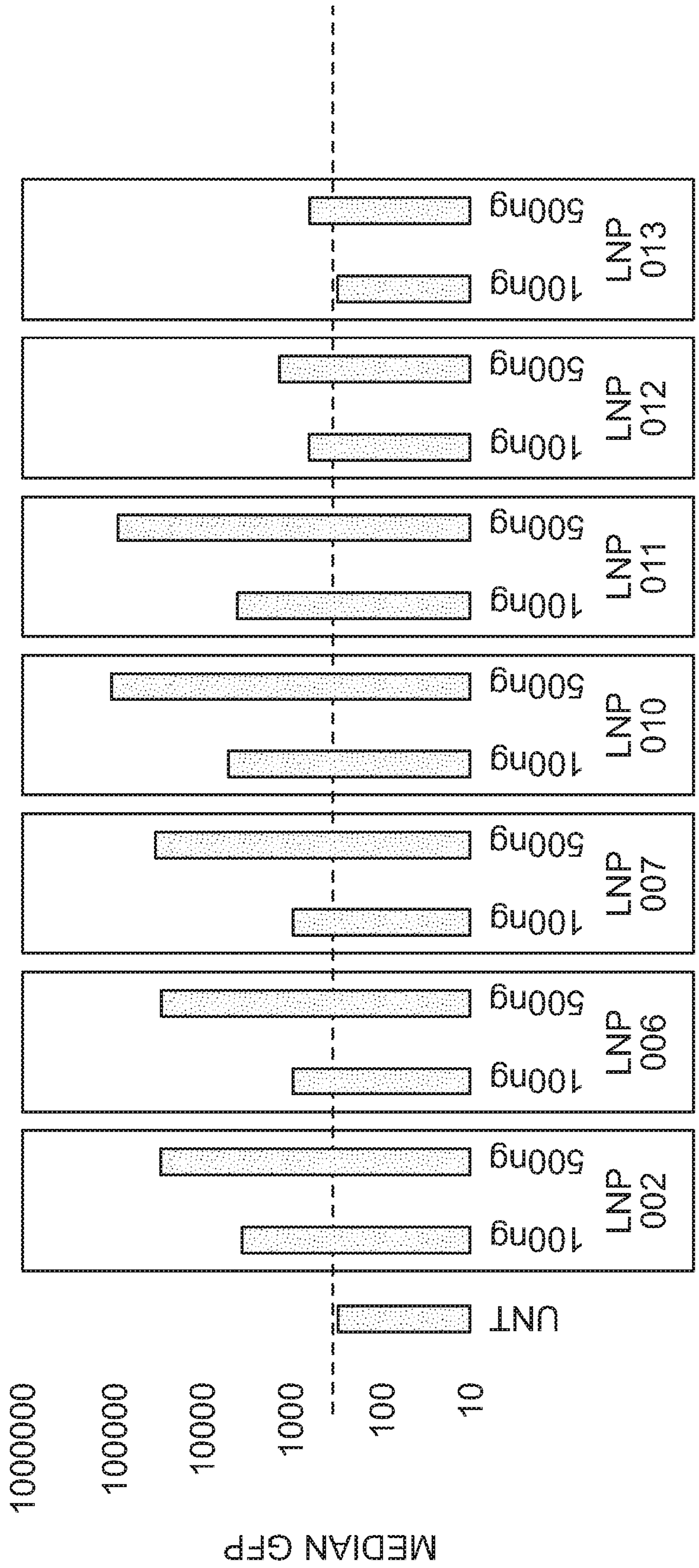
MEDIAN GFP
1000000
100000
10000
1000
100
10
UNT
100ng
500ng
LNP 002
100ng
500ng
LNP 006
100ng
500ng
LNP 007
100ng
500ng
LNP 010
100ng
500ng
LNP 011
100ng
500ng
LNP 012
100ng
500ng
LNP 013

FIG. 1

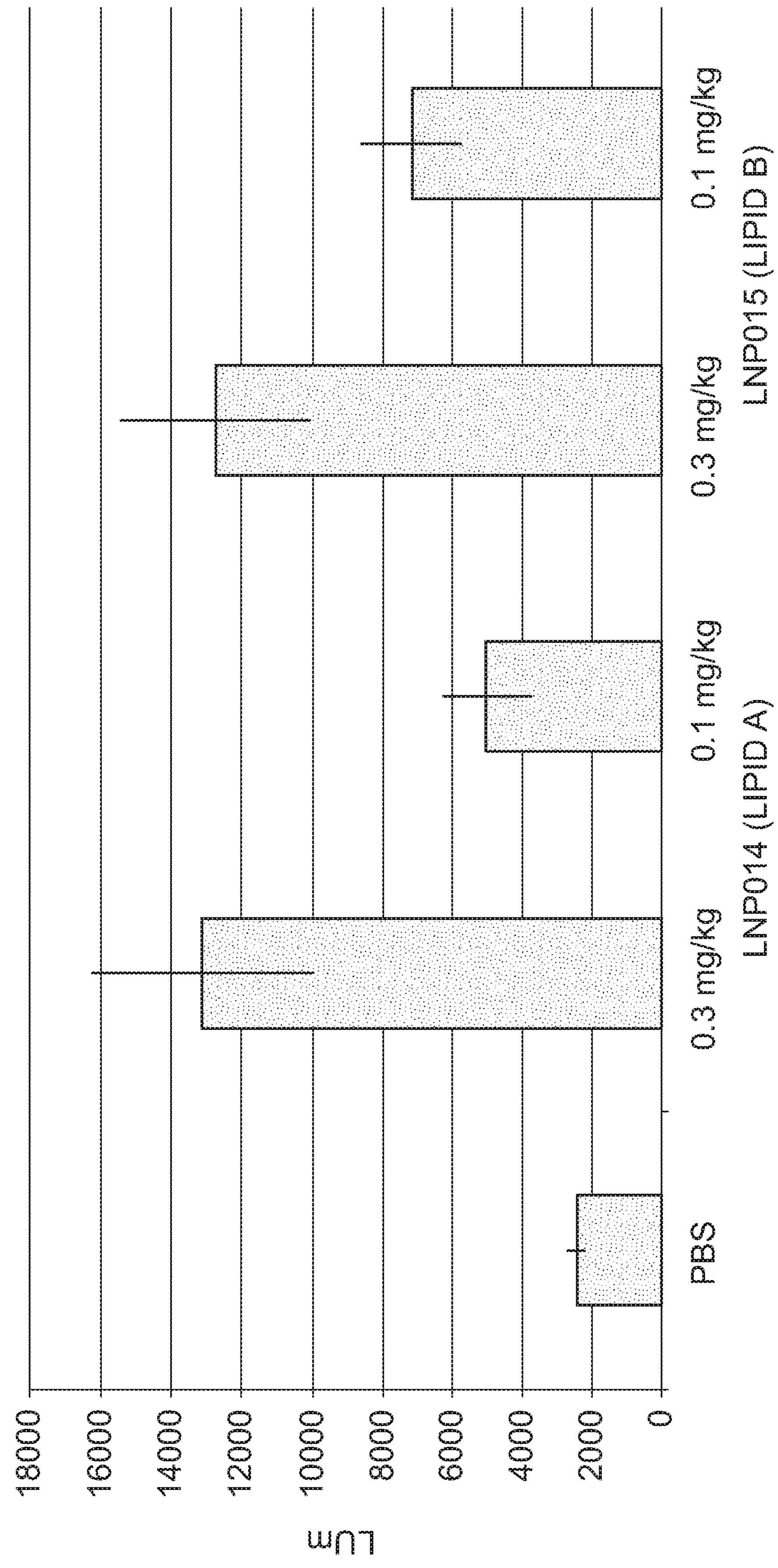
LUm
18000
16000
14000
12000
10000
8000
6000
4000
2000
0
PBS
0.3 mg/kg
0.1 mg/kg
LNP014 (LIPID A)
0.3 mg/kg
0.1 mg/kg
LNP015 (LIPID B)

***FIG. 2***

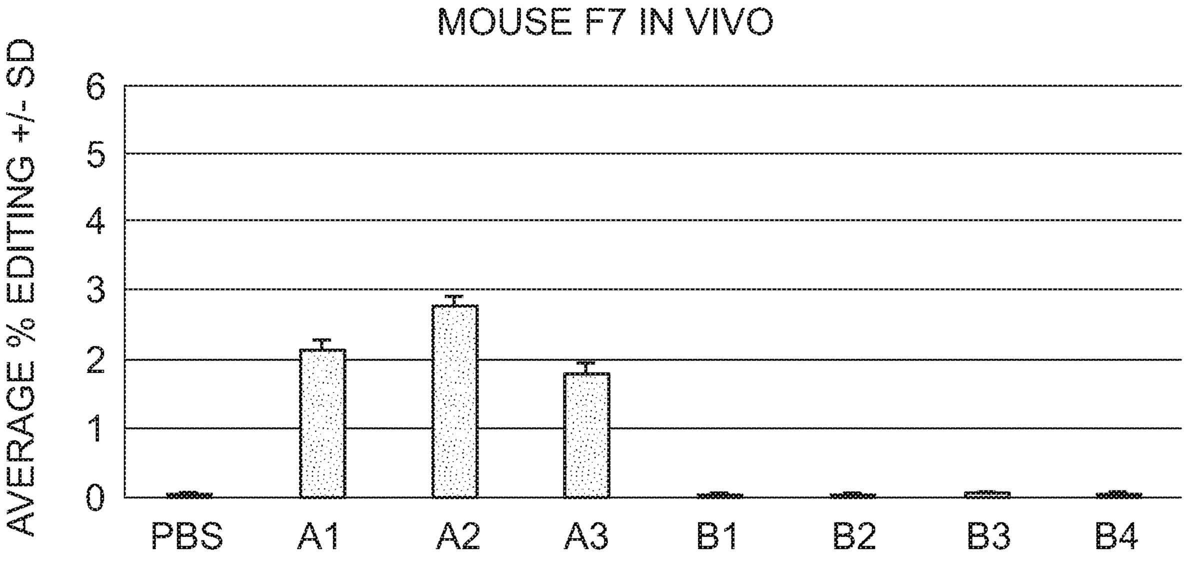
MOUSE F7 IN VIVO
AVERAGE % EDITING +/- SD
6
5
4
3
2
1
0
PBS
A1
A2
A3
B1
B2
B3
B4

FIG. 3A

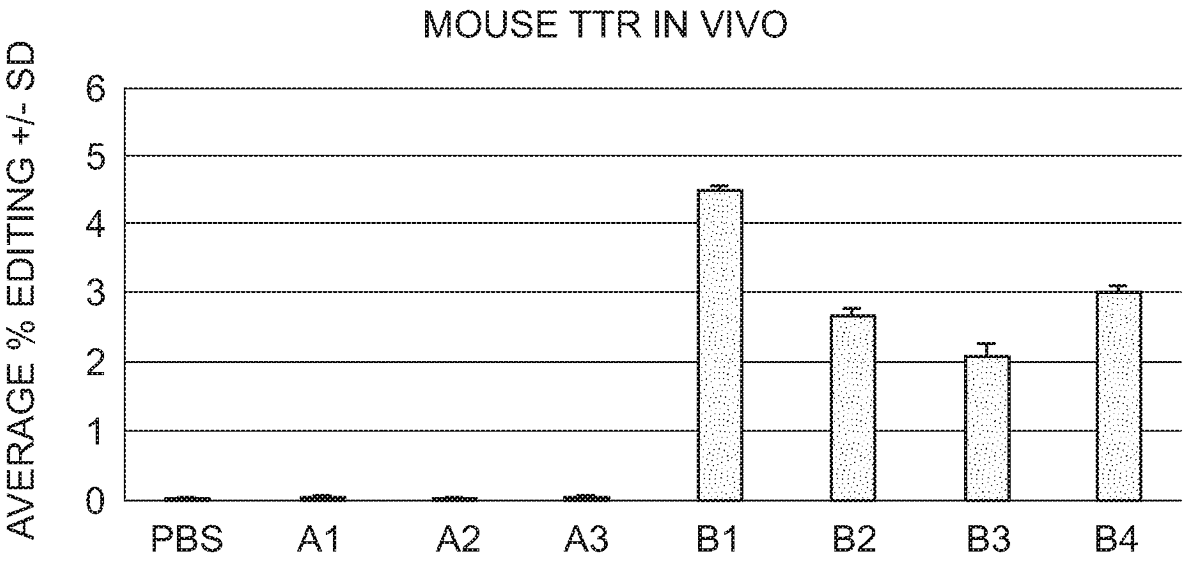
MOUSE TTR IN VIVO
AVERAGE % EDITING +/- SD
6
5
4
3
2
1
0
PBS
A1
A2
A3
B1
B2
B3
B4

FIG. 3B

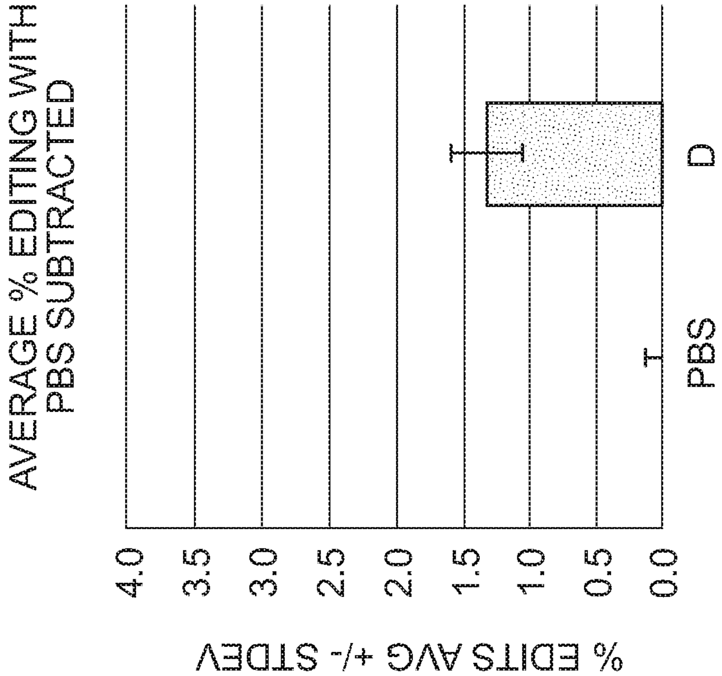
AVERAGE % EDITING WITH
PBS SUBTRACTED
% EDITS AVG +/- STDEV
4.0
3.5
3.0
2.5
2.0
1.5
1.0
0.5
0.0
PBS
D

FIG. 4B

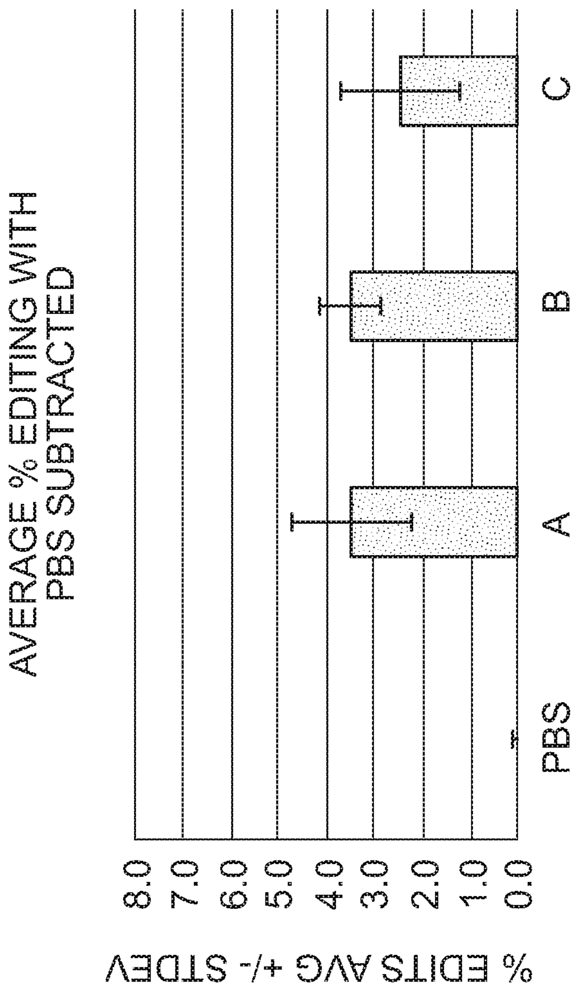
AVERAGE % EDITING WITH
PBS SUBTRACTED
% EDITS AVG +/- STDEV
8.0
7.0
6.0
5.0
4.0
3.0
2.0
1.0
0.0
PBS
A
B
C

FIG. 4A

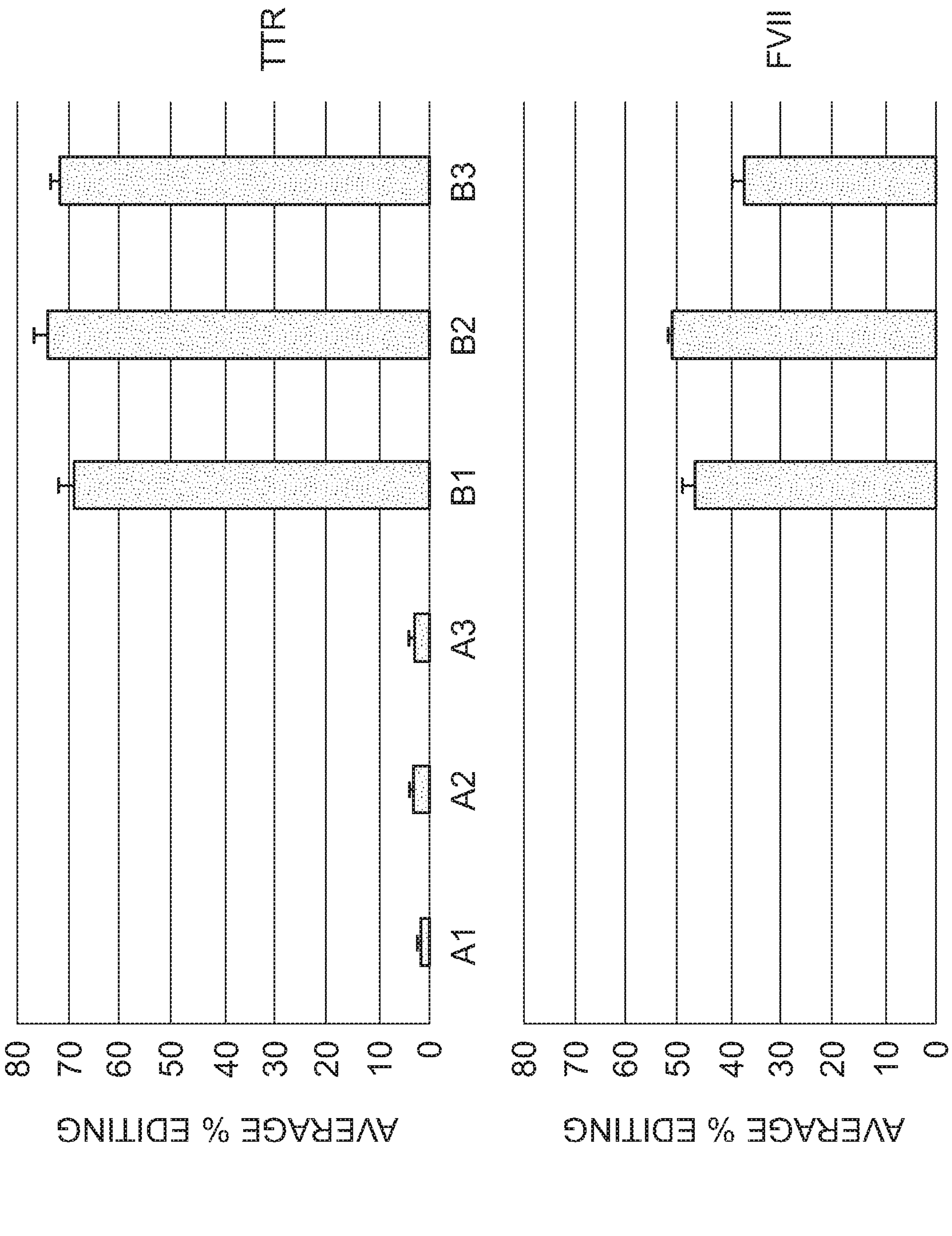
TTR
AVERAGE % EDITING
0
10
20
30
40
50
60
70
80
A1
A2
A3
B1
B2
B3
FVII
AVERAGE % EDITING
0
10
20
30
40
50
60
70
80
C1
C2
C3
D1
D2
D3

FIG. 5

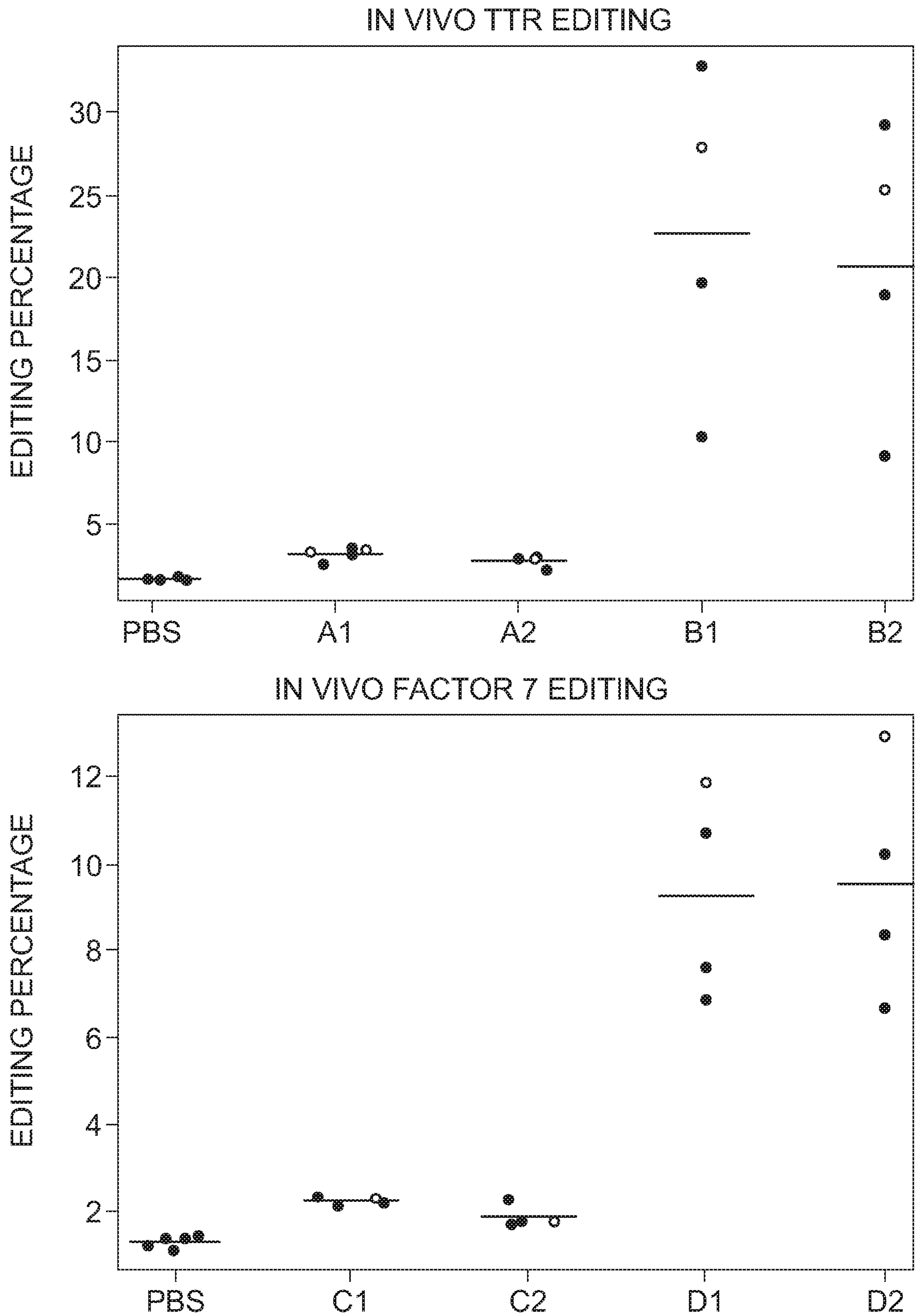
IN VIVO TTR EDITING
EDITING PERCENTAGE
30
25
20
15
10
5
PBS
A1
A2
B1
B2
IN VIVO FACTOR 7 EDITING
EDITING PERCENTAGE
12
10
8
6
4
2
PBS
C1
C2
D1
D2

FIG. 6

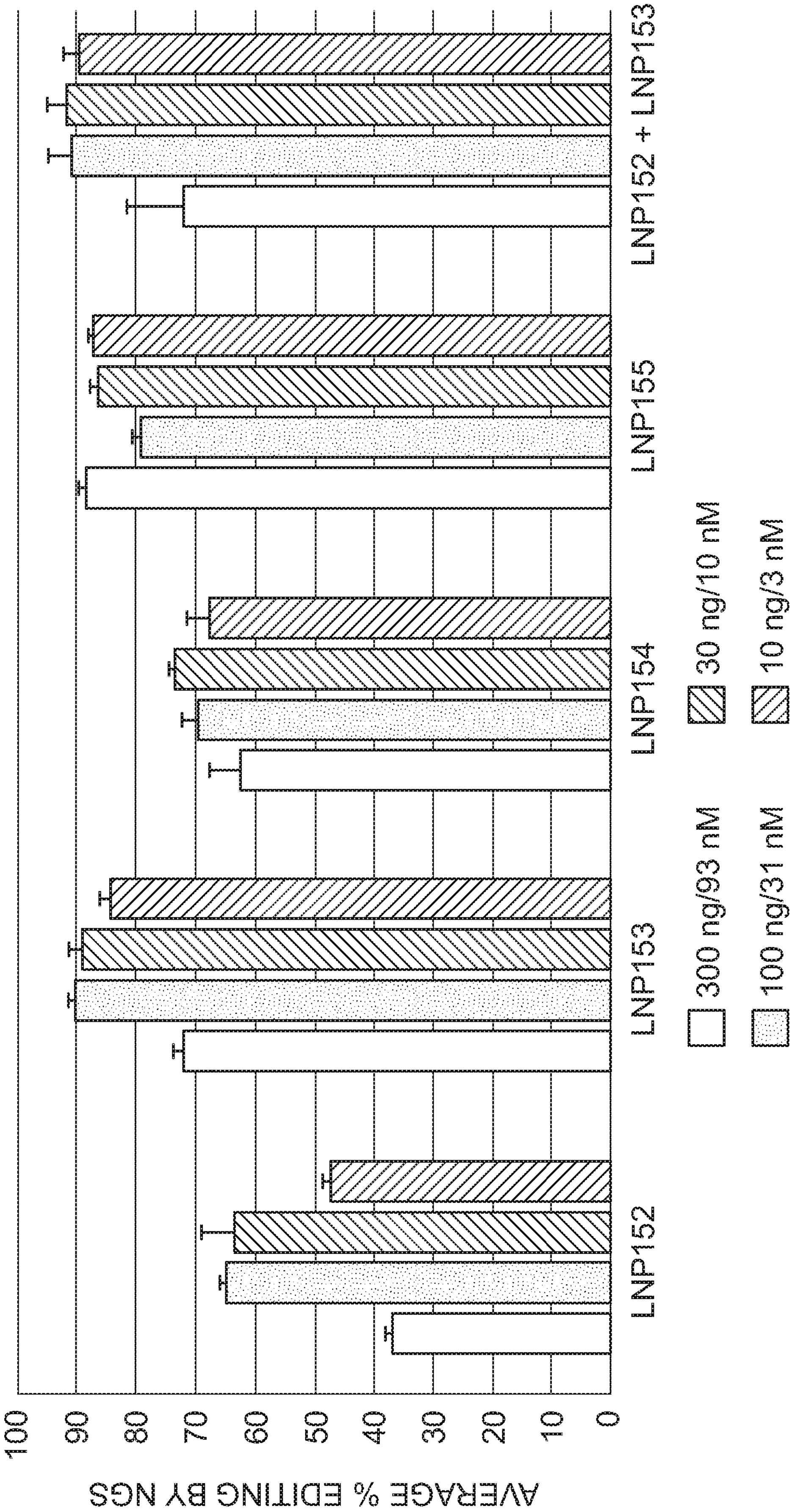
AVERAGE % EDITING BY NGS
0
10
20
30
40
50
60
70
80
90
100
LNP152
LNP153
LNP154
LNP155
LNP152 + LNP153
300 ng/93 nM
100 ng/31 nM
30 ng/10 nM
10 ng/3 nM

FIG. 7

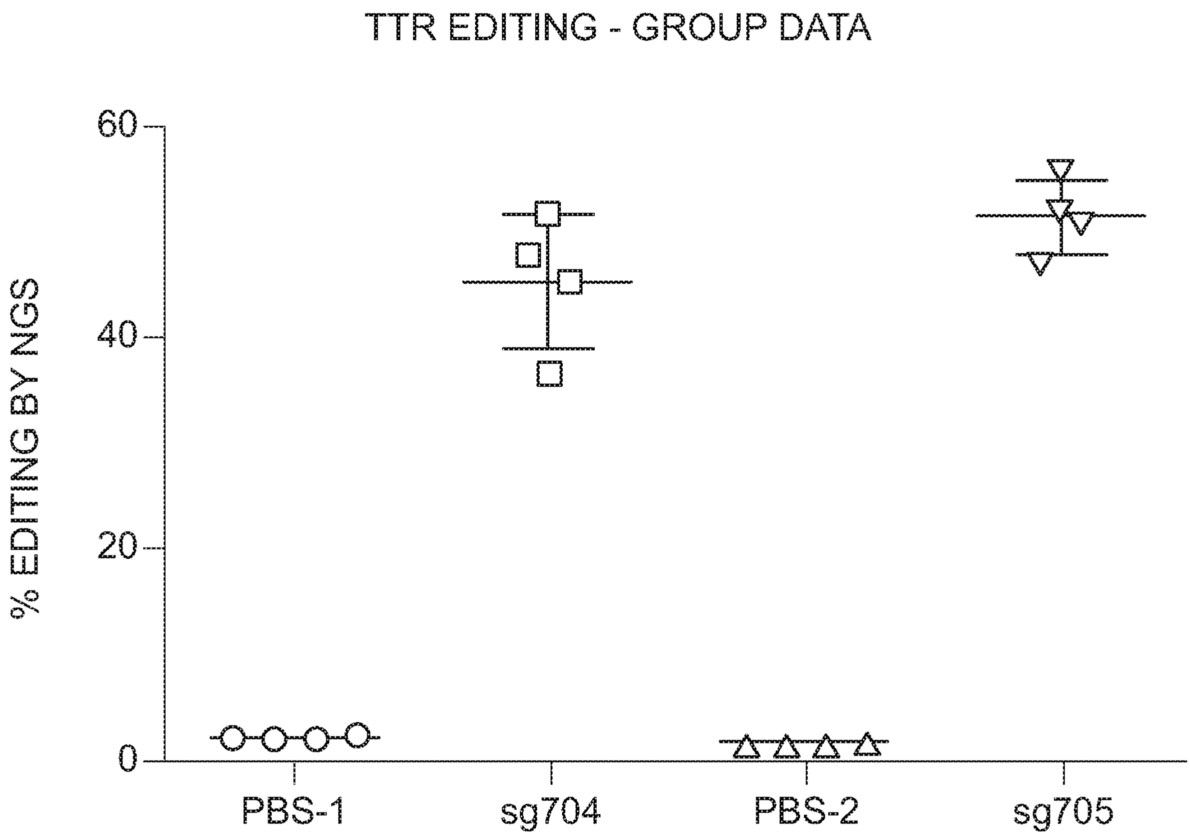
TTR EDITING - GROUP DATA
% EDITING BY NGS
60
40
20
0
PBS-1
sg704
PBS-2
sg705

*FIG. 8A*

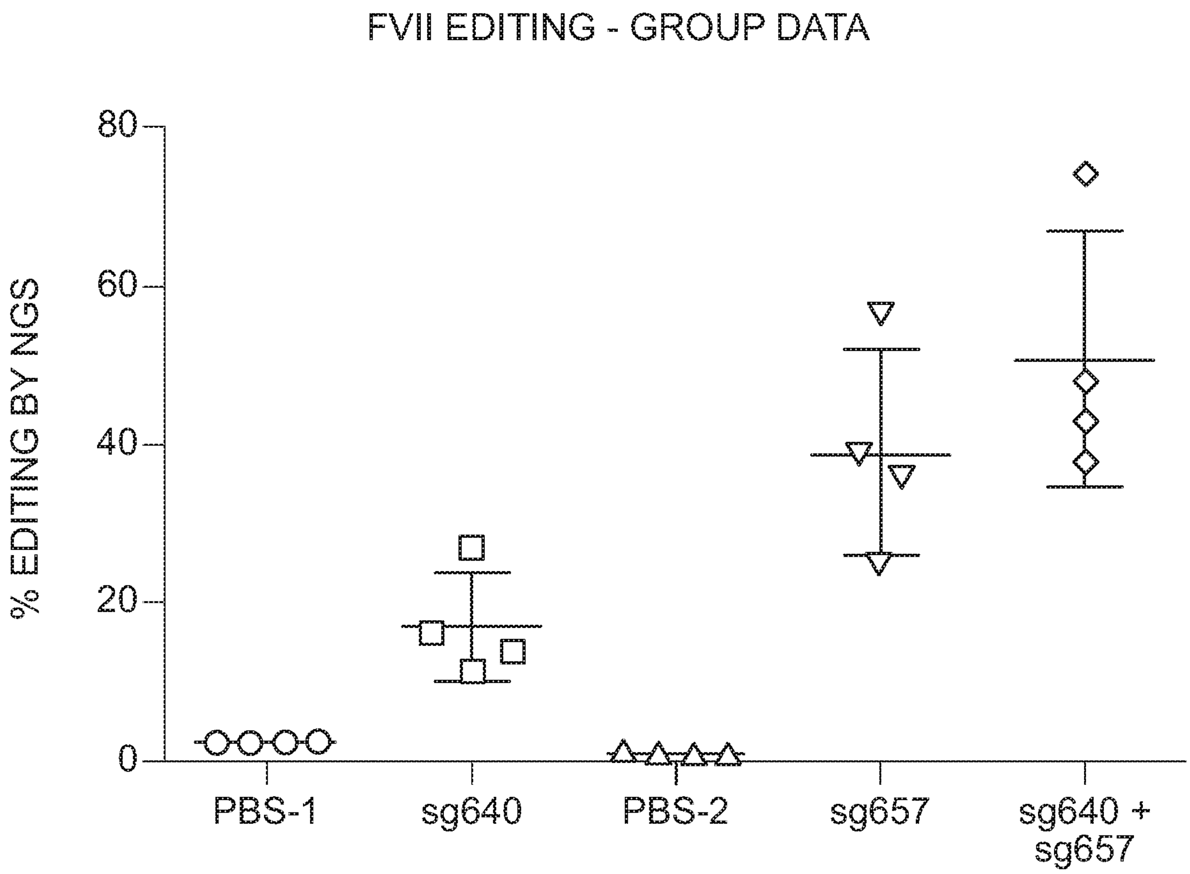
FVII EDITING - GROUP DATA
% EDITING BY NGS
80
60
40
20
0
PBS-1
sg640
PBS-2
sg657
sg640 +
sg657

FIG. 8B

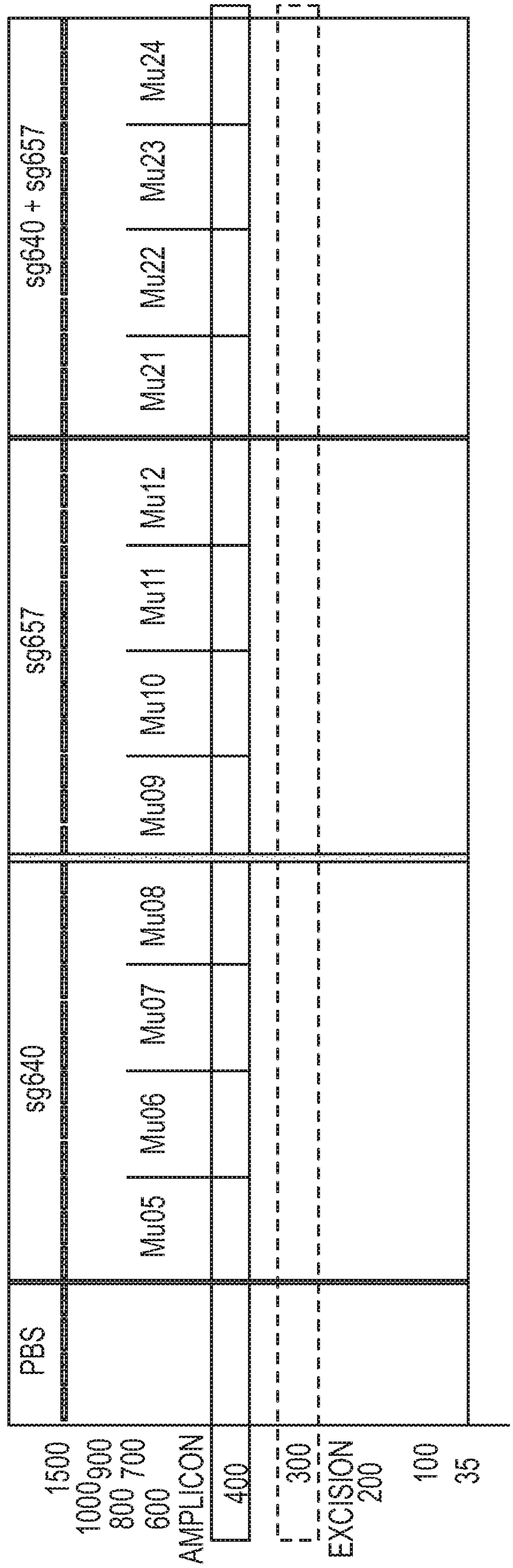
PBS
sg640
sg657
sg640 + sg657
Mu05
Mu06
Mu07
Mu08
Mu09
Mu10
Mu11
Mu12
Mu21
Mu22
Mu23
Mu24
1500
1000
900
800
700
600
AMPLICON
400
300
EXCISION
200
100
35

FIG. 9

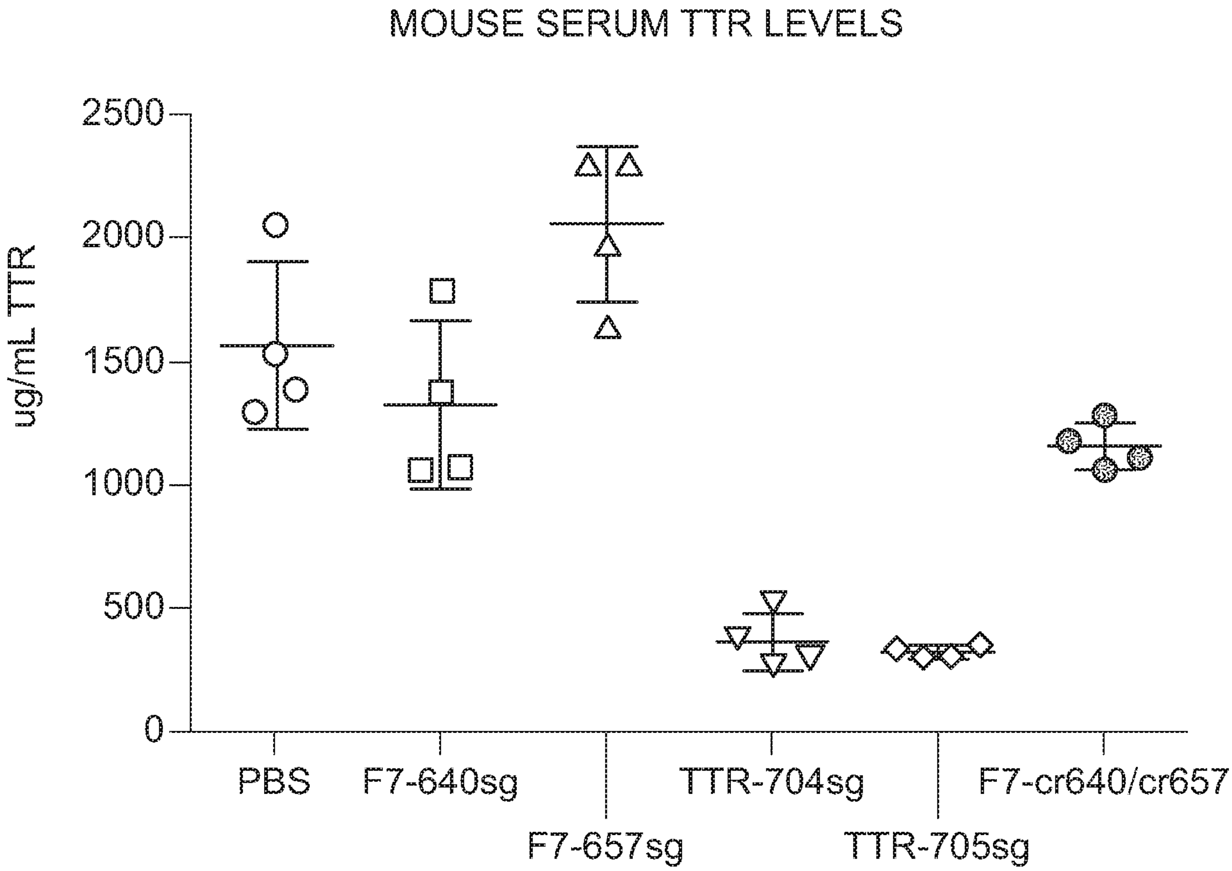
MOUSE SERUM TTR LEVELS
ug/mL TTR
2500
2000
1500
1000
500
0
PBS
F7-640sg
F7-657sg
TTR-704sg
TTR-705sg
F7-cr640/cr657

*FIG. 10*

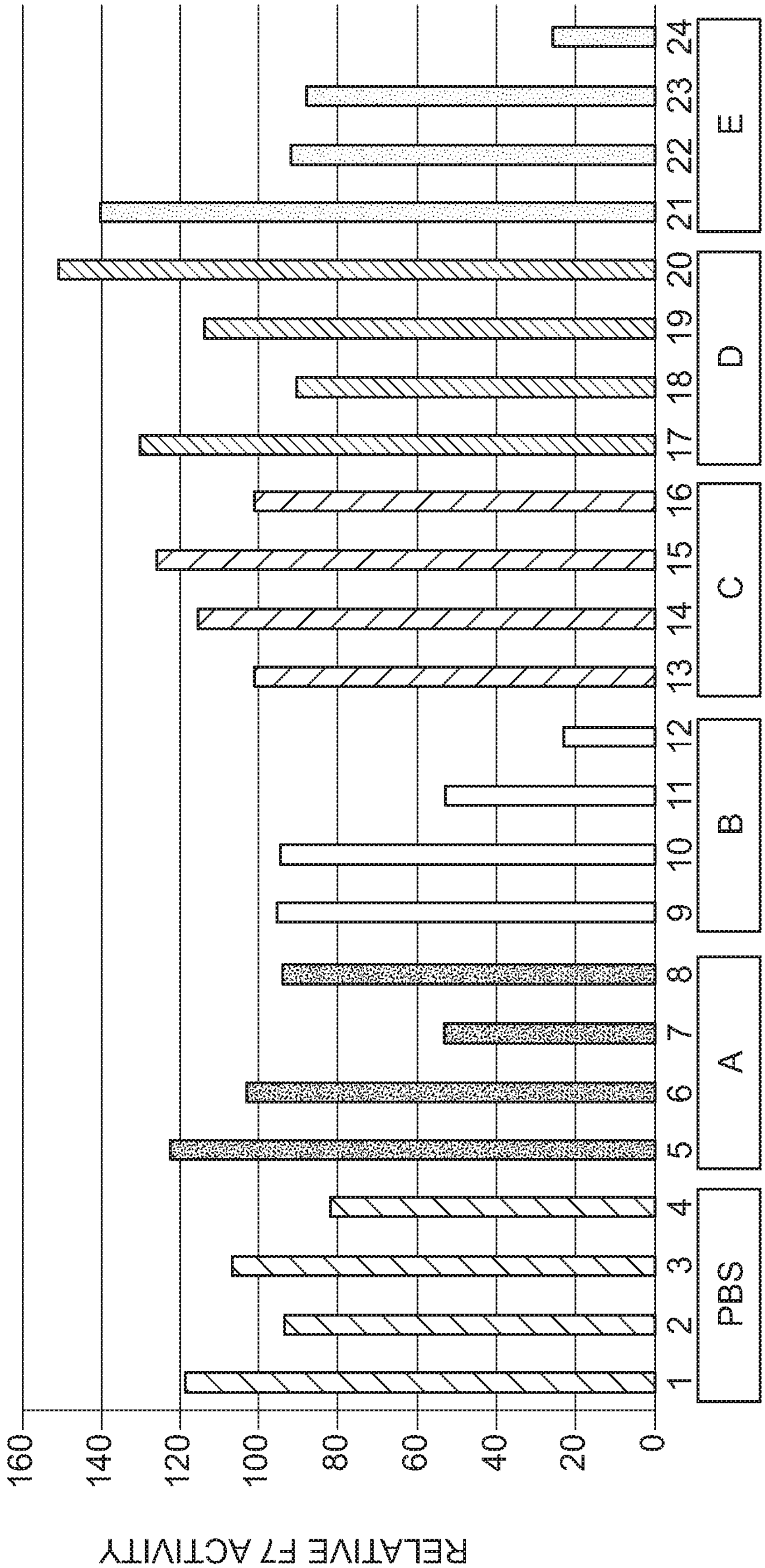
RELATIVE F7 ACTIVITY
0
20
40
60
80
100
120
140
160
1
2
3
4
5
6
7
8
9
10
11
12
13
14
15
16
17
18
19
20
21
22
23
24
PBS
A
B
C
D
E

FIG. 11

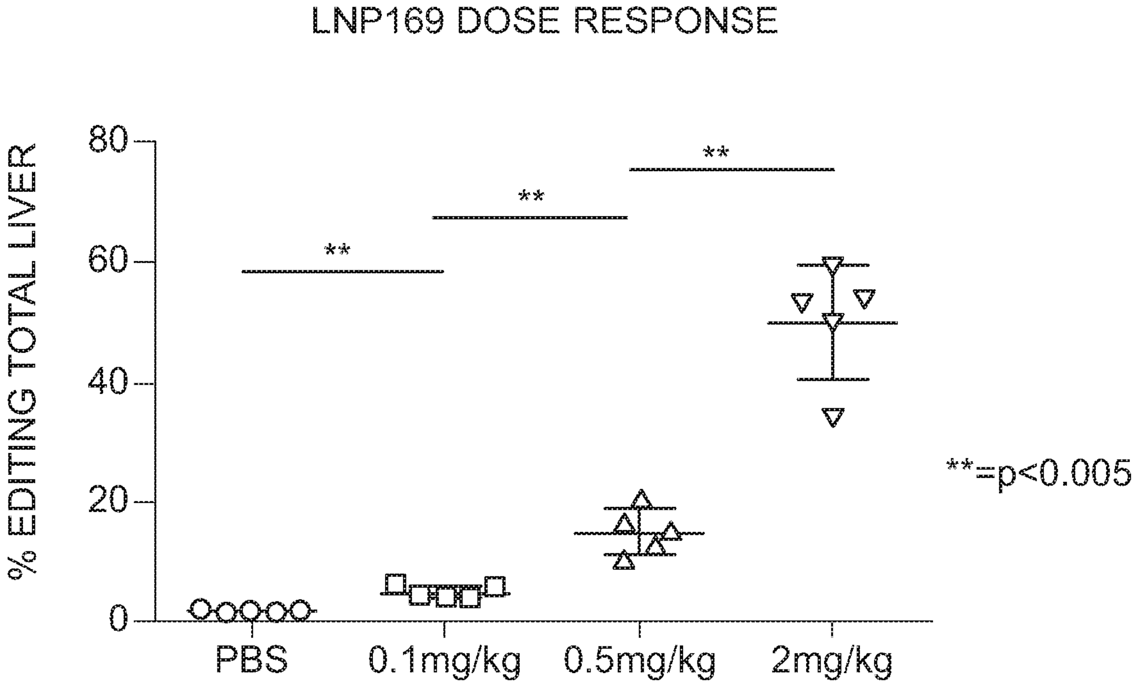
LNP169 DOSE RESPONSE
% EDITING TOTAL LIVER
80
60
40
20
0
**
**
**
PBS
0.1mg/kg
0.5mg/kg
2mg/kg
**=p<0.005

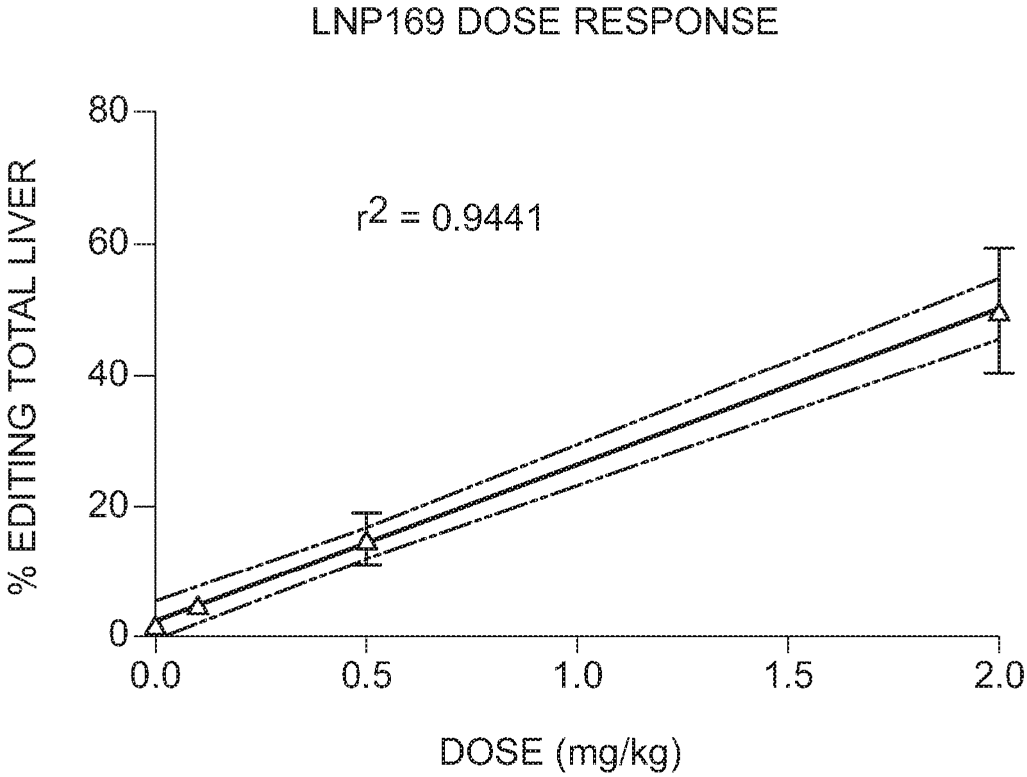
LNP169 DOSE RESPONSE
% EDITING TOTAL LIVER
80
60
40
20
0
r2 = 0.9441
0.0
0.5
1.0
1.5
2.0
DOSE (mg/kg)

*FIG. 12A*

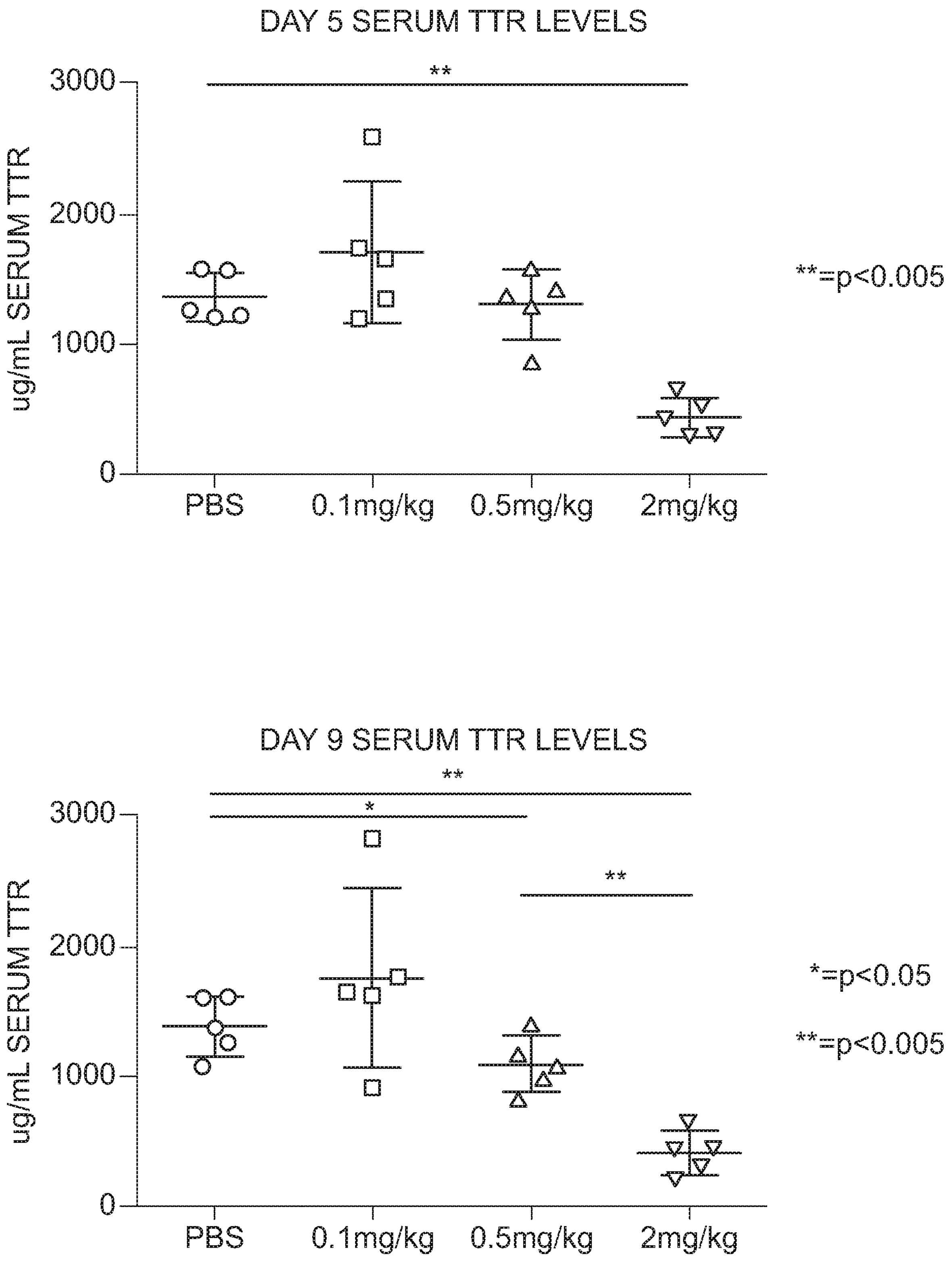
DAY 5 SERUM TTR LEVELS
**
3000
2000
1000
0
ug/mL SERUM TTR
PBS
0.1mg/kg
0.5mg/kg
2mg/kg
**=p<0.005
DAY 9 SERUM TTR LEVELS
**
*
**
3000
2000
1000
0
ug/mL SERUM TTR
PBS
0.1mg/kg
0.5mg/kg
2mg/kg
*=p<0.05
**=p<0.005

FIG. 12B

mRNA:sgRNA RATIO

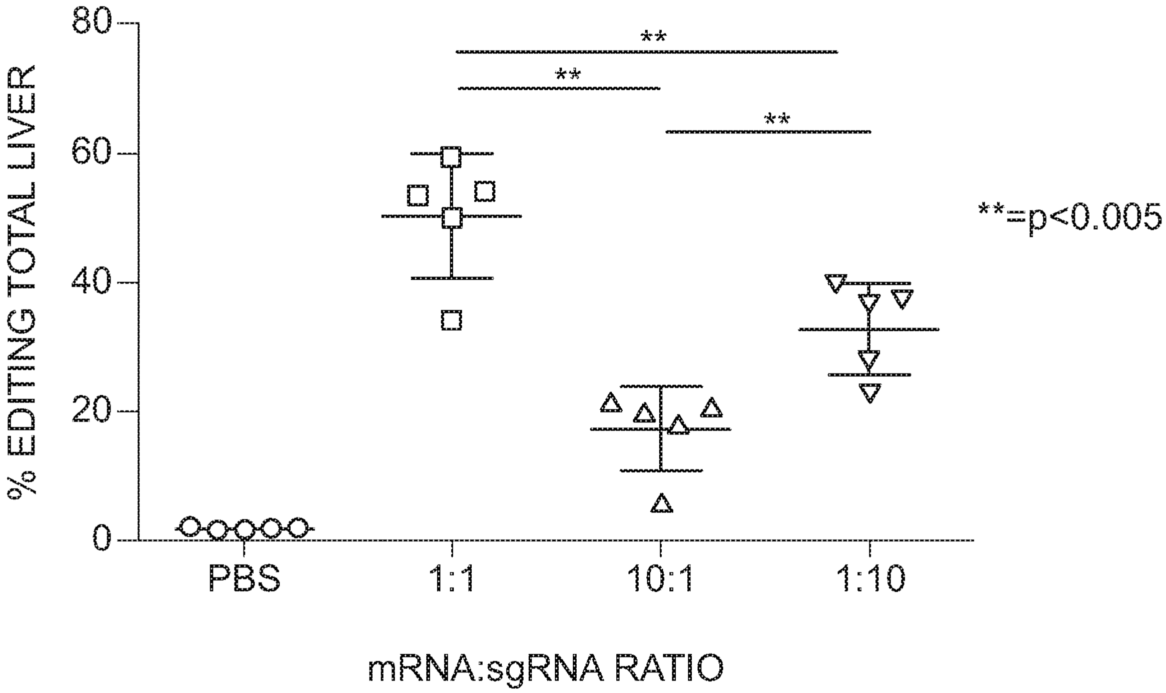


*FIG. 13A*

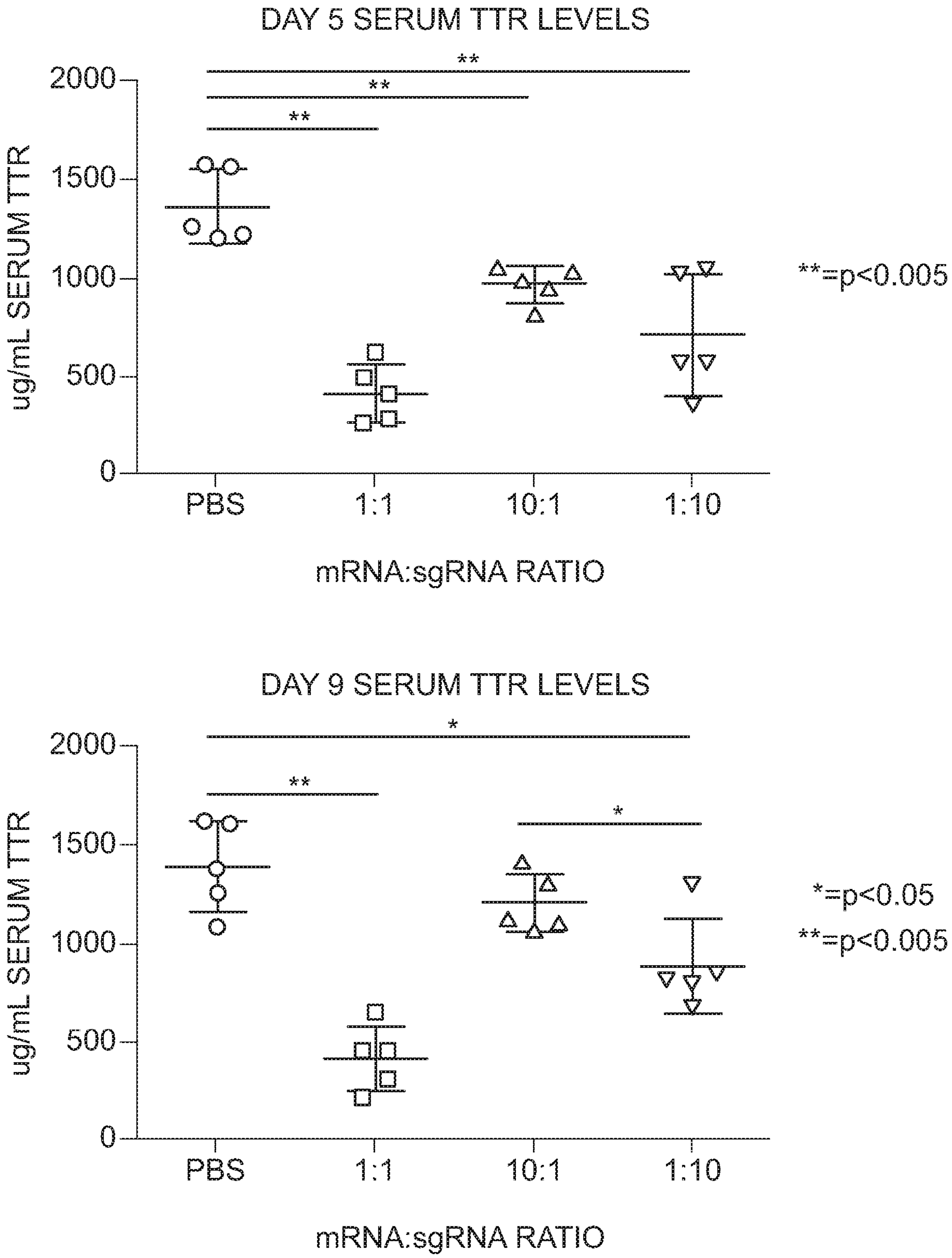
DAY 5 SERUM TTR LEVELS
**
**
**
2000
1500
1000
500
0
ug/mL SERUM TTR
PBS
1:1
10:1
1:10
mRNA:sgRNA RATIO
**=p<0.005
DAY 9 SERUM TTR LEVELS
*
**
*
2000
1500
1000
500
0
ug/mL SERUM TTR
PBS
1:1
10:1
1:10
mRNA:sgRNA RATIO
*=p<0.05
**=p<0.005

*FIG. 13B*

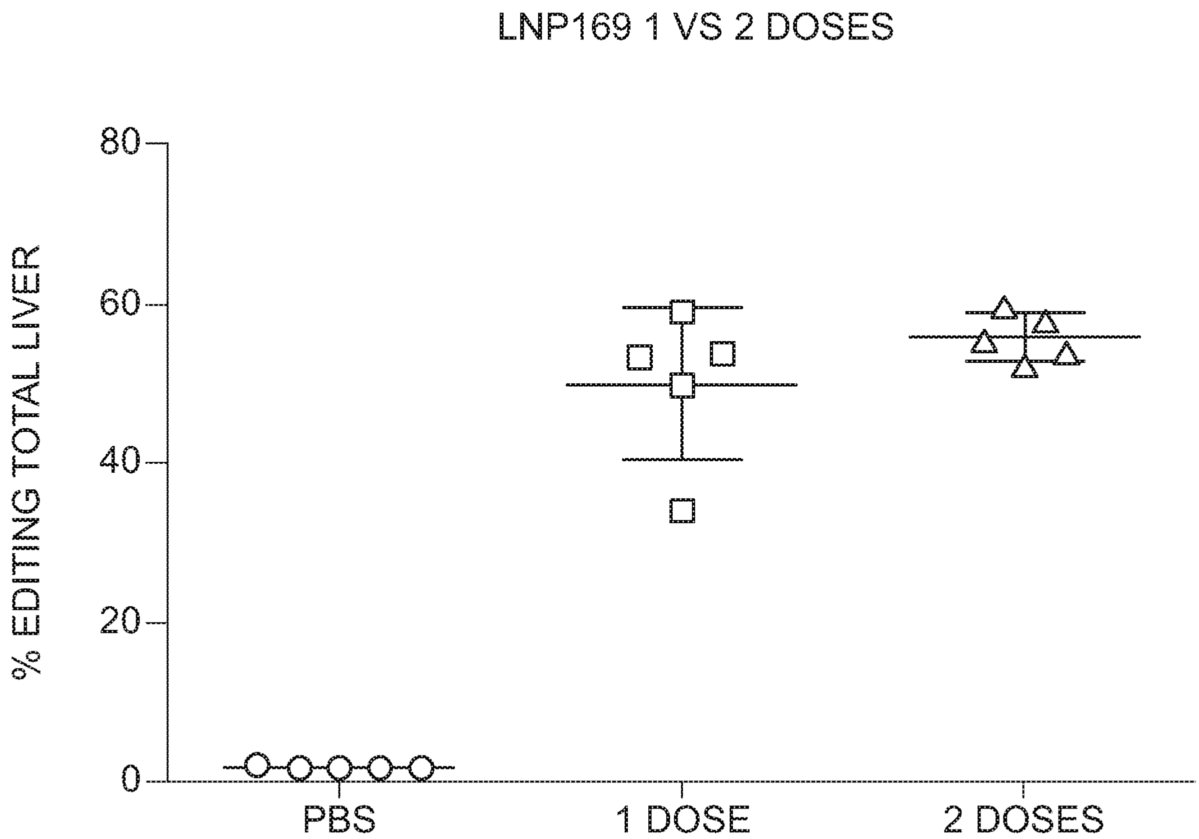
LNP169 1 VS 2 DOSES
% EDITING TOTAL LIVER
80
60
40
20
0
PBS
1 DOSE
2 DOSES

FIG. 14A

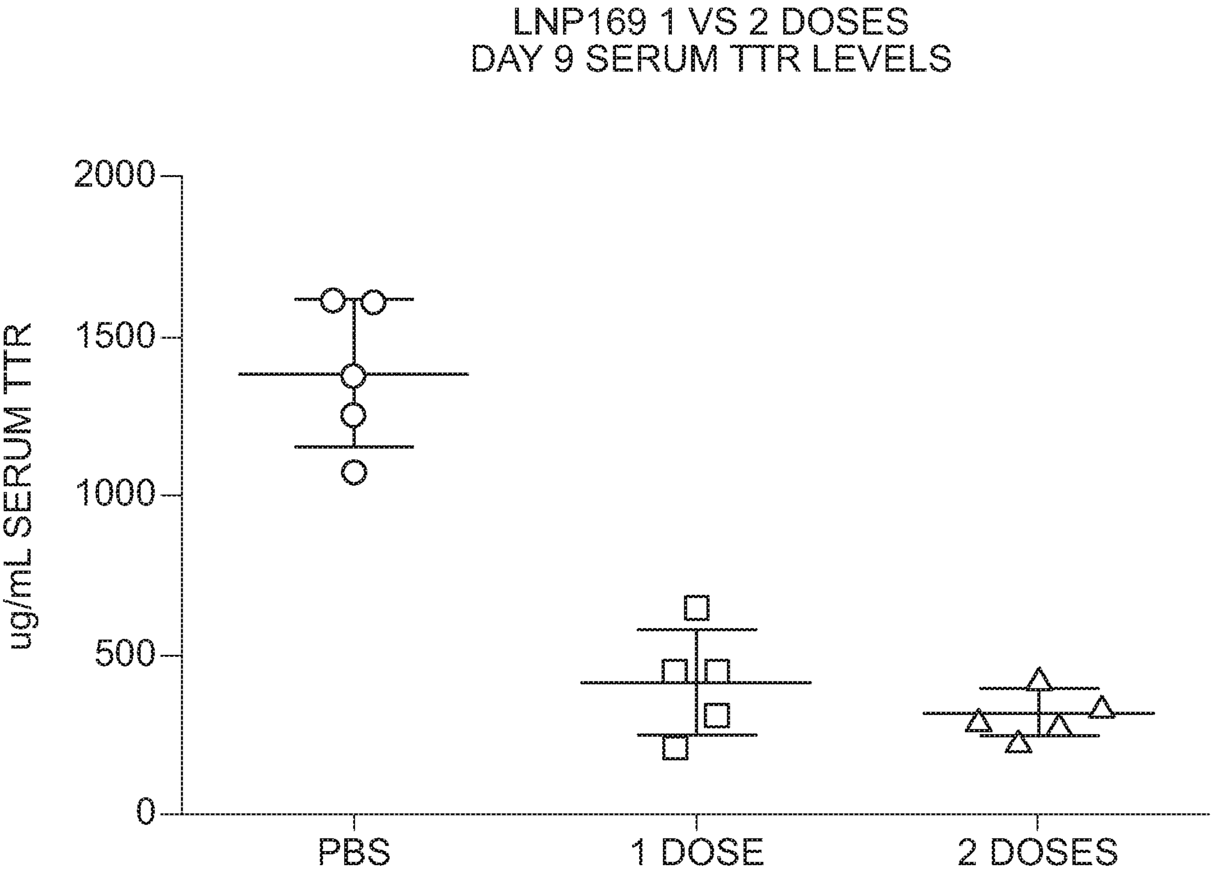
LNP169 1 VS 2 DOSES
DAY 9 SERUM TTR LEVELS
2000
1500
1000
500
0
ug/mL SERUM TTR
PBS
1 DOSE
2 DOSES

*FIG. 14B*

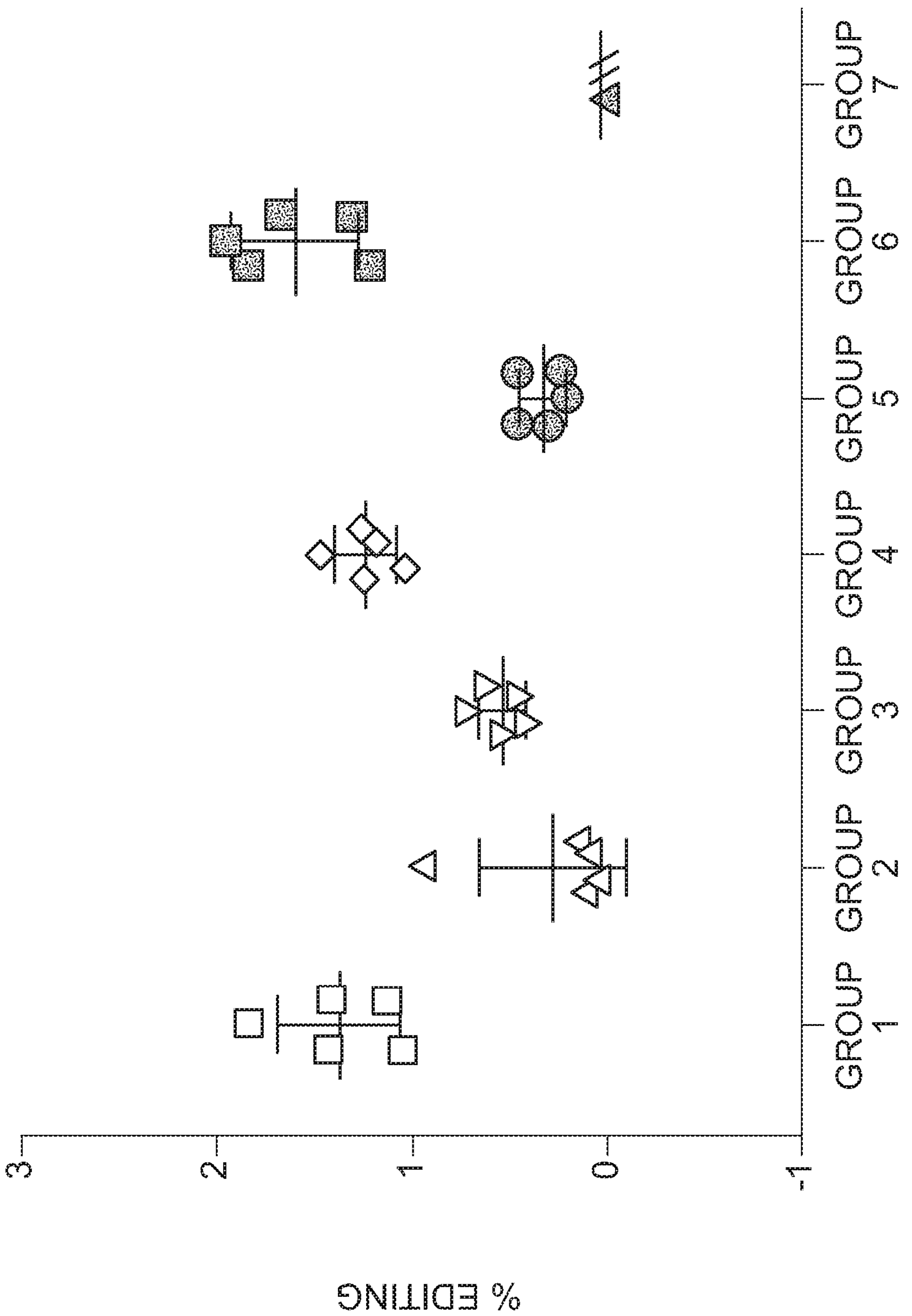
% EDITING
3
2
1
0
-1
GROUP 1
GROUP 2
GROUP 3
GROUP 4
GROUP 5
GROUP 6
GROUP 7

FIG. 15

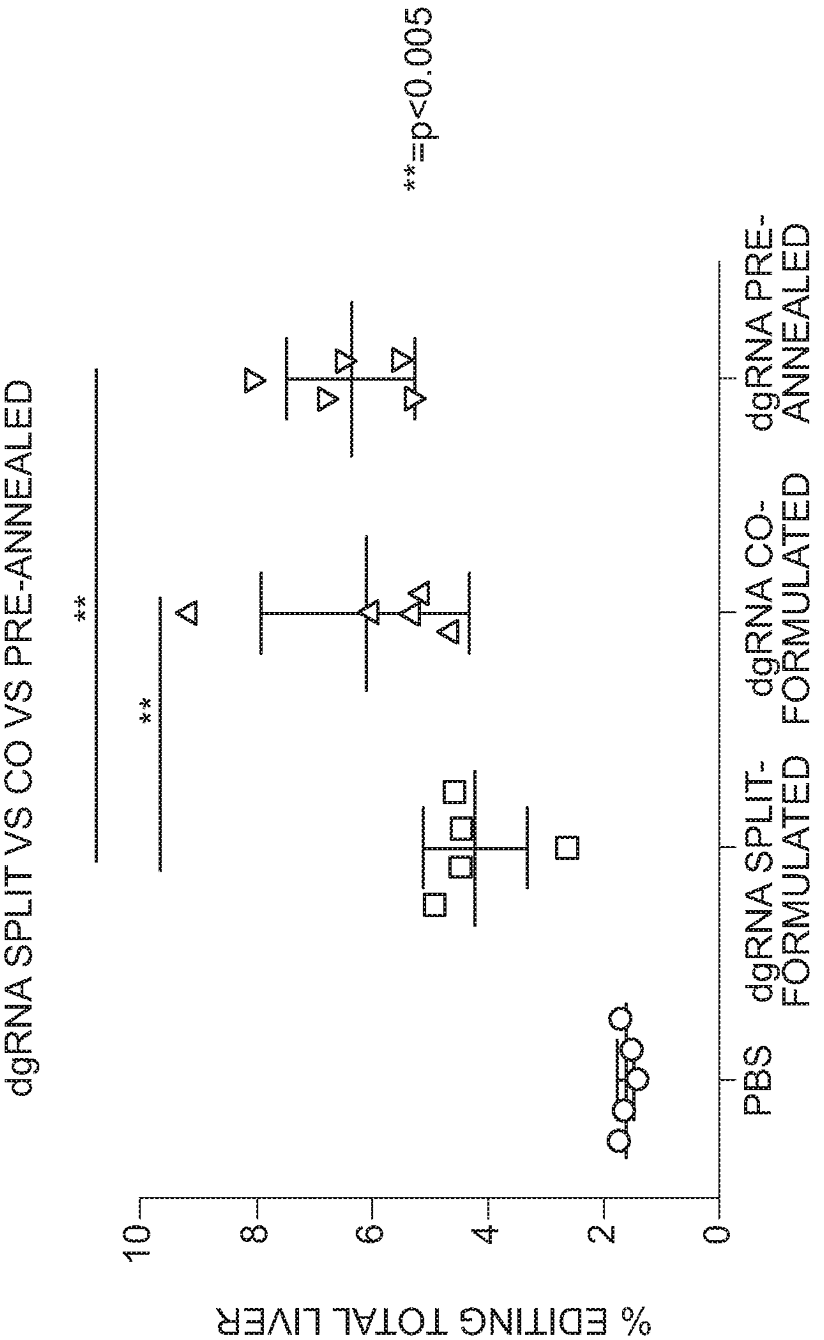

dgRNA SPLIT VS CO VS PRE-ANNEALED
**
**
% EDITING TOTAL LIVER
0
2
4
6
8
10
PBS
dgRNA SPLIT-FORMULATED
dgRNA CO-FORMULATED
dgRNA PRE-ANNEALED
**=p<0.005


*FIG. 16*

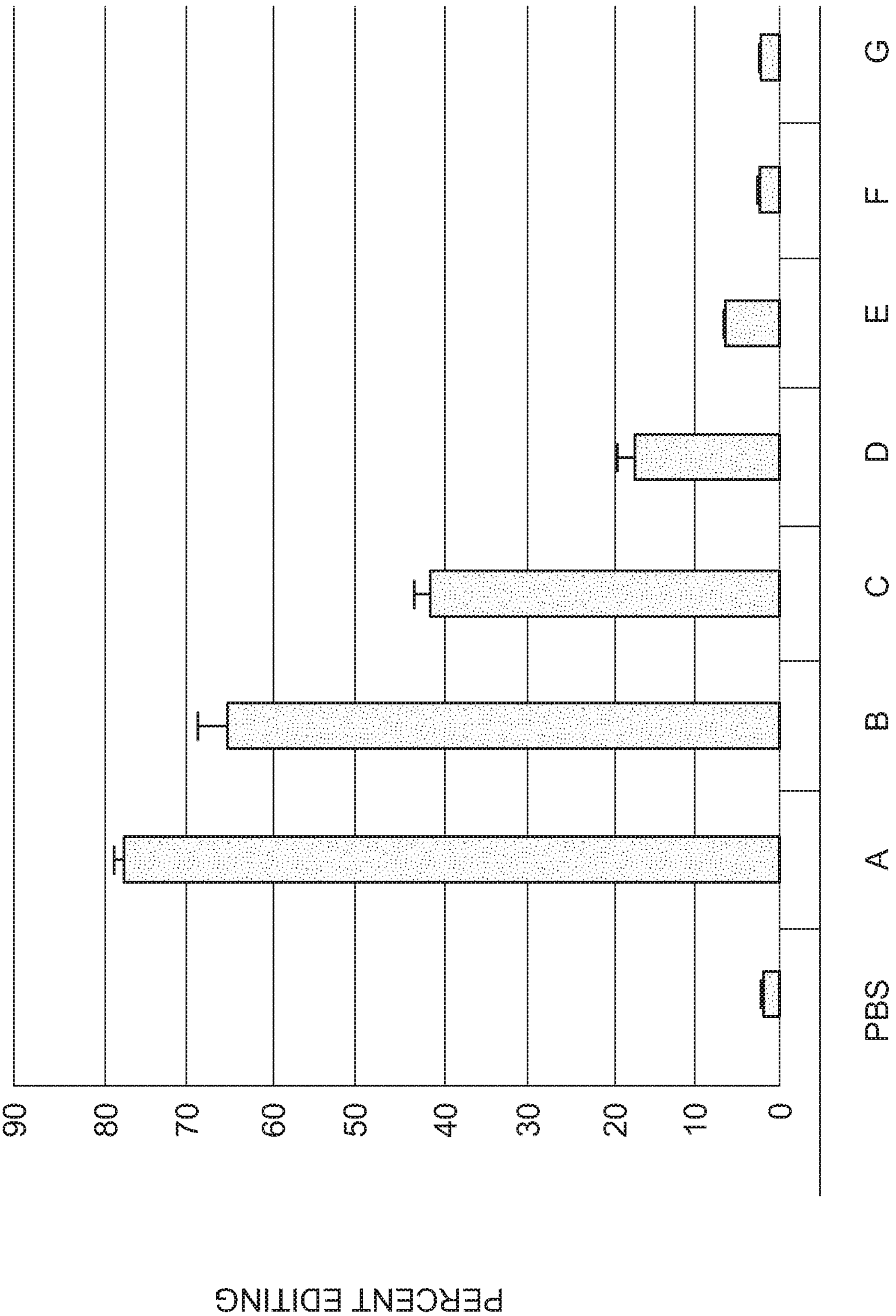


FIG. 17

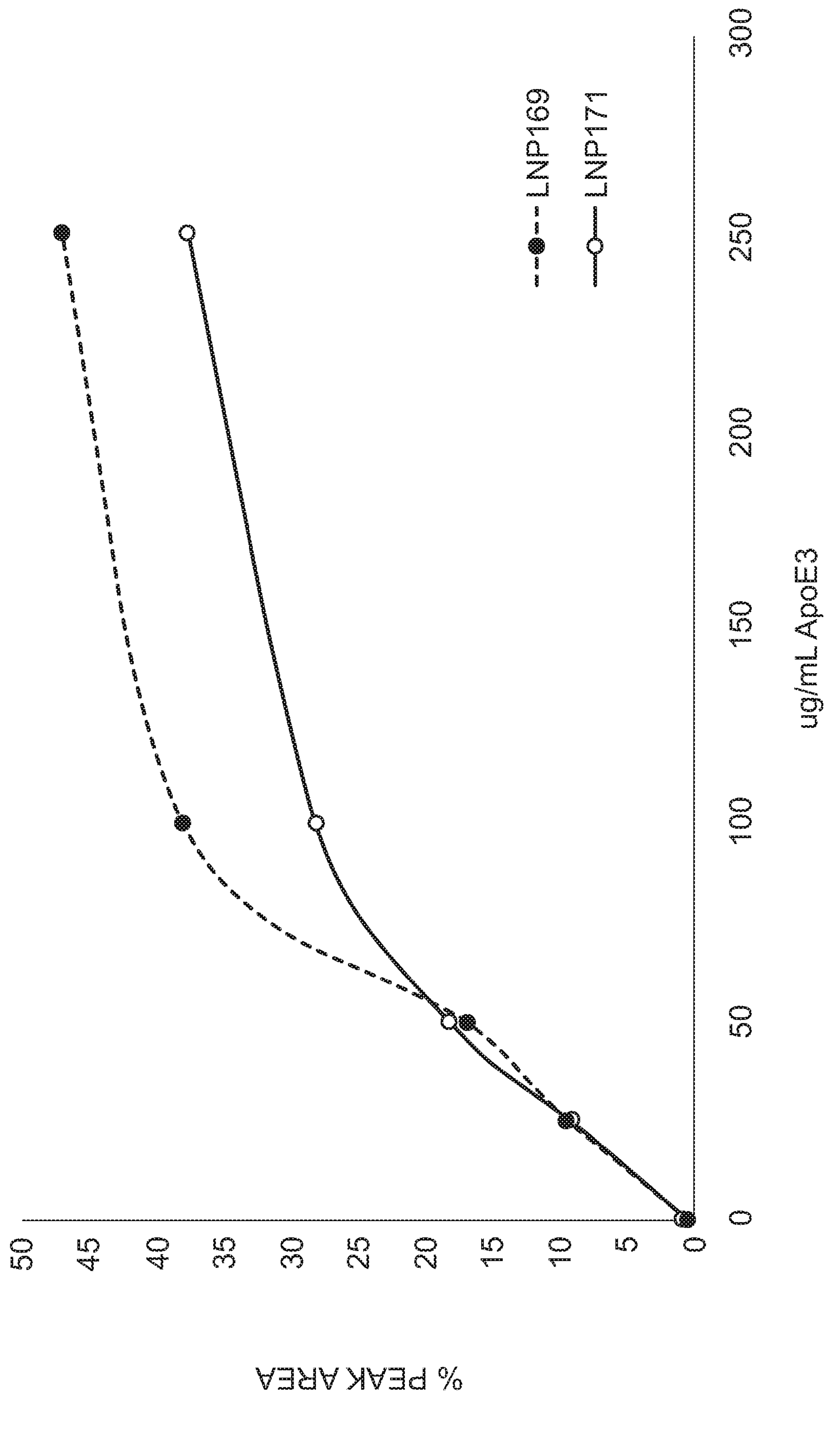
% PEAK AREA
0
5
10
15
20
25
30
35
40
45
50
0
50
100
150
200
250
300
ug/mL ApoE3
LNP169
LNP171

FIG. 18

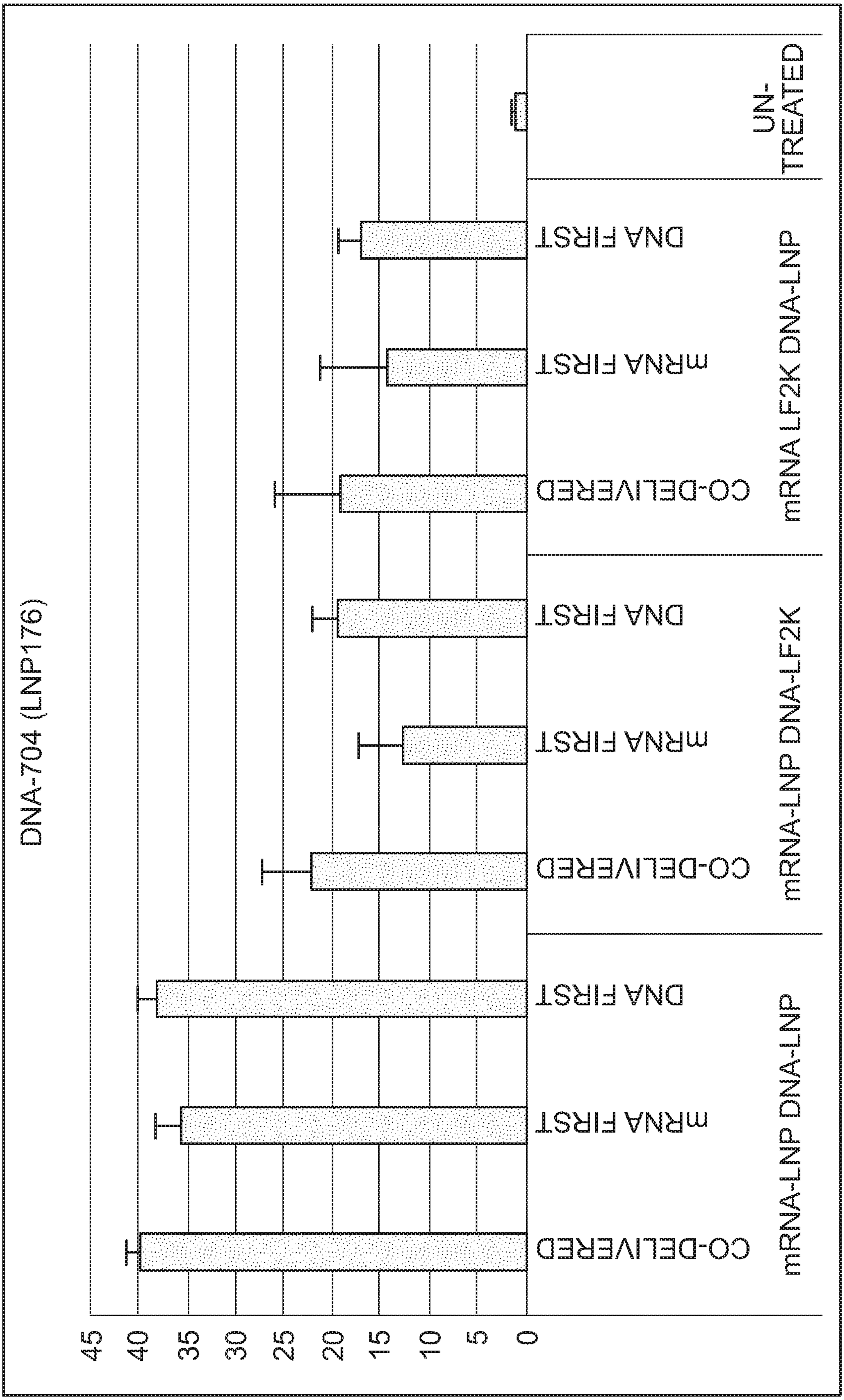
DNA-704 (LNP176)
45
40
35
30
25
20
15
10
5
0
CO-DELIVERED
mRNA FIRST
DNA FIRST
mRNA-LNP DNA-LNP
CO-DELIVERED
mRNA FIRST
DNA FIRST
mRNA-LNP DNA-LF2K
CO-DELIVERED
mRNA FIRST
DNA FIRST
mRNA LF2K DNA-LNP
UN-
TREATED

*FIG. 19*

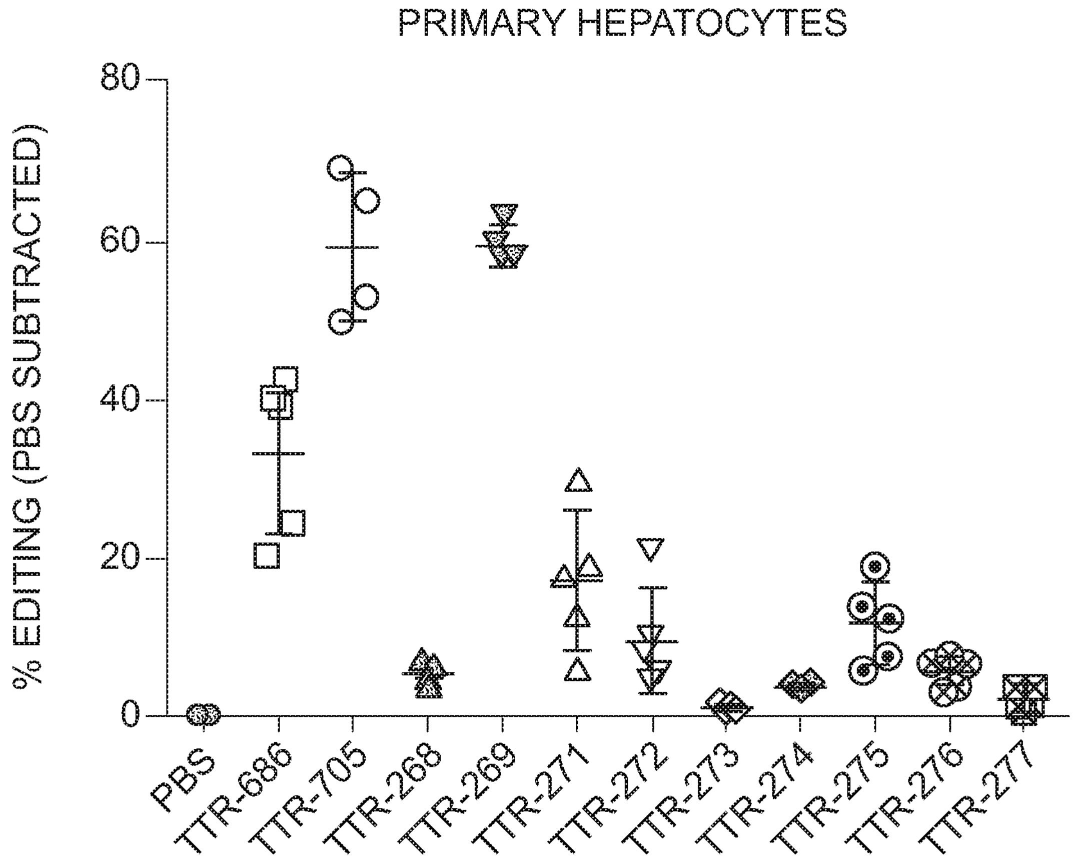
PRIMARY HEPATOCYTES
% EDITING (PBS SUBTRACTED)
80
60
40
20
0
PBS
TTR-686
TTR-705
TTR-268
TTR-269
TTR-271
TTR-272
TTR-273
TTR-274
TTR-275
TTR-276
TTR-277

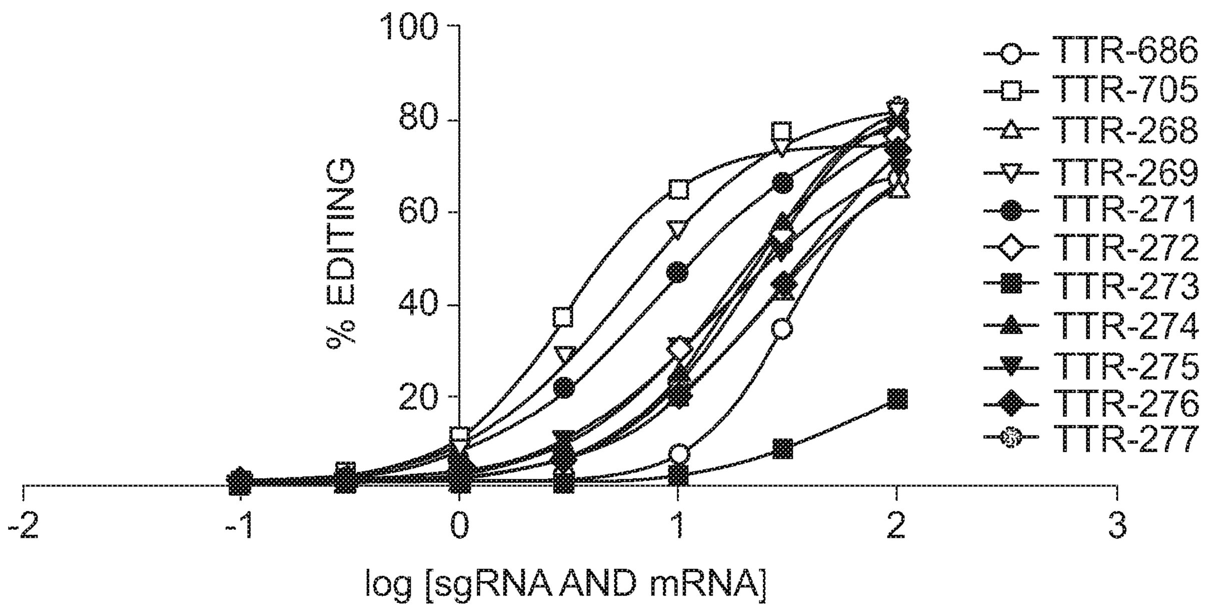
% EDITING
100
80
60
40
20
-2
-1
0
1
2
3
log [sgRNA AND mRNA]
TTR-686
TTR-705
TTR-268
TTR-269
TTR-271
TTR-272
TTR-273
TTR-274
TTR-275
TTR-276
TTR-277

*FIG. 20*

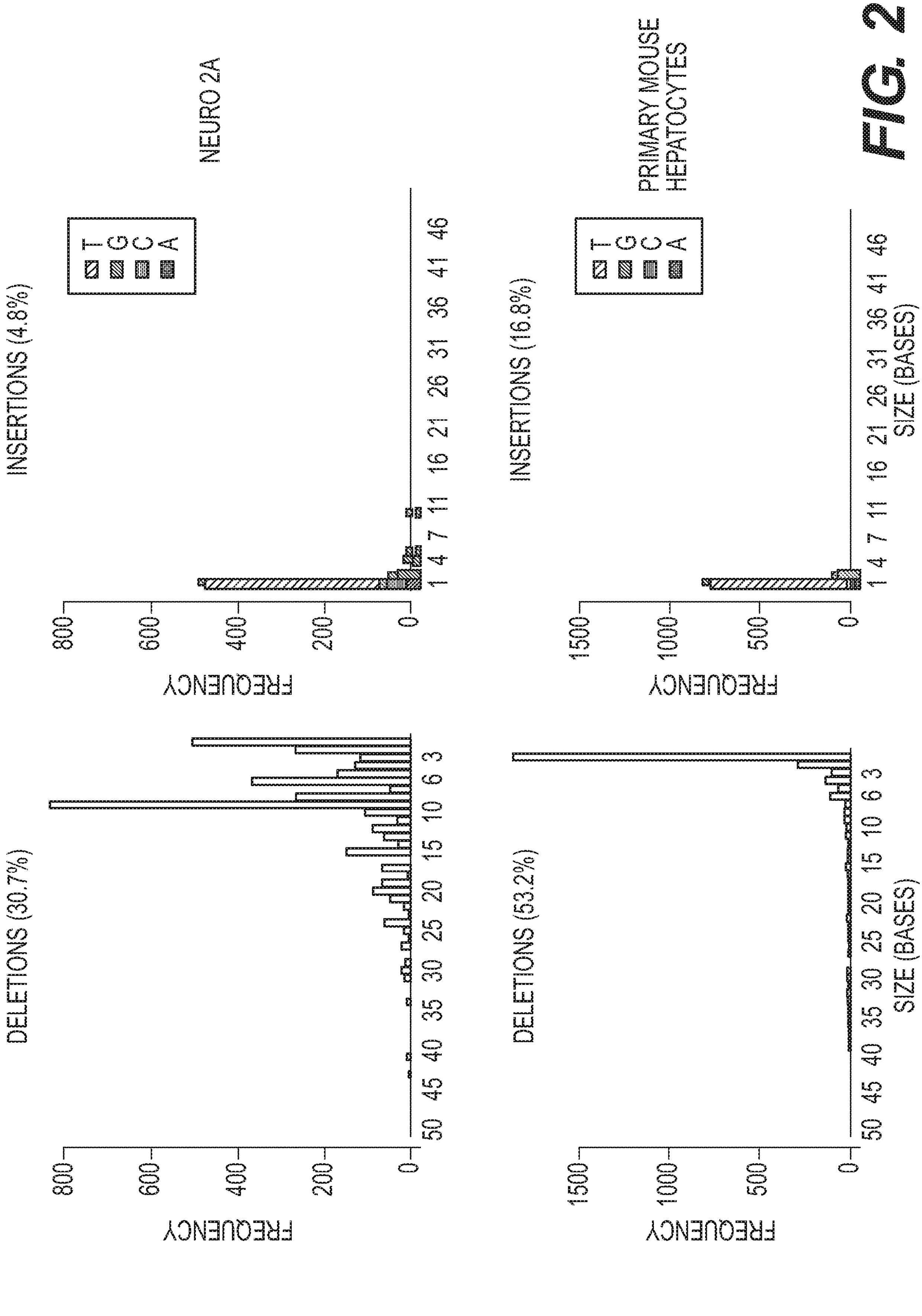
NEURO 2A
DELETIONS (30.7%)
INSERTIONS (4.8%)
T
G
C
A
FREQUENCY
800
600
400
200
0
50 45 40 35 30 25 20 15 10 6 3
1 4 7 11 16 21 26 31 36 41 46
PRIMARY MOUSE HEPATOCYTES
DELETIONS (53.2%)
INSERTIONS (16.8%)
1500
1000
500
SIZE (BASES)

FIG. 21

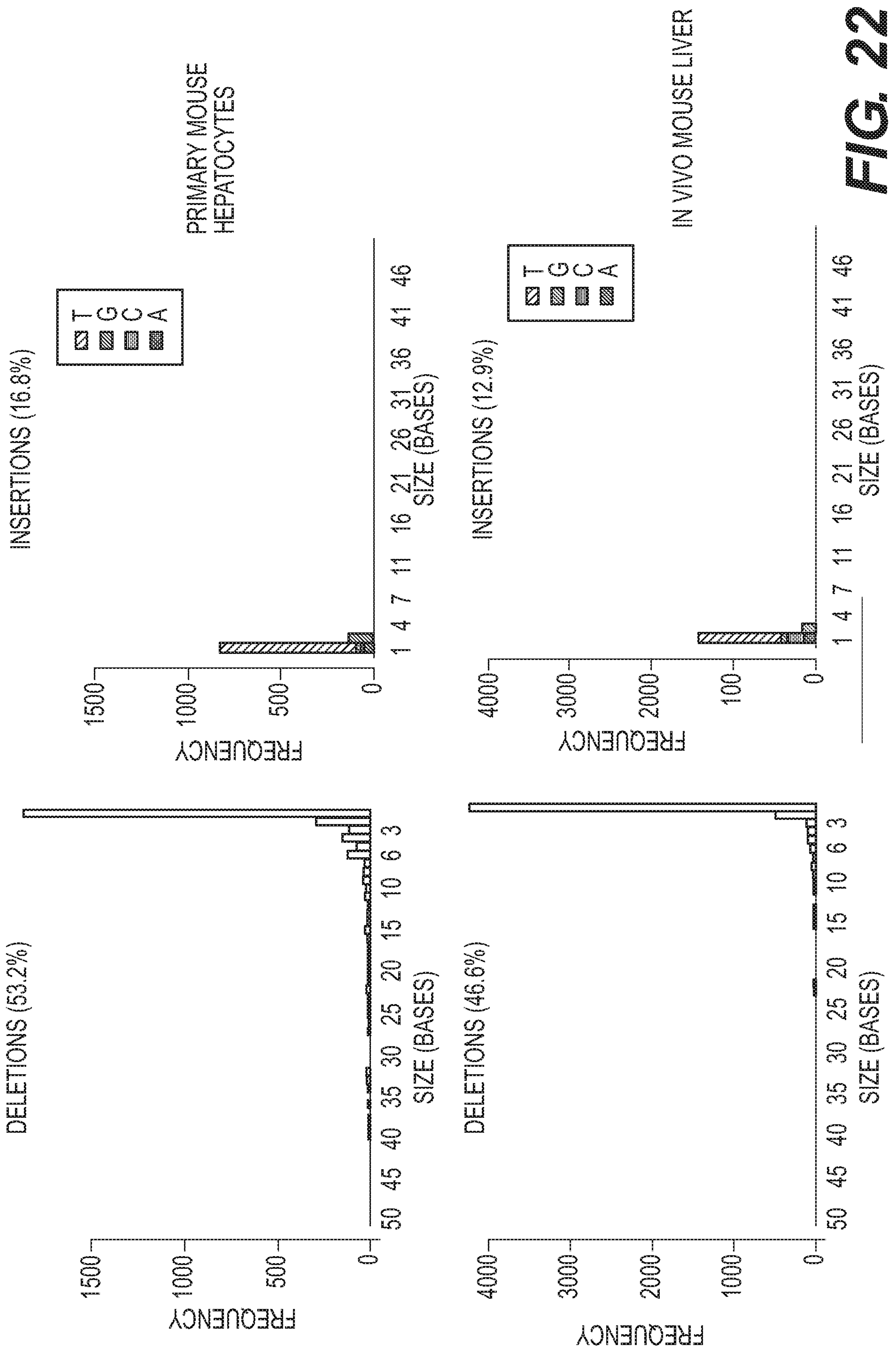
PRIMARY MOUSE HEPATOCYTES
DELETIONS (53.2%)
FREQUENCY
1500
1000
500
0
50 45 40 35 30 25 20 15 10 6 3
SIZE (BASES)
INSERTIONS (16.8%)
T
G
C
A
FREQUENCY
1500
1000
500
0
1 4 7 11 16 21 26 31 36 41 46
SIZE (BASES)
IN VIVO MOUSE LIVER
DELETIONS (46.6%)
FREQUENCY
4000
3000
2000
1000
0
50 45 40 35 30 25 20 15 10 6 3
SIZE (BASES)
INSERTIONS (12.9%)
T
G
C
A
FREQUENCY
4000
3000
2000
100
0
1 4 7 11 16 21 26 31 36 41 46
SIZE (BASES)

FIG. 22

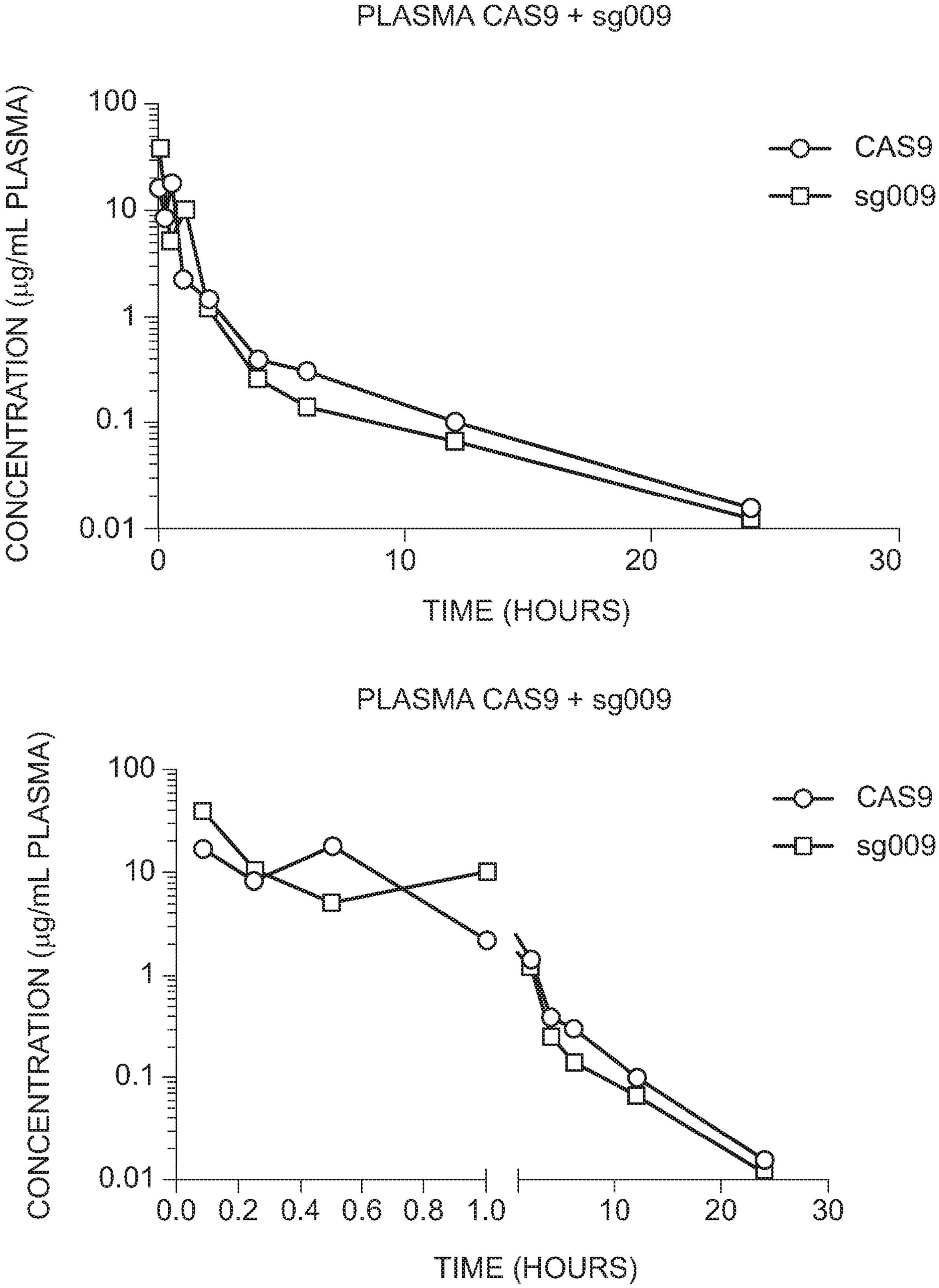
PLASMA CAS9 + sg009
CONCENTRATION (µg/mL PLASMA)
100
10
1
0.1
0.01
CAS9
sg009
0
10
20
30
TIME (HOURS)
PLASMA CAS9 + sg009
CONCENTRATION (µg/mL PLASMA)
100
10
1
0.1
0.01
CAS9
sg009
0.0
0.2
0.4
0.6
0.8
1.0
10
20
30
TIME (HOURS)

*FIG. 23*

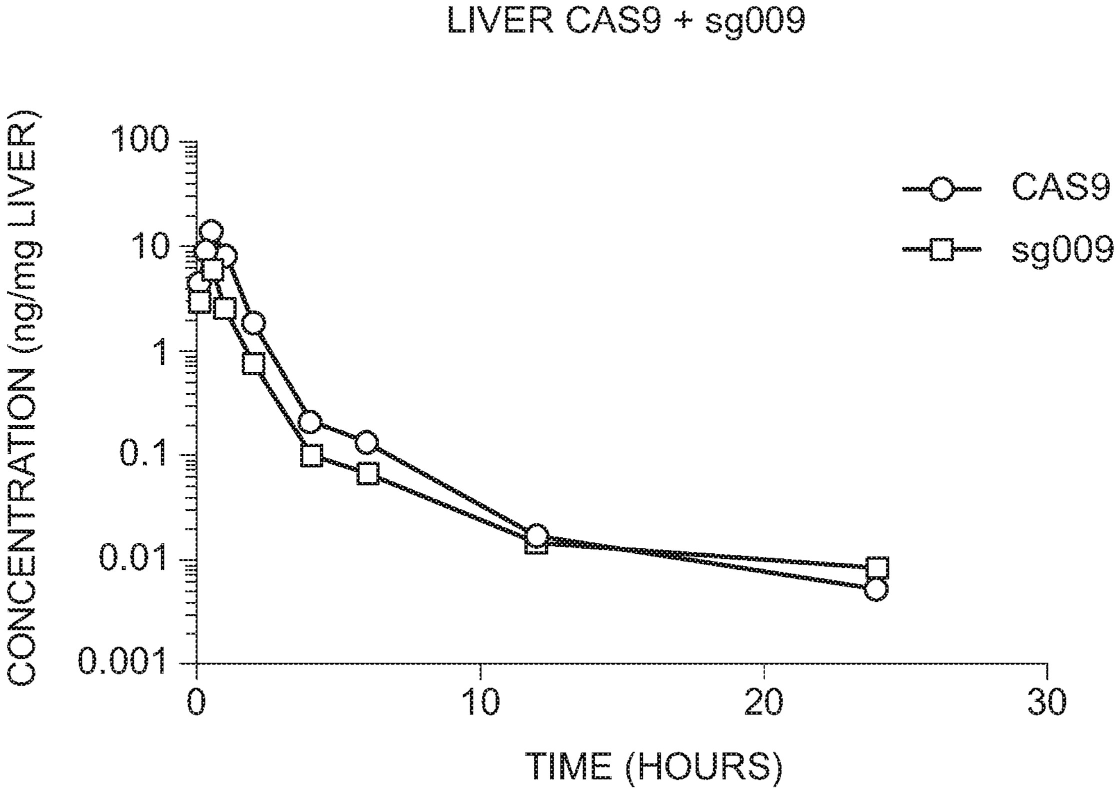
LIVER CAS9 + sg009
CONCENTRATION (ng/mg LIVER)
100
10
1
0.1
0.01
0.001
0
10
20
30
TIME (HOURS)
CAS9
sg009

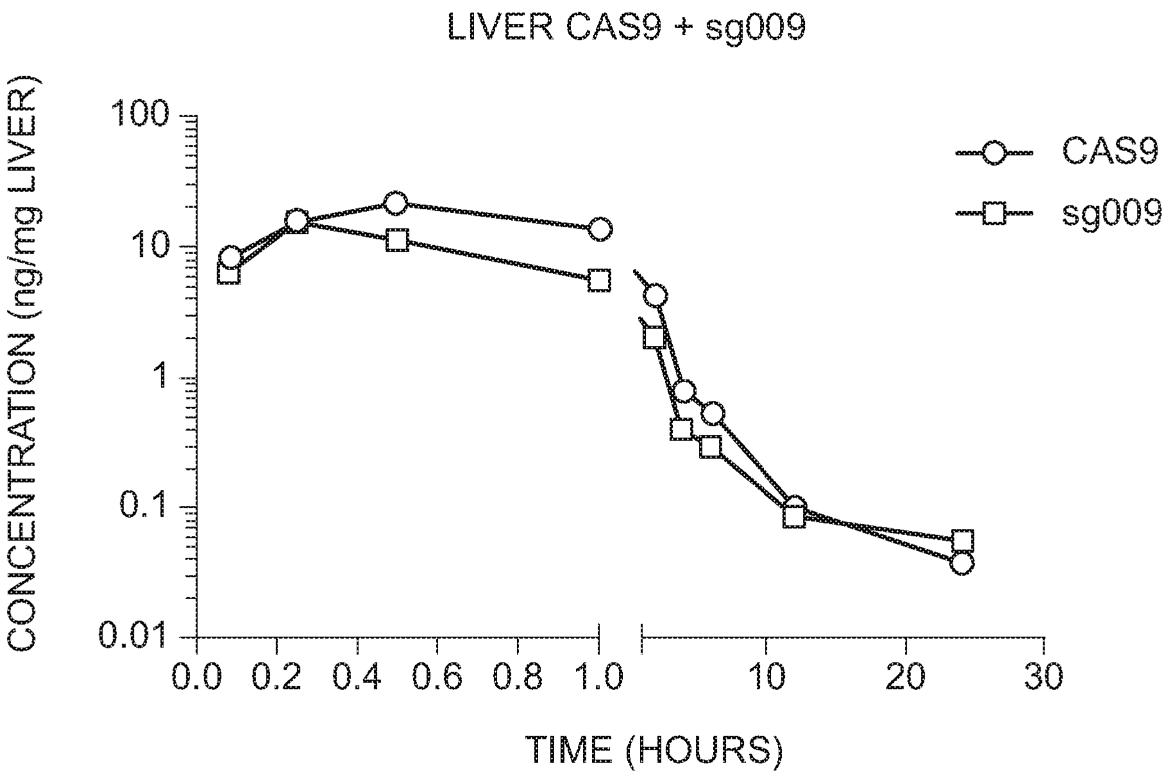
LIVER CAS9 + sg009
CONCENTRATION (ng/mg LIVER)
100
10
1
0.1
0.01
0.0
0.2
0.4
0.6
0.8
1.0
10
20
30
TIME (HOURS)
CAS9
sg009

FIG. 24

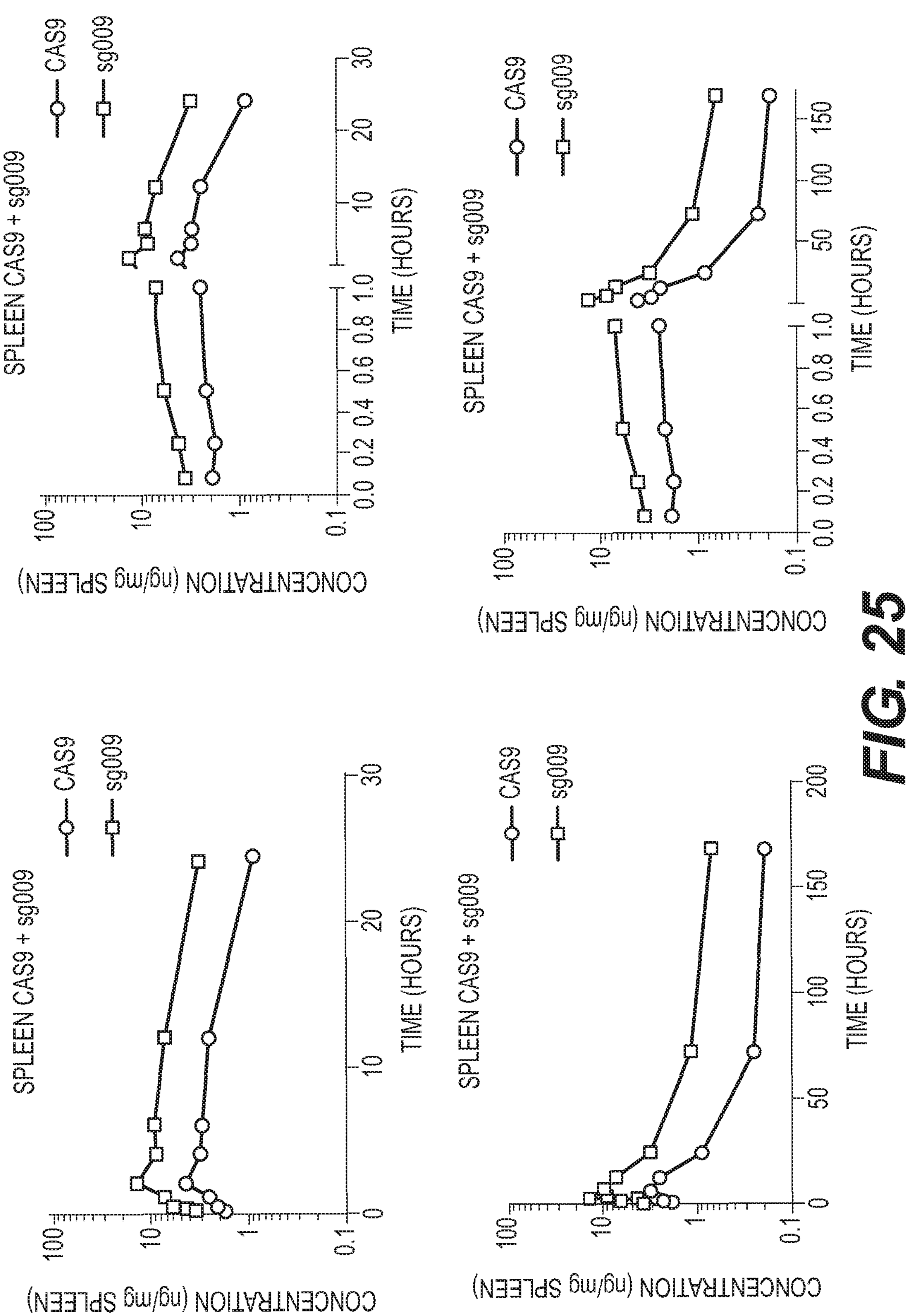
SPLEEN CAS9 + sg009
CAS9
sg009
CONCENTRATION (ng/mg SPLEEN)
100
10
1
0.1
0
10
20
30
TIME (HOURS)
SPLEEN CAS9 + sg009
CAS9
sg009
CONCENTRATION (ng/mg SPLEEN)
100
10
1
0.1
0.0 0.2 0.4 0.6 0.8 1.0
10
20
30
TIME (HOURS)
SPLEEN CAS9 + sg009
CAS9
sg009
CONCENTRATION (ng/mg SPLEEN)
100
10
1
0.1
0
50
100
150
200
TIME (HOURS)
SPLEEN CAS9 + sg009
CAS9
sg009
CONCENTRATION (ng/mg SPLEEN)
100
10
1
0.1
0.0 0.2 0.4 0.6 0.8 1.0
50
100
150
TIME (HOURS)

FIG. 25

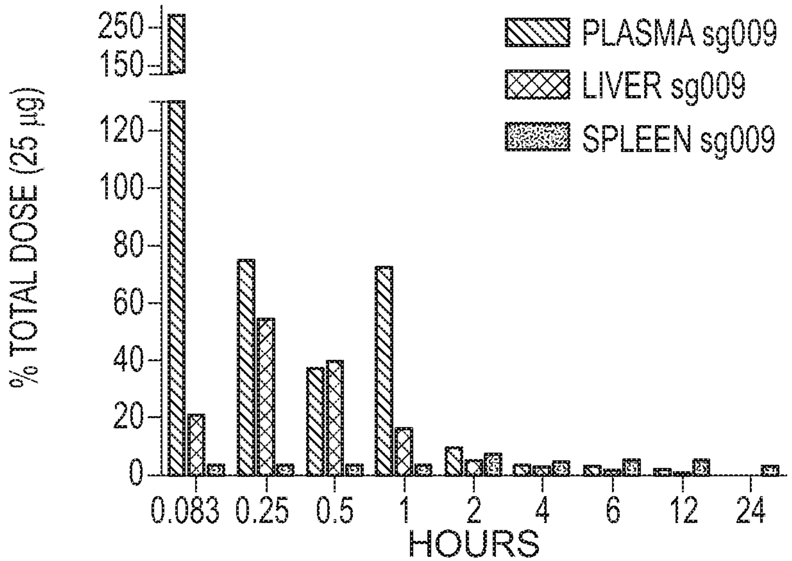


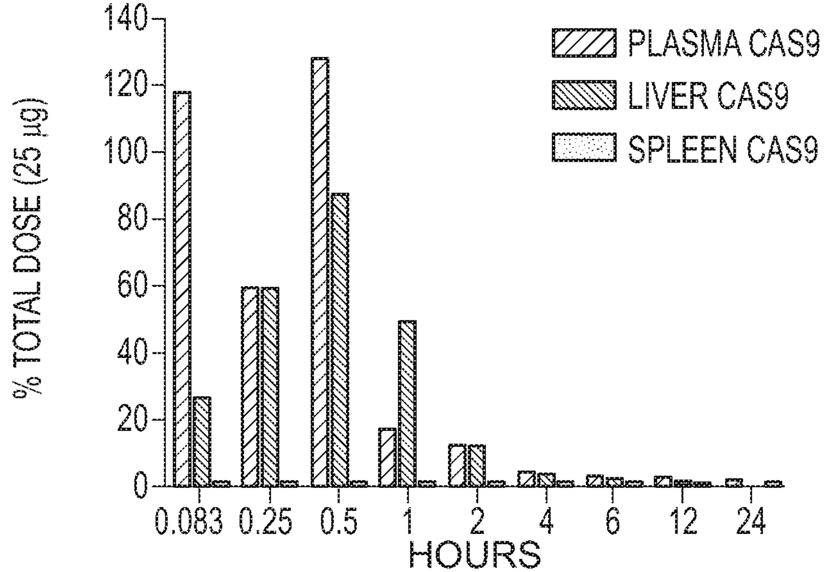


| sg009 % DOSE | | | | |
|---|---|---|---|---|
| TIME (h) | PLASMA | LIVER | SPLEEN | TOTAL % |
| 0.083 | 273.3 | 18.5 | 1.7 | 293.6 |
| 0.25 | 73.6 | 53.6 | 2 | 129.1 |
| 0.5 | 35.9 | 37.8 | 2.8 | 76.6 |
| 1 | 71.9 | 15.6 | 3.4 | 90.9 |
| 2 | 8.5 | 4.7 | 6.5 | 19.7 |
| 4 | 1.8 | 0.6 | 4.1 | 6.5 |
| 6 | 1 | 0.4 | 4.3 | 5.7 |
| 12 | 0.5 | 0.1 | 3.3 | 3.9 |
| 24 | 0.1 | 0.1 | 1.5 | 1.6 |

| Cas9 % DOSE | | | | |
|---|---|---|---|---|
| TIME (h) | PLASMA | LIVER | SPLEEN | TOTAL % |
| 0.083 | 117.1 | 25.4 | 0.9 | 143.4 |
| 0.25 | 57.5 | 57 | 0.8 | 115.3 |
| 0.5 | 127 | 86.3 | 1 | 214.4 |
| 1 | 15.9 | 48.7 | 1.2 | 65.8 |
| 2 | 10.6 | 11.3 | 2.1 | 24 |
| 4 | 2.8 | 1.3 | 1.5 | 5.5 |
| 6 | 2.2 | 0.8 | 1.4 | 4.4 |
| 12 | 0.7 | 0.1 | 1.2 | 2 |
| 24 | 0.1 | 0 | 0.4 | 0.6 |

**FIG. 26A**

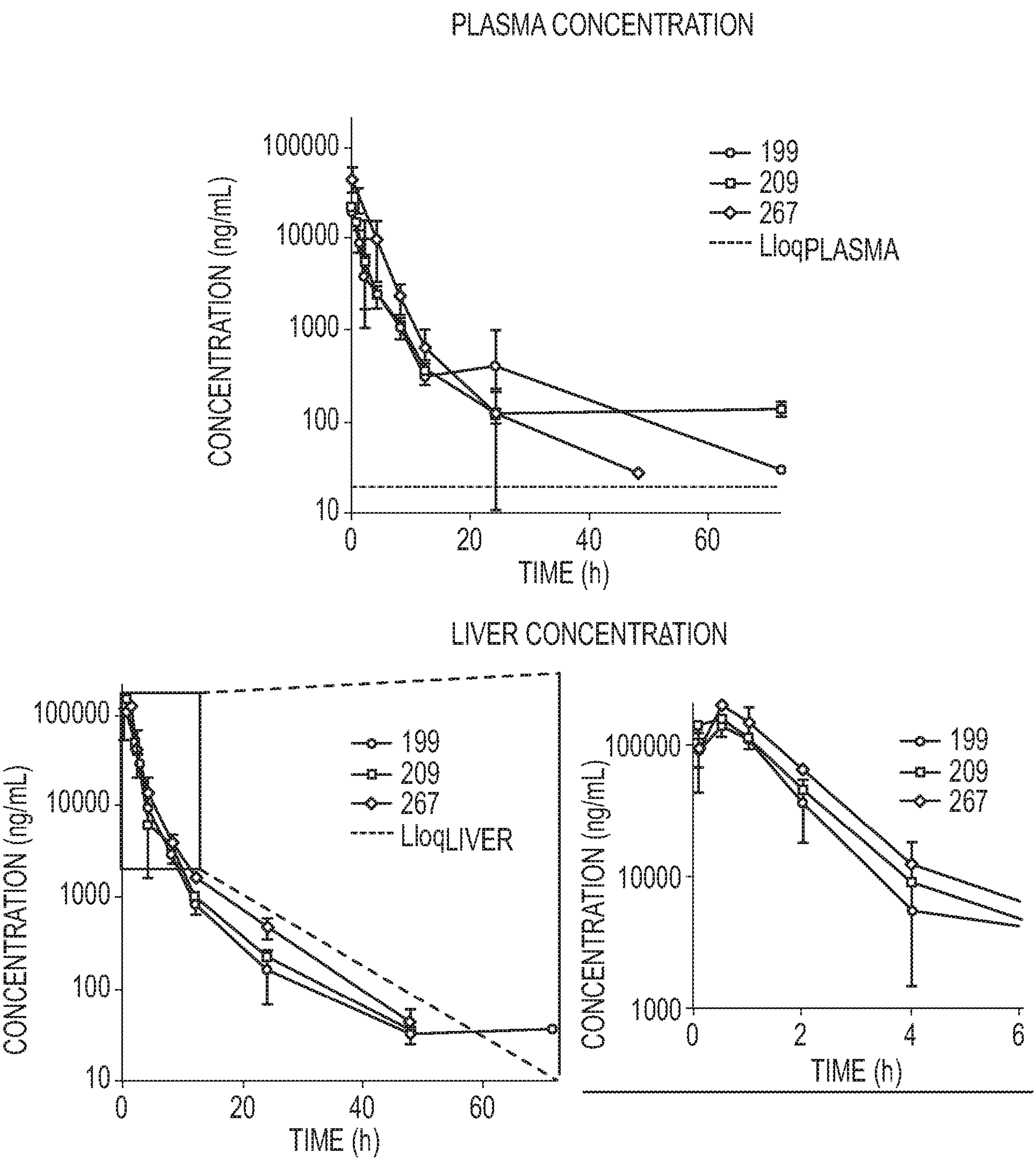
PLASMA CONCENTRATION
CONCENTRATION (ng/mL)
100000
10000
1000
100
10
0
20
40
60
TIME (h)
199
209
267
LloqPLASMA
LIVER CONCENTRATION
CONCENTRATION (ng/mL)
100000
10000
1000
100
10
0
20
40
60
TIME (h)
199
209
267
LloqLIVER
CONCENTRATION (ng/mL)
100000
10000
1000
0
2
4
6
TIME (h)
199
209
267

FIG. 26B

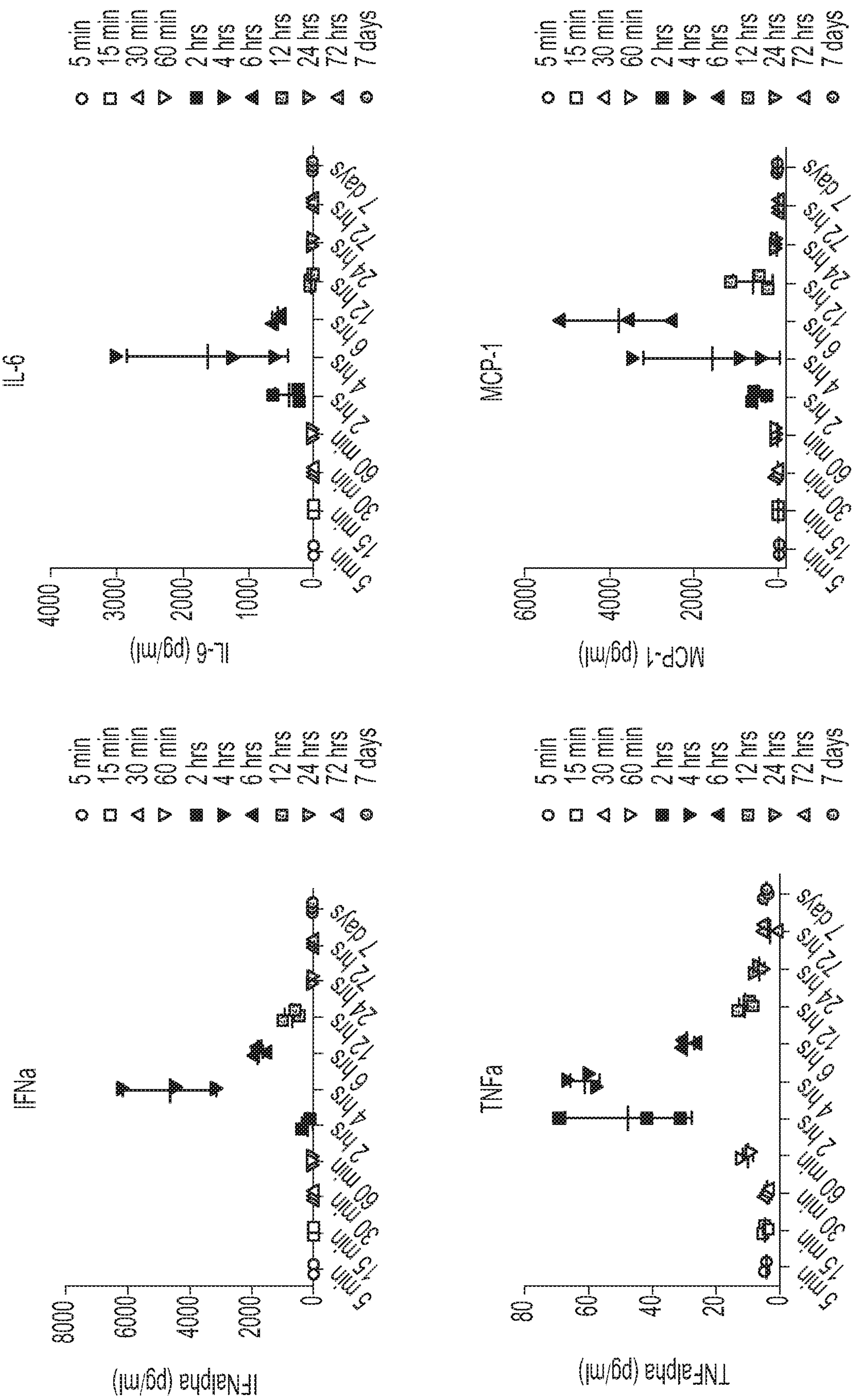
IFNa
IFNalpha (pg/ml)
8000
6000
4000
2000
0
IL-6
IL-6 (pg/ml)
4000
3000
2000
1000
0
TNFa
TNFalpha (pg/ml)
80
60
40
20
0
MCP-1
MCP-1 (pg/ml)
6000
4000
2000
0
5 min
15 min
30 min
60 min
2 hrs
4 hrs
6 hrs
12 hrs
24 hrs
72 hrs
7 days

FIG. 27

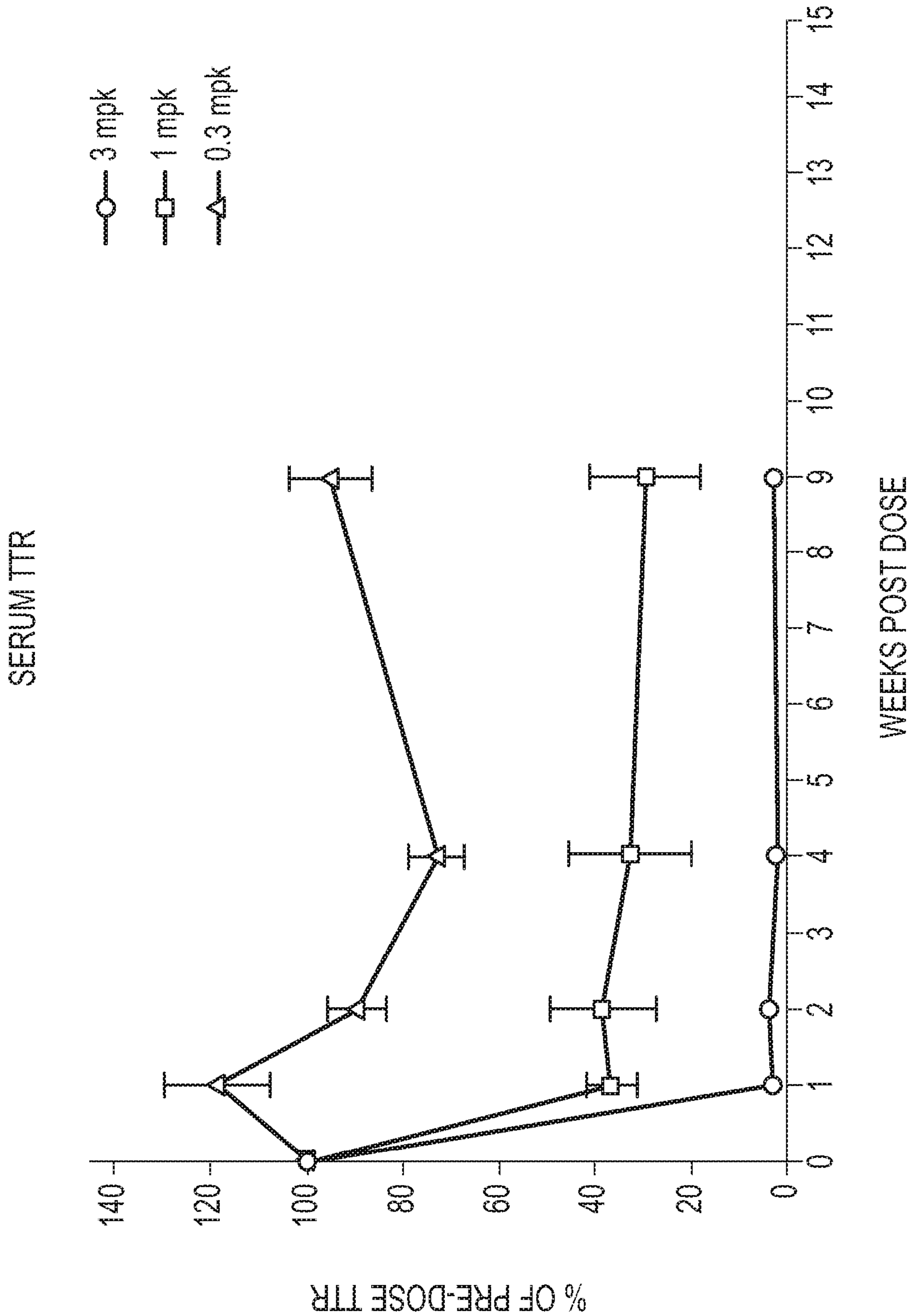
SERUM TTR
3 mpk
1 mpk
0.3 mpk
% OF PRE-DOSE TTR
0
20
40
60
80
100
120
140
WEEKS POST DOSE
0
1
2
3
4
5
6
7
8
9
10
11
12
13
14
15

FIG. 28

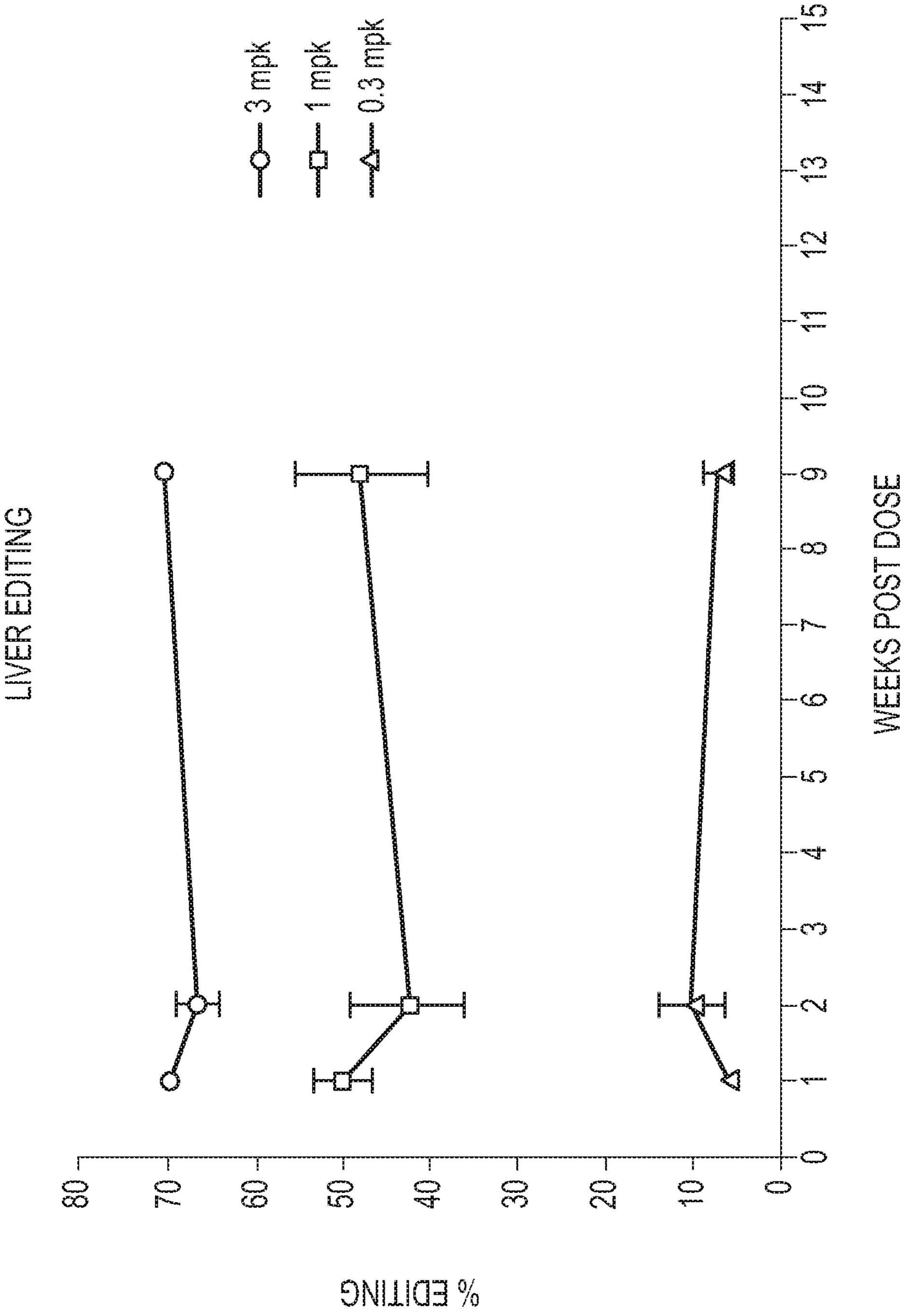
LIVER EDITING
3 mpk
1 mpk
0.3 mpk
% EDITING
0
10
20
30
40
50
60
70
80
WEEKS POST DOSE
0
1
2
3
4
5
6
7
8
9
10
11
12
13
14
15

FIG. 29A

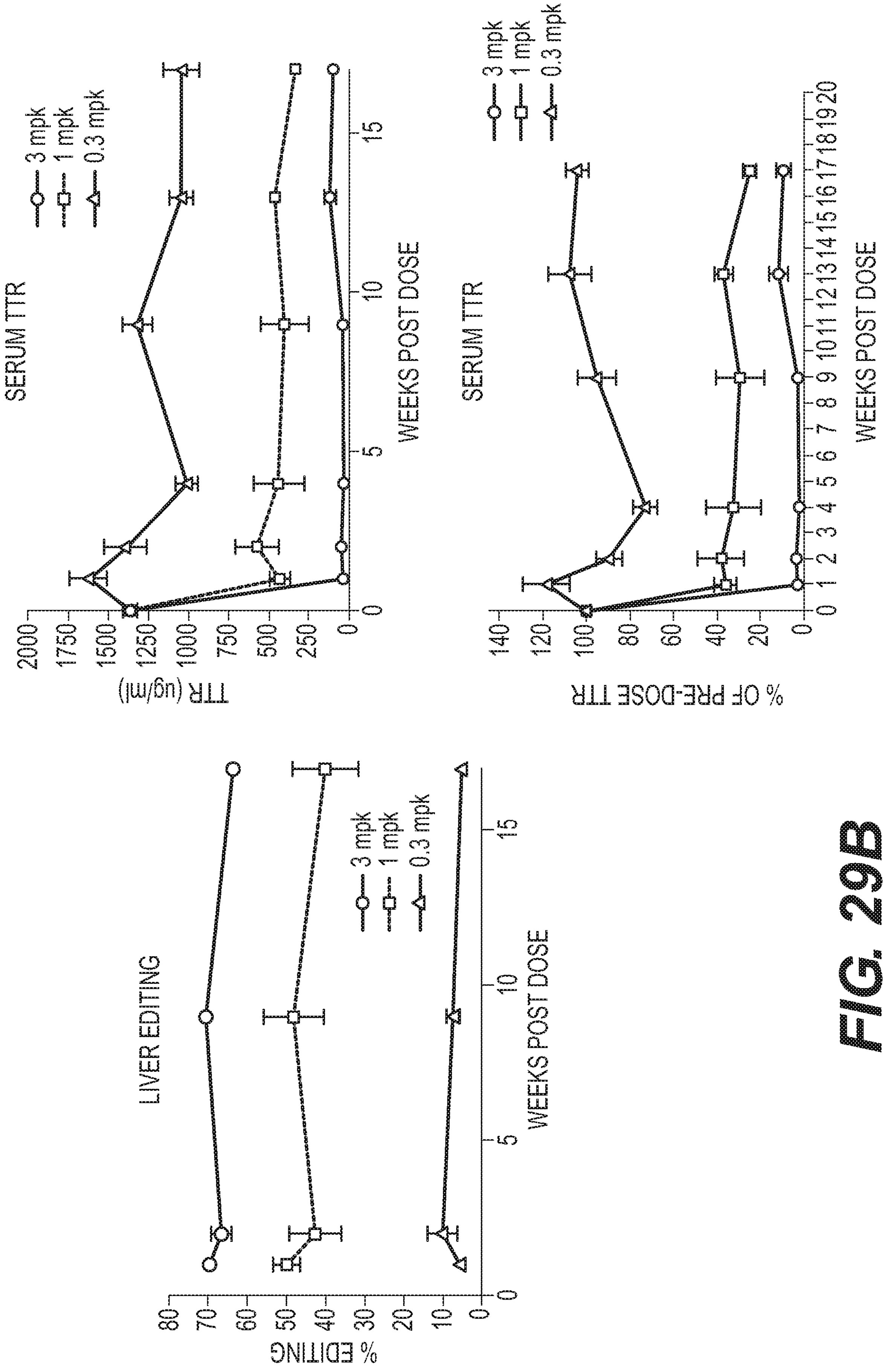
SERUM TTR
TTR (ug/ml)
WEEKS POST DOSE
3 mpk
1 mpk
0.3 mpk
SERUM TTR
% OF PRE-DOSE TTR
WEEKS POST DOSE
LIVER EDITING
% EDITING
WEEKS POST DOSE

FIG. 29B

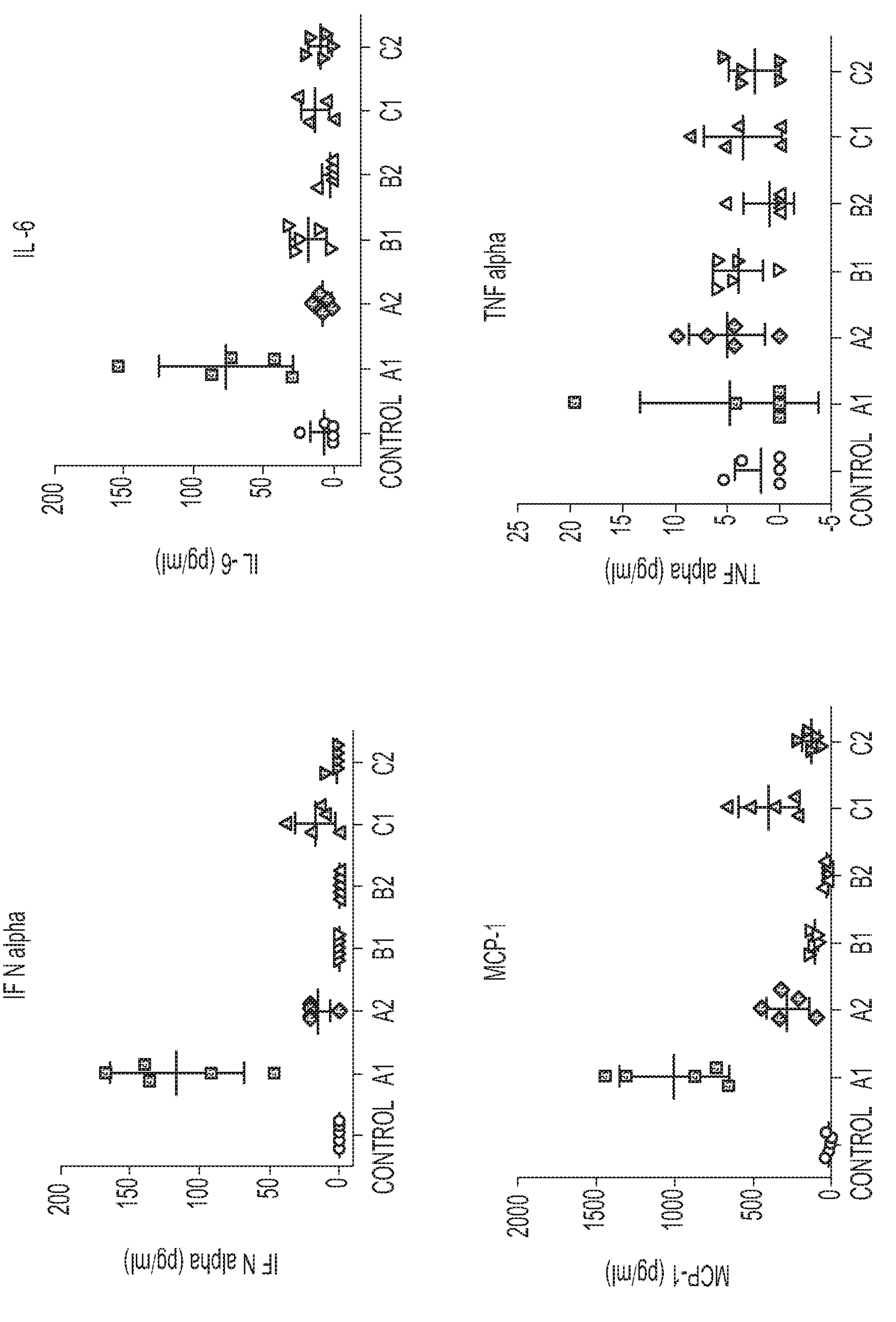
IF N alpha
IF N alpha (pg/ml)
200
150
100
50
0
CONTROL
A1
A2
B1
B2
C1
C2
IL -6
IL -6 (pg/ml)
MCP-1
MCP-1 (pg/ml)
2000
1500
1000
500
TNF alpha
TNF alpha (pg/ml)
25
20
15
10
5
-5

*FIG. 30*

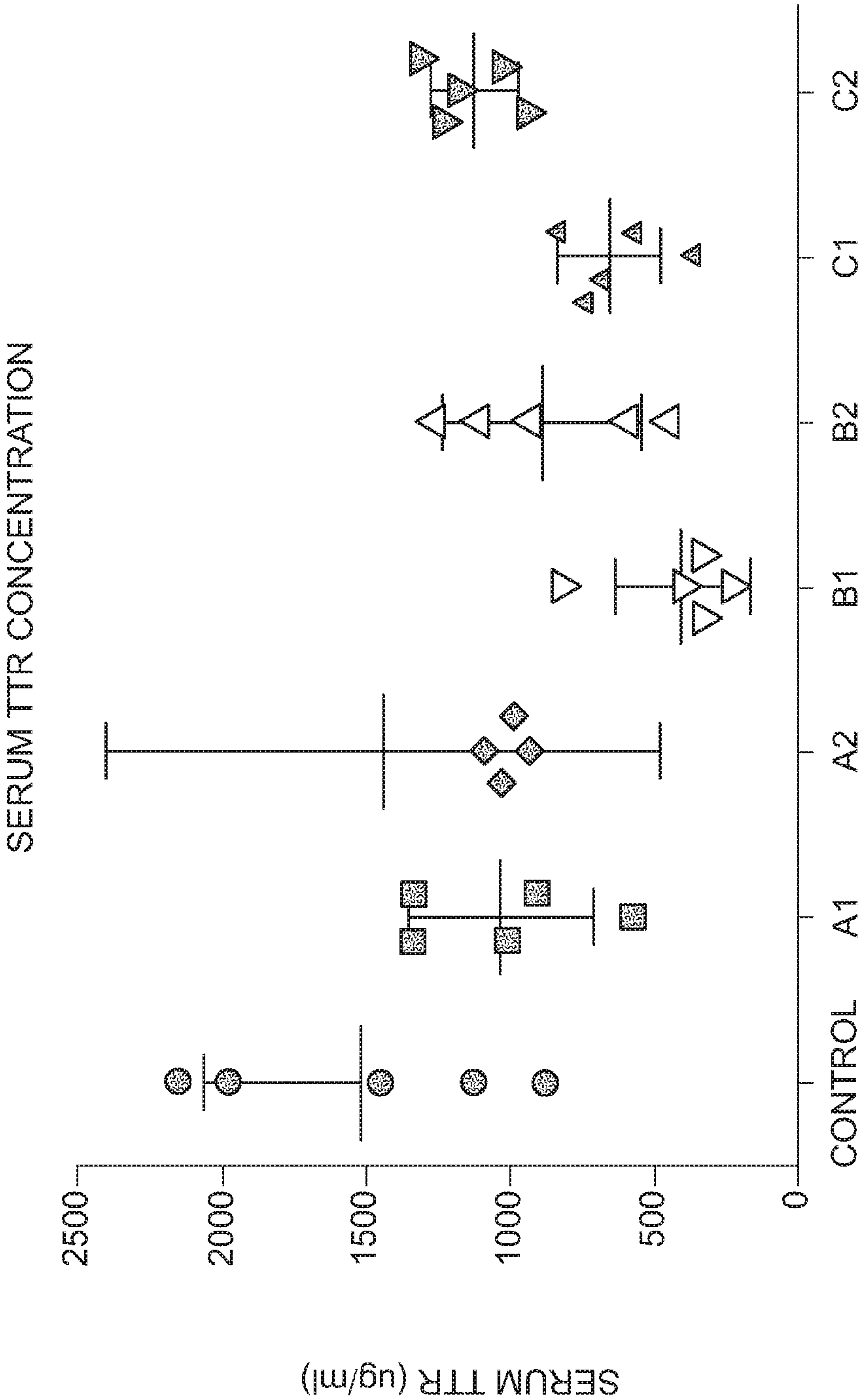
SERUM TTR CONCENTRATION
SERUM TTR (ug/ml)
2500
2000
1500
1000
500
0
CONTROL
A1
A2
B1
B2
C1
C2

*FIG. 31*

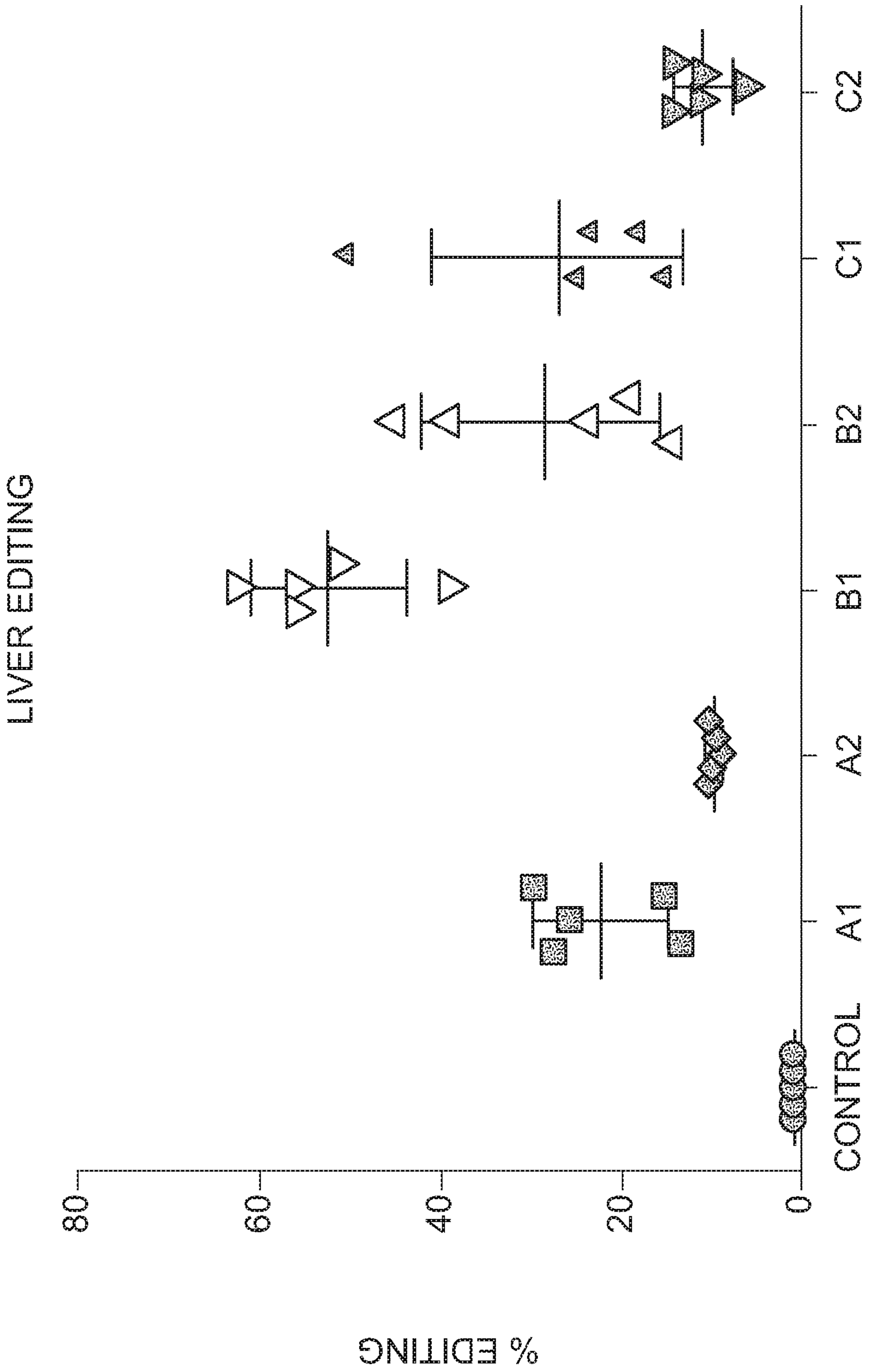


*FIG. 32*

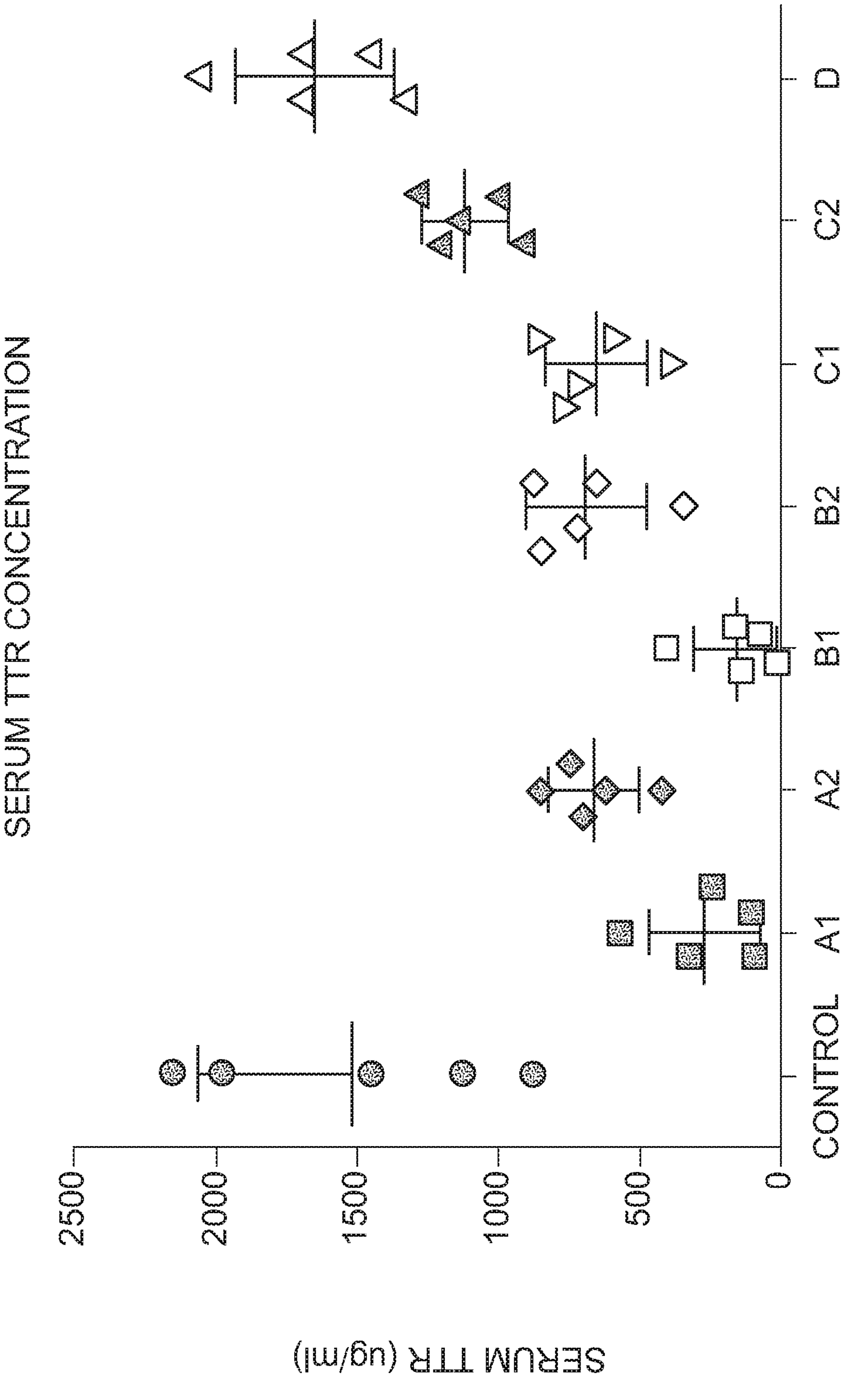
SERUM TTR CONCENTRATION
SERUM TTR (ug/ml)
2500
2000
1500
1000
500
0
CONTROL
A1
A2
B1
B2
C1
C2
D

*FIG. 33*

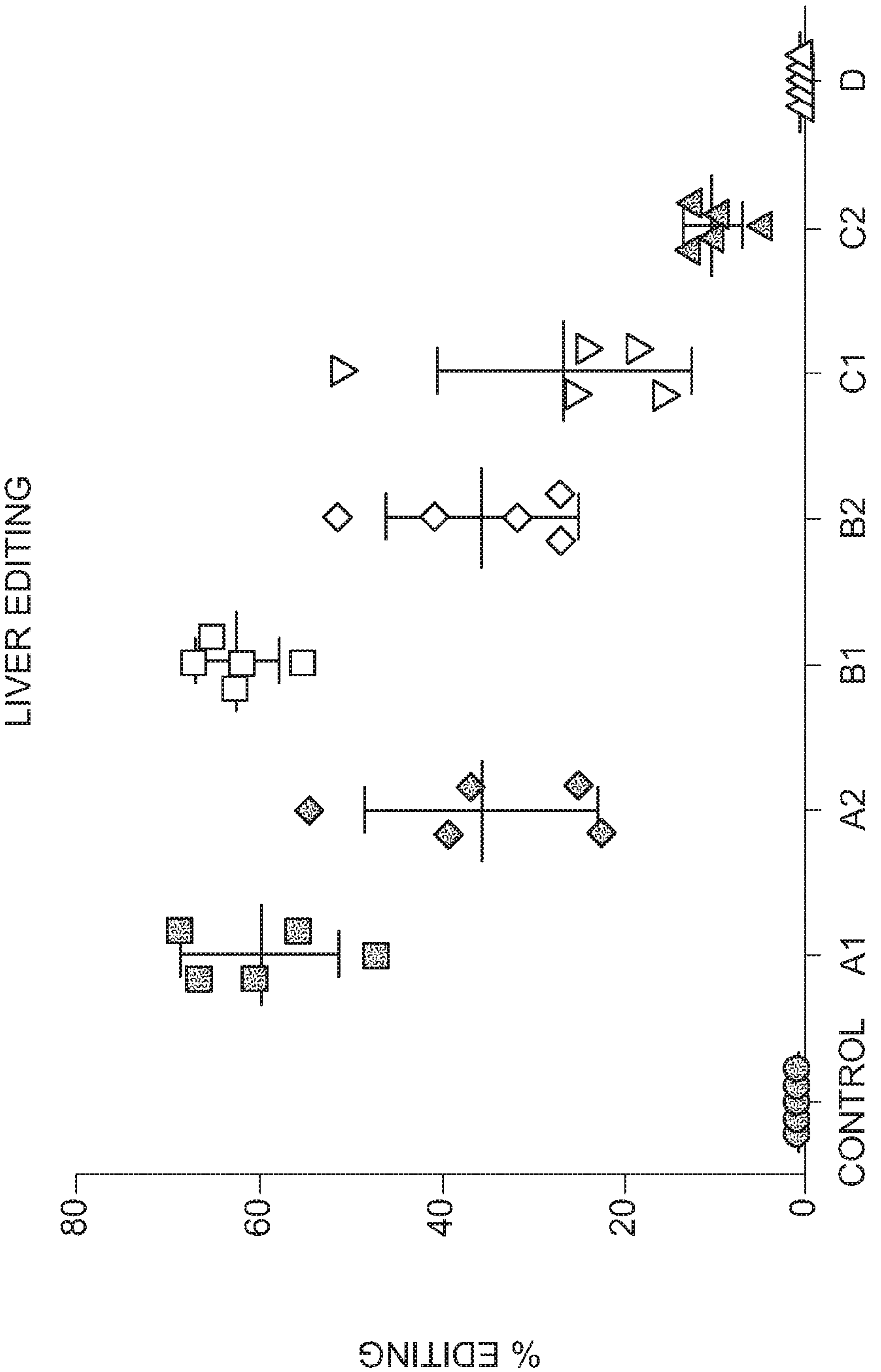


FIG. 34

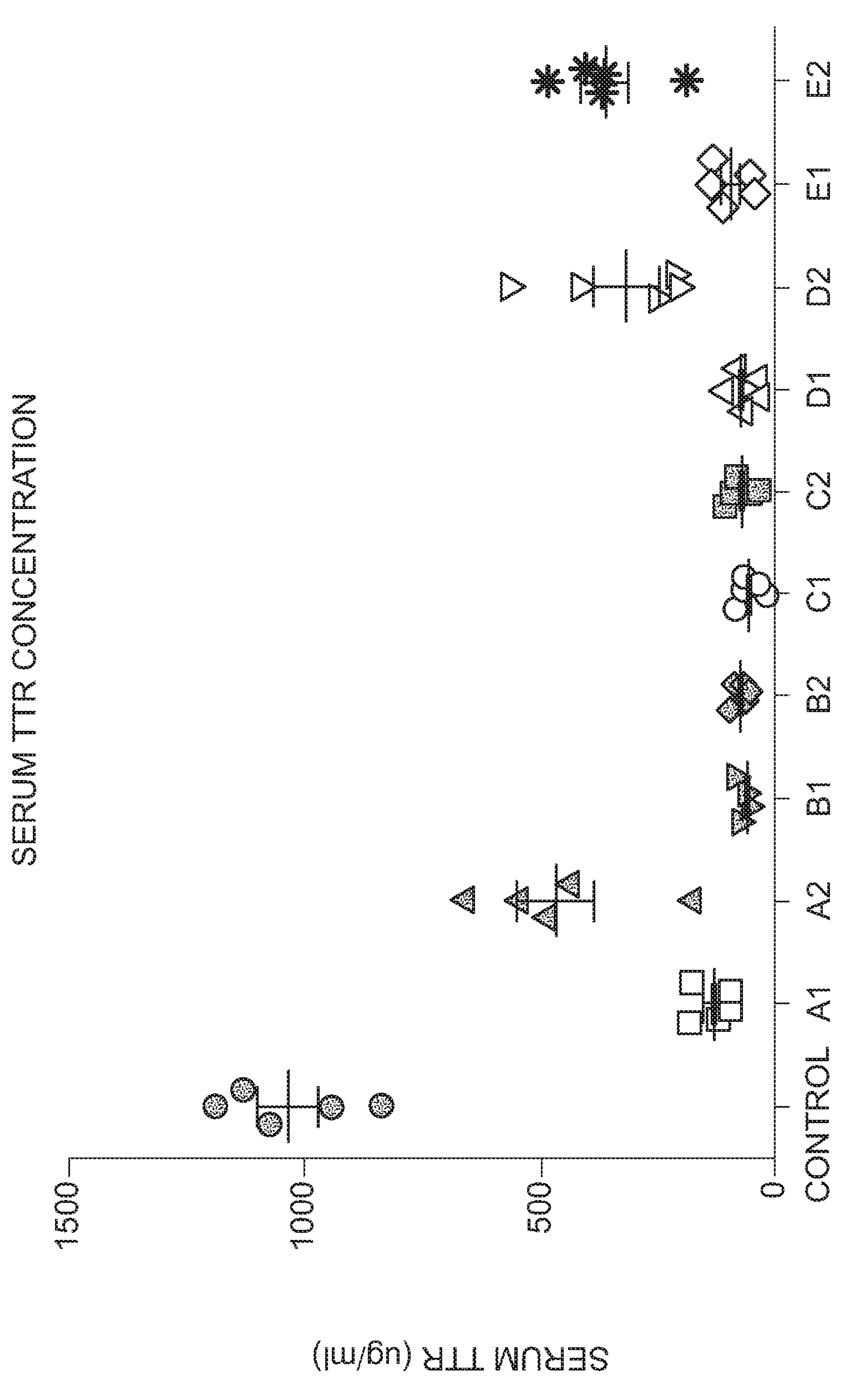
SERUM TTR CONCENTRATION
SERUM TTR (ug/ml)
1500
1000
500
0
CONTROL
A1
A2
B1
B2
C1
C2
D1
D2
E1
E2

FIG. 35

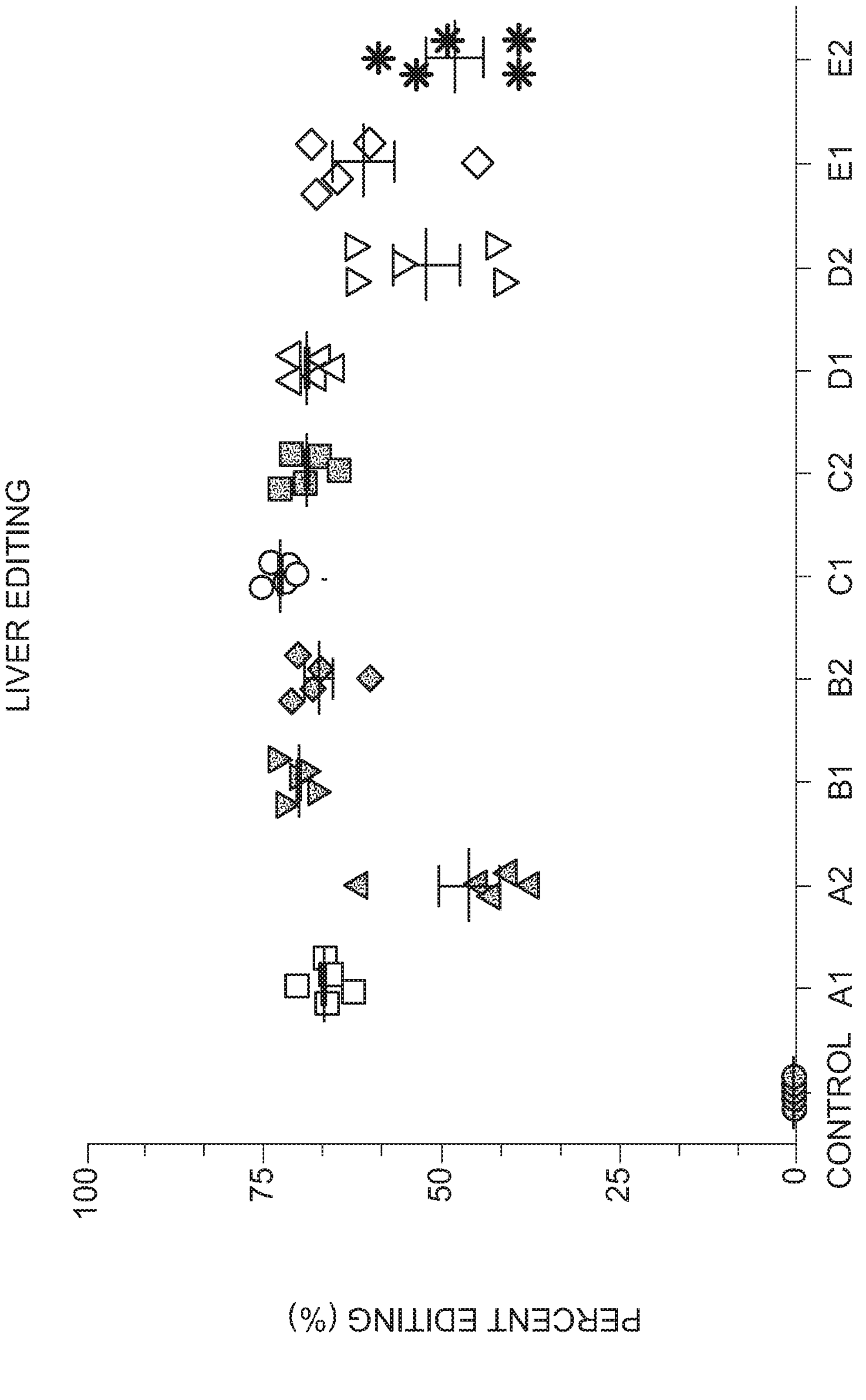
LIVER EDITING
PERCENT EDITING (%)
100
75
50
25
0
CONTROL
A1
A2
B1
B2
C1
C2
D1
D2
E1
E2

*FIG. 36*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62315602
- US 62375776
- US 62433228
- US 62468300
- WO 2014204726 A
- WO 2010129687 A
- WO 2015089406 A
- US 20160304846 A
- WO 2015095340 A
- WO 2014136086 A
- WO 2006007712 A


### Non-patent literature cited in the description


- **WANG et al.** *Arterioscler Thromb Vasc Biol.*, May 2016, vol. 36 (5), 783-786
- **WHITEHEAD et al.** *Nat Commun.*, 2014, vol. 5, 4277
- **MAKAROVA et al.** *Nat Rev Microbiol*, 2015, vol. 13 (11), 722-36
- **SHMAKOV et al.** *Molecular Cell*, 2015, vol. 60, 385-397
- **ZETSCHE et al.** *Cell*, 2015, vol. 163, 1-13
- **MEFFERD et al.** *RNA*, 2015, vol. 21, 1683-9
- **SCHERER et al.** *Nucleic Acids Res.*, 2007, vol. 35, 2620-2628
- **WANG et al.** *Nature*, 2015
- **FONT-BURGADA et al.** *Cell*, 2015, vol. 162, 766-799
- **RAN et al.** *Nature*, 2015, vol. 520, 186-191
- **SCHERPHOF** ; **KAMPS**. The role of hepatocytes in the clearance of liposomes from the blood circulation.. *Prog Lipid Res.*, May 2001, vol. 40 (3), 149-66
- **MAIER, M.A. et al.** Biodegradable Lipids Enabling Rapidly Eliminated Lipid Nanoparticles for Systemic Delivery of RNAi Therapeutics.. *Mol. Ther.*, 2013, vol. 21 (8), 1570-78
- **ROMBERG et al.** *Pharmaceutical Research*, 2008, vol. 25 (1), 55-71
- **HOEKSTRA et al.** *Biochimica et Biophysica Acta*, 2004, vol. 1660, 41-52
- **J. MILTON HARRIS**. *Poly(ethylene glycol) chemistry: biotechnical and, biomedical applications*, 1992
- **LAPECORELLA, M** ; **MARIANI, G.** Factor VII deficiency: defining the clinical picture and optimizing therapeutic options.. *Haemophilia*, 2008, vol. 14, 1170-1175
- **PATEL, K.** ; **HAWKINS, P.** Cardiac amyloidosis: where are we today?. *J. Intern. Med.*, 2008, vol. 278, 126-144
- **KARIKO et al.** *Nucleic Acids Research*, 2011, vol. 39 (21), e142
- **CHO et al.** Targeted genome engineering in human cells with the Cas9 RNA-guided endonuclease. *Nature Biotechnology*, 2013, vol. 31, 230-232